(19) Europäisches Patentamt / European Patent Office / Office européen des brevets

(11) **EP 3 982 729 B1**

(12) **EUROPEAN PATENT SPECIFICATION**

(45) Date of publication and mention
of the grant of the patent:
**19.11.2025 Bulletin 2025/47**

(21) Application number: **20734125.6**

(22) Date of filing: **14.06.2020**

(51) International Patent Classification (IPC):
*A01N 37/46* (2006.01)    *A01N 63/50* (2020.01)
*A01N 63/23* (2020.01)    *C12N 1/20* (2006.01)
*C12R 1/07* (2006.01)    *A01P 7/04* (2006.01)

(52) Cooperative Patent Classification (CPC):
(C-Sets available)
**A01N 63/23; A01N 63/50; C12N 1/20; C12N 1/205;**
C12R 2001/075    (Cont.)

(86) International application number:
**PCT/EP2020/066418**

(87) International publication number:
**WO 2020/249811 (17.12.2020 Gazette 2020/51)**

(54) **BACILLUS THURINGIENSIS STRAIN**

BACILLUS-THURINGIENSIS-STAMM

SOUCHE DE BACILLUS THURINGIENSIS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.06.2019 EP 19382497**

(43) Date of publication of application:
**20.04.2022 Bulletin 2022/16**

(73) Proprietor: **Bioinsectis S.L.**
**31192 Mutilva, Navarra (ES)**

(72) Inventor: **CABALLERO SÁNCHEZ, Javier**
**31192 Multiva, Navarra (ES)**

(74) Representative: **Elzaburu S.L.P.**
**Edificio Torre de Cristal**
**Paseo de la Castellana 259 C, planta 28**
**28046 Madrid (ES)**

(56) References cited:
**EP-A1- 0 366 397      WO-A2-2012/131495
WO-A2-2013/134734      WO-A2-95/04146
CN-B- 103 333 230      US-A1- 2009 005 306
US-A1- 2010 160 231      US-A1- 2018 127 771**

- **RICARDO ANTONIO POLANCZYK ET AL: "Effectiveness of Bacillus thuringiensis strains against Spodoptera frugiperda (lepidoptera: noctuidae).", BRAZILIAN JOURNAL OF MICROBIOLOGY, vol. 31, 1 September 2000 (2000-09-01), BR, pages 165 - 167, XP055623992, ISSN: 1517-8382, DOI: 10.1590/S1517-83822000000300003**
- **DEISE CAPALBO ET AL: "Solid-state fermentation of Bacillus thuringiensis tolworthi to control fall armyworm in maize", EJB ELECTRONIC JOURNAL OF BIOTECHNOLOGY, vol. 4, no. 2, 1 August 2001 (2001-08-01), CL, pages 717 - 3458, XP055625776, ISSN: 0717-3458**
- **MARLITON R BARRETO ET AL: "BIOLOGICAL CONTROL Insecticidal Activity of Culture Supernatants from Bacillus thuringiensis Berliner Strains Against Spodoptera frugiperda Smith (Lepidoptera: Noctuidae) Larvae", AN. SOC. ENTOMOL. BRASIL, vol. 28, 1 January 1999 (1999-01-01), pages 675 - 685, XP055532281**

Remarks:
The complete document including Reference Table(s) and the Sequence Listing(s) can be downloaded from the EPO website

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52)  Cooperative Patent Classification (CPC): (Cont.)

C-Sets
**A01N 63/50, A01N 63/23**

**Description**

**FIELD OF THE INVENTION**

The present invention refers to the field of pest control, specifically to *Bacillus thuringiensis* pest control. More particularly, the invention refers to bacterial strain of *Bacillus thuringiensis* (Bt), SVBS-1801, with proved insecticidal activity against *Spodoptera frugiperda.* The currently available Bt-based products in the market are either ineffective or poorly efficient in controlling *Spodoptera frugiperda* pests.

**BACKGROUND OF THE INVENTION**

*Bacillus thuringiensis* (Bt) is an aerobic and Gram positive soil bacterium, which has been taxonomically classified in the *Bacillus cereus* group of bacteria thanks to its spore forming ability in the absence of nutrients. Bt is known for synthetizing a wide variety of toxins, which target insect pests and other organisms that damage the cultivated plants and significantly reduce their production yield (http://www.lifesci.sussex.ac.uk/home/Neil_Crickmore/Bt/toxins2. html). Several Bt toxins are the result of the aggregation of proteins synthesized by the bacterium. This occurs during the sporulation phase and results in the formation of a parasporal body (crystal) that constitutes a differential characteristic of Bt compared to other species of the same taxonomic group. The crystal comprises $\partial$-endotoxins Cry and Cyt, which are insect-targeted toxins and cytolytic proteins, respectively. The latter has a rather unspecific activity and it is only found in a few Bt strains. The crystal forming proteins contain disulfide bridges and hydrophobic bonds in their structure that render them protease-resistant. Consequently, these proteins can accumulate within the cytoplasm of Bt vegetative cells and facilitate the release of highly concentrated active compounds once the sporulation phase has concluded.

Bt has been traditionally regarded as an effective pest control agent for crops and storage products since its characterization. As a result, the past decades have witnessed an onset of sampling programs throughout the globe leading to the discovery of a wide variety of strains with differential insecticidal properties. Such microorganisms were initially classified according to their flagellar antigen (Lecadet *et al.,* 1999). However, it is now widely accepted that there is no link between the insecticidal properties of a certain strain and its antigen H type. Each Bt strain shows a unique toxicity pattern that depends on the relative proportion of the toxins it produces. Some Bt strains may produce a thermostable secreted exotoxin or β-exotoxin that competes with ATP for the RNA polymerase binding site, therefore, making it toxic to a wide variety of species and preventing it from being used as an insecticide (Sebesta & Horská, 1970).

Schnepf and Whiteley were the first to demonstrate the insecticidal properties of Bt crystals by cloning and expressing a *cry* gene in *Escherichia coli* (Schnepf and Whiteley, 1981). Ever since, many other *cry* and *cyt* genes have been proven to have specific insecticidal activity against insect species classified in the orders Lepidoptera (Baig *et al.,* 2010), Diptera (Roh *et al.,* 2010), Coleoptera (López-Pazos *et al.,* 2010) and Hymenoptera (Sharma *et al.,* 2008). Other phytophages like mites (Frankenhuyzen, 2009) and nematodes (Hu *et al.,* 2010) are also susceptible to such Bt toxins.

There are some noteworthy toxin variants that do not integrate in Bt crystals but that also exhibit insecticidal properties of interest. Such is the case of proteins Cry1I, Vip (vegetative insecticidal proteins) (Estruch *et al.,* 1996) and Sip (secreted insecticidal proteins) (Donovan *et al.,* 2006), which are secreted to the medium during vegetative growth of the cells. Interestingly, all toxin variants with described insecticidal activity (Cry, Cyt, Vip, Sip) are often encoded in in plasmids of around 40 kbp, although in some cases the corresponding genes may also be found in the cell chromosome.

Since Carozzi and colleagues (Carozzi *et al.,* 1991) first introduced the Polymerase Chain Reaction (PCR) as a method for detecting Bt insecticidal genes, it has been the most widespread approach for classifying Bt collections until recent times (Cerón *et al.* 1995; Ferrandis *et al.* 1999). However, recent advancements in high-throughput sequencing have made it possible to achieve a full genetic characterization of a certain Bt strain with a reasonable time/cost ratio. Knowledge of the full chromosomic and plasmidic sequences of Bt strains allow the detection of the cluster of genes responsible for their insecticidal properties, which has often been used as a guideline for determining the hosts of such particular strains. Nonetheless, such an estimation results inaccurate since it does not take into consideration the expression pattern of each gene that ultimately dictates the relative proportion of toxins found in the crystals or secreted to the medium.

The full list of available biocide genes is formed by more than 1000 different sequences (Crickmore, N. et al., 2018) and have been classified following Crickmore's proposed standards (Crickmore *et al.* 1998), which are based on nucleotide and aminoacidic sequences. The way this classifying system works is as follows: all *cry* genes with the same number (eg. *cry1*) share an identity percentage of 45% or higher. If they also share the same capital letter (eg. *cry1A*) then such identity must be 78% or higher. Additionally, if followed by the same lower case letter (eg. *cry1Aa*), the identity between them is considered to be of at least 95%. Finally, to differentiate two genes that encode proteins with an identity of 95% or higher, an additional number may be added after the lower-case letter (eg. *cry1Aa1*).

The number of described *cry* genes is constantly growing, with some of them showing identity percentages below 45% that depict them as new candidates with potential unique insecticidal properties. As a result, the number of hosts that are susceptible to Bt toxins is significantly broadening, including new insect, mite and nematodes species. Although the interest of Bt is mainly targeted towards pest control, the discovery of new genes has made the finding of proteins of other fields of study possible. Such is the case of parasporins, a family of proteins with proven efficacy as antitumoral agents (Ohba et al 2009).

The first formulated product based on Bt was manufactured in France in 1938 and, during the 60s, a considerable amount of Bt industrial products were formulated and produced in the USA, URSS, France and Germany (Milner, 1994). In the 80s, the interest for Bt derived products increased dramatically since chemical insecticides had already started to show flaws due to the appearance of resistant individuals among the insect populations. However, with the arrival of synthetic pyrethroids such interest was soon lost. The increasing interest in replacing chemically synthesized insecticidal compounds by natural compounds or, even, by living organisms able to control pests that, at the same time, are not harmful for the crops and/or vertebrate animals such as humans, have led to a renovated interest in Bt-based products.

Today, knowledge of Bt diversity and its insecticidal gene distribution, thanks to sampling programs throughout the globe, portray this bacterium as a source for generating new insecticides. For these types of products to be used in developed countries they must adjust to the current regulations, which in most cases require the Bt strains to be β-exotoxin free. The reason for this is that such exotoxins display a rather unspecific wide range of action that may result harmful to other organisms, including humans (Sebesta y Horská, 1970).

Insecticidal characterization of Bt strains has been traditionally addressed by performing bioassays. However, due to their resource time consuming nature they have been partially replaced by the identification of *cry* genes through PCR. However, gene content may only be used as an estimate, as it does not show information on the amount of expression of each gene (Masson *et al.,* 1998). Protein interactions within the crystal may also impact its insecticidal characteristics, so that mixtures of functionally diverse Cry and/or Cyt proteins can be more effective than expected (synergism) or less effective (antagonism) (Tabashnik, 1992); consequently, the toxicity of a mixture of Bt proteins (and, usually, Bt strains produce a group of Cry proteins) cannot be predicted from the knowledge about individual endotoxins (Hilbeck and Otto, 2015). Therefore, bioassays are still needed as a complementary source of information to fully characterize a Bt strain.

Thus, the search for novel Bt strains active against specific insect or nematode pests is hindered not only by the fact that each Bt strain shows a unique toxicity pattern that depends on the toxins it produces, their relative proportion and the possible and unpredictable synergistic or antagonistic effects that might occur among the toxins produced, but also because Cry proteins with high degrees of homology might show important differences in specificity and functionality that make difficult to try to extrapolate its activity to predict the activity of homologous proteins that might be produced by other Bt strains, not being obvious that a Bt strain that produces a Cry protein very similar to another one might have activity against the same group of insects or the degree of activity that the homologous Cry protein might show when it is interacting with other Bt proteins in the mixture of proteins produced by a Bt strain.

An additional complication that makes the search and identification of Bt strains with high activity against a particular insect difficult and non obvious is that the available information about the activity and specificity of some Cry proteins or about the insecticidal potential of some already known Bt strains is incomplete, not supported by experimental data or based on assays and results expressed in units or relative to magnitudes different from those usually handled by those skilled in the art, thus reducing the utility and applicability of the published information.

For instance, in the particular case of the control of *Spodoptera frugiperda,* one can find several different publications referred to Bt strains that are said to have activity against *S. frugiperda* without including assays that confirm it or including results expressed in units different from the standard units used for those skilled in the art to express insecticidal activity, making difficult to assess its degree of effectivity. One can also find publications reporting activity of some Cry proteins against *S. frugiperda* which are not based on assays against this lepidopteran or which are based on assays carried out with the individual proteins, which do not allow to infer the activity that the assayed protein might have when it is interacting with other proteins produced by a Bt strain or the differences in activity and specificity that might arise from changes in the amino acid sequence, even if a high degree of homology is maintained with regard to other Cry protein, as explained above.

The European patent application EP-0366397-A1, for instance, describes a Bt strain (PS814) which is said to have activity against lepidopteran pests. However, no assays of activity against *S. frugiperda* are shown. The strain comprises in its genome a gene with 100% identity and full overlap with the sequence of the gene known as *cry1Ea7* (accession number AY894137 in the database btnomenclature, http://www.btnomenclature.info), which is presented in the document as a delta endotoxin gene which is active against lepidopteran pests, but there is no assay demonstrating any activity of the corresponding Cry protein against any insect pests at all.

US application US2010/160231-A1 discloses compositions including coding sequences encoding for amino acid sequences that are said to correspond to pesticidal polypeptides, as well as methods for conferring pesticidal activity to bacteria and other cells and tissues, including seeds. The coding sequence of the delta-endotoxin named axmi-150 in particular, shows full overlap and 100% identity with the sequence of gene known as *cry1Nb1* (accession number KC156678 in the database btnomenclature, http://www.btnomenclature.info). Although the document includes a long list of insects that could be controlled by recombinant strains containing the gene or compositions comprising the polypeptide gene product, the insecticidal activity of proteinAXMI-150 is only assayed and shown against four pests, none of them being a member of the *Spodoptera* genus and particularly, none of them being *S. frugiperda:* European Corn Borer *(Ostrinia nubilalis),* Velvet Bean Caterpillar *(Anticarsia gemmatalis),* Diamondback Moth *(Plutella xylostella),* Southwest Corn borer *(Diatrella grandiosella).*

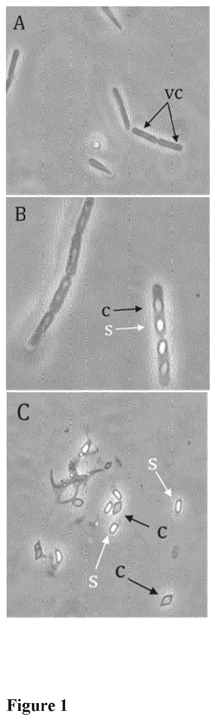

Polanczyk and coworkers (Polanczyk et al., 2000) reported assays with several *Bacillus thuringiensis* strains against *Spodoptera frugiperda.* Figure 1 of said article shows the mortality rates of *S. frugiperda* for each of the tested strains. The strain with the best efficacy belongs to serovar *aizawai,* like the one present in the commercial product Xentari®. The results are presented as bacteria per volume unity (cells/ml), which differs from the otherwise standard units (micrograms/ml) for the preparation of the active ingredient in commercial insecticides, what makes difficult to assess and compare the insecticidal capacity of such strain with that of the commercial insecticides currently on use. The data provided about the strain does not make obvious the search for alternative natural strains with better insecticidal capacity against *S. frugiperda.*

Capalbo and coworkers (Capalbo et al., 2001) discloses a method for fermentation of a *Bt* strain, *Bt* var. *tolworthi* which was previously described to be active against S. *frugiperda.* The fermentation is carried out in solid-state, not in a liquid medium. No specific data about the strain itself, such as its *cry* gene content or proteins is shown. Assays about its effectiveness are included, showing 0.37 mg of biomass/mL as lethal concentration capable to kill 50% of the insects, the $LC_{50}$, but the methodology to determine such value is absent of the Material and Methods section of the document, which makes difficult to assess and compare the insecticidal activity of the strains with that of other Bt strains.

Barreto and coworkers (Barreto et al., 1999) discloses assays of the activity against *S. frugiperda* of Vip proteins of several *Bt* strain. The summary of the document mentions the striking differences among all the described strains despite all of them belonging to the same serovar (Berliner). This highlights the difficulty of finding *Bt* strains with high activity against a specific species (eg. *S. frugiperda*) based only on genetic and molecular characteristics. In the publication of Barreto et al , the assayed supernatants with higher activity are said to possibly contain β-exotoxins (see the end of the left column of page 680 and its continuation in the right column), a genetic feature that is not desired for the object of the present invention, since they are not applicable for bioinsecticides, and that is not present in strain SBVS-1801. Throughout this publication, , there are no evidences showing linking the described strains to SBVS-1801, compositions obtained from its culture or its use as insecticide or for the preparation of insecticides.

Other different published documents describe individual delta-endotoxin proteins from Bt strains (either isolated therefrom or obtained by mutation of natural proteins) which are said to have a certain activity against *S. frugiperda.* However, most of said documents teaches or discusses information that is not complete or not corroborated by appropriate assays, which render them documents with reduced utility for the search of novel, and natural, Bt strains with high activity against *S. frugiperda:* One skilled in the art will found that, for instance, some of the documents do not disclose experimental data from combinations of several Cry / Cyt proteins, therefore, missing the effects due to interactions between insecticidal proteins, which is what would occur if produced by a Bt strain in natural conditions, or that the protein concentrations that are used for the efficacy tests are not the ones produced by a natural Bt strain, which depends on the culture conditions. It must be taken into account, as well, that the production of a specific polypeptide by a Bt strain does not imply that the strain will generate a crystal with high activity against S. *frugiperda.* Some of the particular aspects of such documents are as follows:

International patent application published as WO 2012/131495 A2 discloses several mutant polypeptides of a natural delta-endotoxin, Cry1Ab. The mutants derived from Cry1Ab are variants that contain amino acid substitutions. Such recombinant proteins show activity against *Spodoptera frugiperda,* some of them even higher than that of the natural polypeptide. Although the conditions in which the assays were performed are not well described, the experiments only involve protein Cry1Ab and its derived mutants as the active ingredient. Thus, WO 2012/131495 lacks interaction assays between Cry proteins, which can have a dramatic effect on the overall insecticidal potency. Also, the concentrations used are artificial and therefore it is difficult to anticipate if they would be compatible with those produced from a Bt strain. Moreover, the only microorganisms mentioned in WO 2012/131495 are those transformed with the nucleic acids that encode the mutant polypeptides. Therefore, the mentioned international application is difficult to be considered as an appropriate source of information for identifying novel natural and alternative Bt strains with activity against S. *frugiperda.*

Chinese patent CN 103333230 B describes a delta-endotoxin, Cry1Da3, which is encoded by the DNA sequence that can be found in the btnomenclature database with the accession number HQ439784. Although the activity of Cry1Da3 against small cabbage moth *(Pieris brassicae)* and beet armyworm *(Spodoptera exigua)* is addressed, other species of the genus *Spodoptera* are not mentioned, particularly not having any mention to *S. frugiperda* and the possible effects against it.

International Patent Application WO 95/04146 A2 discloses a gene *(cryET4)* and a protein (CryET4) with high identity with those of the gene known as *cry1Ja1* (accession number L32019 in the btnomenclature database) and its corresponding encoded protein. Noteworthily, the first paragraph of page 5 highlights how Cry proteins with a high degree of identity can show quantitatively different toxicities for individual insect species. A Bt strain transformed with the *cryET4* gene is also described. Example 4 of WO 95/04146 A2 shows that the purified CryET4 protein shows activity against *S. frugiperda.* Nevertheless, the activity of the Bt strain wherefrom the gene was isolated and the crystal composition obtained after its fermentation are not addressed, an information that is crucial for determining if it constitutes a novel, natural and alternative Bt strain for controlling S. *frugiperda.*

US patent application US 2009/005306 discloses a Bt gene which encodes a protein, Cry2Adshi, with insecticidal activity against several insect pests. However, the results shown in Table 1 of the mentioned US application indicate that the protein is not active against *S. frugiperda.* This is further confirmed in Table 3 of Example 3, which indicates that "No Activity" was detected against fall armyworm *(S. frugiperda).* Moreover, the main object of the invention disclosed in said US applications appears to be the generation of transgenic plants expressing the genes disclosed in US 2009/005306 and not the identification of novel, natural Bt strains with high activity against *S. frugiperda.*

US patent application US 2018/127771 A1 discloses several proteins derived from Bt strains with activity against the Asian Cytrus Psyllid *(Diaphorina citri),* abbreviated ACP, which belongs to a different order (Hemiptera) than *S. frugiperda* (Lepidoptera). In fact, *S. frugiperda* is not even mentioned in the US patent application. Example 1 states that the overall goal of the study was to identify a Bt crystal toxin with toxicity against ACP. Moreover, the identified toxins are intended to be used to control plant y hemipteran insects through their implementation in transgenic plants.

International Patent Application WO 2013/134734 A2 discloses two proteins with high identity with the proteins encoded by the genes *cry1Ab24* and *cry1Ja1* already mentioned. However, they are not the main object of the invention. WO 2013/134734 A2 focuses on polypeptides that result from the combination of several peptides, some of them with insecticidal properties, and the improvement of their expression in transgenic plants. Foliar bioassays related to the control of *S. exigua* are shown, carried out with a sprayed-dried powder containing peptides derived from a Bt protein and an Inhibitor Cysteine Knot peptide. But *S. frugiperda* is simply mentioned as one of the insects whose control is of interest, without providing any assays against this species.

Thus, despite the genetic diversity found in Bt strains, but consistently with the difficulties of finding *Bt* strains with high activity against a specific species such as. *S. frugiperda* based only on genetic and molecular characteristics, commercially available bioinsecticides to control lepidopterans rely on just a few from serovars *kurstaki* and *aizawai.* As one of the most widespread products, Dipel®, utilizes an active ingredient made from a combination of spores and crystal proteins from strain ABTS-351 (serovar *kurstaki).* Crystals produced by strain ABTS-351 comprise proteins Cry1Aa, Cry1Ab, Cry1Ac and Cry2Aa, which are found in a relative proportion that depends on the composition of the medium and the growing conditions. Dipel®, as well as other commercial formulated products based on strain ABTS-351, are efficient for controlling a broad spectrum of larvae species belonging to the order Lepidoptera, including species from the *Heliothis, Plutella* and *Lobesia* genus. However, it is widely accepted that such products are poorly effective against insect species belonging to the genus *Spodoptera,* including *S. frugiperda, S. littoralis, S. exigua,* etc. Since these species damage crops with critical negative results for the economy, strain ABTS-1857 (serovar *aizawai*) was selected as a new source of insecticidal toxins. Xentari® is currently the most notorious commercial insecticide based on ABTS-1857 strain, carrying proteins Cry1Ca, Cry1Da, Cry1Aa, and Cry1Ab. The first two proteins are the main responsible insecticidal agents for its effectiveness against *Spodoptera* species, although synergistic effects due to the presence of the other proteins cannot be discarded.

Despite the existence of Bt based products in the market, insect populations have already developed different degrees of resistance to them and the efforts in finding new Bt strains for pest control is on the rise. It is necessary to identify new Bt strains with greater insecticidal potency and wider host range. Specially for the purposes of the present invention, it would be particularly interesting if it were possible to find and identify a new Bt strain with improved insecticidal activity against *Spodoptera frugiperda.* Preferably, the new Bt strain should be a natural strain, isolated from an environmental sample. Also preferably, in order to facilitate the use of the strain, or of the products based on it, the new Bt strain should not expressed β-exotoxin, to avoid the risk of cell mortality to a wide variety of species.

The present invention provides a solution to such problem.

## SUMMARY OF THE INVENTION

The present invention refers to a strain of *Bacillus thuringiensis* (the strain of *B. thuringiensis* of the present invention), which is characterized by being the strain SVBS-1801 deposited in the Colección Española de Cultivos Tipo (CECT) with the deposit reference number CECT 9753.

In a second aspect, the invention also refers to a method for preparing a mixture of spores and crystals of the *B. thuringiensis* strain of the present invention, SBVS-1801, which comprises the steps of:

a) growing SBVS-1801 bacteria until more than 95% of the bacterial cells have undergone sporulation, have lysed and released their crystals and spores to the culture medium;
b) purifying the crystals and spores by provoking their sedimentation from the culture medium where they are in suspension, preferably by centrifugation, and collecting the precipitate;

c) optionally, washing the precipitate with a protease inhibiting solution and provoking again the sedimentation of the crystals and spores;
d) storing the purified crystals and spores either:

   a. at room temperature, after having lyophilized the precipitate obtained in step b) or c), or
   b. at a temperature between -18°C and -20°C or lower, after having resuspended the precipitate obtained in b) or c) in water or in an aqueous solution.

In a third aspect, the invention relates to a method for obtaining purified crystals (parasporal crystals) of the *B. thuringiensis* strain of the present invention, SVBS-1801, which method comprises the steps of:

   i) washing a mixture of crystals and spores of SVBS-1801 with NaCl 1M and centrifuging it at 9000g for 10 min.,
   ii) collecting the pellet and resuspending it in PBS,
   iii) adding hexane to the suspension obtained in step ii) and vortexing it;
   iv) centrifuging the suspension of iii) at 6000 g, 4°C, 10 min,
   v) repeating steps ii) to iv) at least three times,
   vi) colleting the pellet of crystals obtained in v) and washing it three times in cold destilled water.

The mixture of crystals and spores used to obtain the purified crystals can be the mixture obtained after step b) or c) of the method for preparing a mixture of spores and crystals of the *B. thuringiensis* strain of the present invention, SBVS-1801, which is the second aspect of the present invention.

In a fourth aspect, the invention also refers to a composition which comprises at least one of the group of a) vegetative cells, b) bacterial cells containing spores, c) spores, d) crystals (parasporal crystals), e) a mixture of spores, crystals and crystal proteins, f) at least one Cry protein not included in a crystal, g) at least a Vip protein, or combinations thereof, of the *B. thuringiensis* strain of the present invention, in which composition, if no member of the group defined in a) to e) is present, i) when at least a Cry protein is present, at least protein Cry1Ja1-like (SEQ ID NO:8) is present, and/or ii) all three proteins of the group of Vip1Ca1 (SEQ ID NO:14), Vip2Ac1 (SEQ ID NO:16) and Vip3Af3 (SEQ ID NO: 18) will be present in the composition. Such a composition is considered a composition of the present invention. Optionally, in an embodiment compatible with any other one, the composition can also include, additionally, a portion of the supernatant obtained after having performed steps a) and b) of the method for preparing a mixture of spores and crystals of the *B. thuringiensis* strain of the present invention. Preferred embodiments of the composition of the invention are those where the composition comprises at least either: a) a mixture of spores and crystals; b) a mixture of spores, crystals and a portion of the supernatant obtained by performing steps a) and b) the method of preparing a mixture of spores and crystals of strain SBVS-1801 which is an aspect of the present invention; c) crystals; d) crystals and a portion of the supernatant obtained by performing steps a) and b) the method of preparing a mixture of spores and crystals of strain SBVS-1801 which is an aspect of the present invention. Preferred are, as well, those compositions which comprise at least a mixture of spores, crystals and crystal proteins of strain SBVS-1801 (which are typical components of the compositions used to prepare insecticides from the culture medium of a Bt strains). Also preferred are those compositions which comprise Cry proteins not included in crystals and wherein the composition comprises at least all the Cry proteins of the group of SEQ ID NO:4 (Cry1Da3), SEQ ID NO:6 (Cry1Ea7) and SEQ ID NO:8 (Cry1Ja1-like), not being included in crystals. The compositions of the present invention can also additionally comprise at least an agriculturally acceptable excipient.

Yet another aspect of the invention is the use of the *Bacillus thuringiensis* strain of the present invention and or a composition of the present invention as an insecticide or for the preparation of an insecticide. Preferably, it will be used for the control of insect pests of the species *S. frugiperda*. Also preferably, the composition will be used to protect plants from pests. The plants can be maize plants, as in Example 8, or any other plant such as, for instance, cotton (another plant where *S. frugiperda* is the cause of important damages and where the use of transgenic plants expressing Cry proteins has been done before), rice, sorghum, sugarcane and others.

Disclosed, but not forming part of the invention is a method for the identification of the presence of a *B. thuringiensis* strain of the present invention, which comprises a step where it is determined either

   a) that the genome of the strain comprises:

      i) at least a gene or DNA region whose sequence is selected of the group of SEQ ID NO:1 or SEQ ID NO:7, or combinations thereof; and/or
      ii) at least all the genes or DNA regions of the group of SEQ ID NO:1, SEQ ID NO:3, SEQ ID NO:5, SEQ ID NO:7, SEQ ID NO:9 and SEQ ID NO:11, and/or
      iii) at least all the genes or DNA regions of the group of SEQ ID NO:13, SEQ ID NO:15 or SEQ ID NO:17, and/or
      iv) all the genes or DNA regions of the group of SEQ ID NO:1, SEQ ID NO:3 SEQ ID NO:5, SEQ ID NO:7, SEQ ID NO:9, SEQ ID NO:11, SEQ ID NO:13, SEQ ID NO:15 and SEQ ID NO:17, or

b) that the strain expresses:

i) at least a polypeptide whose amino acid sequence is selected of the group of SEQ ID NO:2 or SEQ ID NO:8, or combinations thereof, and/or

ii) at least all the polypeptides of the group of polypeptides whose amino acid sequence is SEQ ID NO:2, SEQ ID NO:4, SEQ ID NO:6, SEQ ID NO:8, SEQ ID NO:10 or SEQ ID NO:12, and/or

iii) at least all the polypeptides of the group of polypeptides whose amino acid sequence is SEQ ID NO:14, SEQ ID NO:16 or SEQ ID NO:18.

iv) all the polypeptides of the group of polypeptides whose amino acid sequence is SEQ ID NO:2, SEQ ID NO:4, SEQ ID NO:6, SEQ ID NO:8, SEQ ID NO:10, SEQ ID NO:12, SEQ ID NO:14, SEQ ID NO:16 or SEQ ID NO:18.

Also disclosed, but not forming part of the invention is a DNA molecule of the invention, that is, a nucleic acid molecule comprising a nucleotide sequence selected from the group of:

a) a nucleotide sequence selected of the group of SEQ ID NO:1 and SEQ ID NO:7;

b) a nucleotide sequence which encodes a polypeptide comprising an amino acid sequence of the group of SEQ ID NO:2 and SEQ ID NO: 8;

c) a nucleotide sequence which encodes a polypeptide having at least 99% of identity with an amino acid sequence of the group of SEQ ID NO:2 and SEQ ID NO:8 and which has pesticidal activity (for instance, nematocidal and/or insecticidal activity).

Consequently, also disclosed, but not forming part of the invention, is an expression vector comprising a DNA molecule as described above.

In the same line, disclosed, but not forming part of the invention, is a recombinant virus, bacterium, fungus or yeast, a plant cell or a whole plant or a part of a plant, which comprises a DNA molecule as described above. The DNA molecule will be preferably operatively linked to a promoter and, optionally, to one or more additional control elements (such as an enhancer), and it is integrated in its genome.

## BRIEF DESCRIPTION OF THE FIGURES

**Figure 1.** Morphology of SVBS-1801: A) vegetative cells (VC); B) bacterial cells containing the fully formed spore and crystal just before cell lysis; and C) spores and crystals already released as a result of the lysis of the bacterial cell.

**Figure 2.** Results of the comparative analysis of the beta-exotoxin production by the positive control strain BT2 (strain Bt HD2) and the sample strain BT4 (SVBS-1801).

**Figure 3.** SDS PAGE of ABTS-351 (Dipel®), ABTS-1857 (Xentari®) and SVBS-1801 purified crystals (Lanes 2, 3 and 4, respectively). A molecular weight marker is included (lane 1).

**Figure 4.** Nucleotide sequence alignment of the SVBS-1801 *cry1Ab24-like* gene (Sbjct) and its most similar reference gene *cry1Ab24* (Query).

**Figure 5.** Nucleotide sequence alignment of the SVBS-1801 *cry1Ja1-like* gene (Sbjct) and its most similar reference gene *cry1Ja1* (Query).

**Figure 6.** Amino acidic sequence of the SVBS-1801 Cry1Ab24-like protein (Sbjct) and its most similar reference protein Cry1Ab24 (Query), where it can be observed that the reference protein Cry1Ab24 has 26 additional amino acids between the position 793 and 794 of the sequence of the SVBS-1801 Cry1Ab24-like protein sequence (SEQ ID NO:2)

**Figure 7.** Amino acidic sequence of the SVBS-1801 Cry1Ja1-like protein (Sbjct) and its most similar reference protein Cry1Ja1 (Query).

**Figure 8.** Mortality percentages for *S. frugiperda* larvae collected 12, 24, or 36 hours after treatment with the ABTS-1857 and SVBS-1801 strains using 100 mg/L of the spore/crystal mixture.

## DETAILED DESCRIPTION OF THE INVENTION

The present invention is based on the finding, isolation and characterization of strain SVBS-1801 of *B. thuringiensis*, which has proven insecticidal activity against *S. frugiperda* larvae. It contains at least 6 *cry* genes and 3 *vip* genes, respectively.

Two of the *cry* genes found in this strain (those whose coding sequences match SEQ ID NO:1 and SEQ ID NO:7, respectively) can be considered genes which have not been previously isolated and sequenced, because they are not identical in their coding sequence to any known *cry* gene, so that the determination of the presence in the genome of any one of said two genes or preferably both of them, can be used to identify a *B. thuringiensis* bacterium as a bacterium of this strain. However, their degree of identity with reference known genes, *cry1Ab24* and *cry1Ja1,* respectively, is high (98% in the first case and 94% in the second one), so that they have not been considered strictly different genes and they have been named *cry1Ab24-like* and *cry1Ja1-like,* respectively.

The strain SVBS-1801 possesses a set of 6 *cry* genes and 3 *vip* genes different from those of other known varieties. Each of the mentioned sets of genes can be considered unique and characteristic of this strain, not having been described in other B. *thuringiensis* strains.

Therefore, the determination of the simultaneous presence of the set of 6 cry genes or of the 3 vip genes or, preferably, of the 9 genes, is useful to identify the strain and distinguish it from other Bt strains. Any method for identifying a *B. thuringiensis* strain which comprises the determination of the presence of any (or both of them) of the genes of SEQ ID NO:1 and SEQ ID NO:7, and/or the simultaneous presence of the set of 6 *cry* genes of SEQ ID NO:1, SEQ ID NO:3, SEQ ID NO:5, SEQ ID NO:7, SEQ ID NO:9 and SEQ ID NO:11 and/or the simultaneous presence of the set of 3 *vip* genes of SEQ ID NO:13, SEQ ID NO:15 and SEQ ID NO:17 or the simultaneous presence of the two set of genes, concluding that the strain is the *B. thuringiensis* strain SVBS-1801 if the mentioned genes for each alternative are present, are mentioned, but do not form part of the invention. The detection of the expression of at least one of the proteins encoded by SEQ ID NO:1 and SEQ ID NO:7, that is, the proteins of SEQ ID NO:2 and SEQ ID NO:8, respectively, can be also used to identify the SVBS-1801 strain. Analogously, the strain can be identified by determining the expression of the polypeptides encoded by the sets of genes mentioned above, that is, determining, and confirming, the expression of at least all the polypeptides of the group of polypeptides whose amino acid sequence is SEQ ID NO:2, SEQ ID NO:4, SEQ ID NO:6, SEQ ID NO:8, SEQ ID NO:10 or SEQ ID NO:12 (the set of Cry proteins encoded, respectively, by the set of genes of SEQ ID NO:1, SEQ ID NO:3, SEQ ID NO:5, SEQ ID NO:7, SEQ ID NO:9 and SEQ ID NO:11, whose simultaneous presence in a Bt genome is characteristic of the Bt strain of the present invention), and/or the expression of at least all the polypeptides of the group of polypeptides whose amino acid sequence is SEQ ID NO:14, SEQ ID NO:16 or SEQ ID NO:18 (the set of Vip proteins encoded, respectively, by the set of genes of SEQ ID NO:13, SEQ ID NO:15 and SEQ ID NO:17 whose simultaneous presence in a Bt genome is characteristic of the Bt strain of the present invention) or all the polypeptides of the group of polypeptides whose amino acid sequence is SEQ ID NO:2, SEQ ID NO:4, SEQ ID NO:6, SEQ ID NO:8, SEQ ID NO:10, SEQ ID NO:12, SEQ ID NO:14, SEQ ID NO:16 or SEQ ID NO:18, can be used to identify the presence of the Bt strain SVBS-1801 of the present invention.

As it is used in the present application, "identifying the presence" can be understood as determining the presence of the Bt strain SVBS-1801 in a sample or identifying that a certain strain is the Bt strain SVBS-1801 of the present invention. As genes *cry1Ab24-like and cry1Ja1-like* are novel and different even from the most similar known genes *(cry1Ab24* and *cry1Ja1)* and their simultaneous presence is the genome of a Bt strain has not been described previously, they can be also used to define the strain of the present invention particularly *cry1Ja1-like* or the protein encoded by it, because it exhibits activity against *S. frugiperda.* As commented above, neither the simultaneous presence of the set of *cry* genes of SEQ ID NO:1, SEQ ID NO:3, SEQ ID NO:5, SEQ ID NO:7, SEQ ID NO:9 and SEQ ID NO:11 nor the simultaneous presence of the set of *vip* genes of SEQ ID NO:13, SEQ ID NO:15 or SEQ ID NO:17 have been previously reported for a Bt strain, so that any of such two set of genes, or the simultaneous presence in the genome of all of them, can be used, too, to define the Bt strain of the present invention by means of features that are characteristic of said strain and that were not obvious or expected to find, particularly in a strain not created in a laboratory but isolated from an environmental source. This comment of the utility for defining the strain by means of the specific combinations of *cry* and *vip* genes that can be found in about can be extended to the proteins encoded by the mentioned genes.

Strain SVBS-1801 which was deposited on Colección Española de Cultivos Tipo (CECT) on 14 November 2018, and received the accession number: CECT 9753, is one aspect of the present invention.

Regarding the proteins encoded by the *cry* and/or *vip* genes found in the SVBS-1801 Bt strain, all identified Cry proteins may be present in the crystal, while Vip proteins should be found in the supernatant of the growth medium after being secreted during the growth phase.

It is noteworthy that the toxicity spectrum of this strain includes *S. frugiperda,* which is not satisfactorily controlled by the currently biological available products in the market. No previous knowledge indicated that a strain showing the combination of genes mentioned above could have activity against *S. frugiperda,* and, specially, there was no previous knowledge indicating that a strain with such particular combination of genes could show higher activity against *Spodoptera frugiperda* than the strains used to prepare the commercial insecticides that are currently used against this pest, as can be shown in the Examples below. And this is particularly remarkable since the finding of Bt strains with high activity against a specific species, based on genetic and molecular characteristics, is a difficult and unobvious task. As discussed above, such difficulties arise not only from the fact that Cry proteins with high homology might show different activities and specificities against pests, but also, and very especially, because it is unpredictable which combinatorial effects might occur among the different toxins produced by a Bt strain, its type (synergistic or antagonistic), and the degree of influence that the combinatorial effects might have on the insecticidal activity of a Bt strain and its fermentation products. In fact, the present application discloses synergistic effects among three of the Cry proteins produced by the SVBS-1801, given rise to an increase of activity with regard to the expected activity resulting from the simple combination of the activity of each one of said proteins, which is a combinatorial effect that was totally unexpected.

Thus, the present invention provides Bt strain, SVBS-1801, isolated from an environmental source and that lacks expression of β-exotoxin, that can be used in the control of *Spodoptera frugiperda* pests. Also encompassed by the invention are methods for preparing compositions comprising mixture of crystals and spores of SVBS-1801 (the kind of compositions that are usually used to prepare commercial insecticides), methods for preparing purified crystals of the strain, compositions comprising different components produced by the strain or resulting from its growing: mixtures of crystals and spores where other components might be also present such as Cry proteins not included in parasporal crystals, vegetative bacteria or Vip proteins, composition with Cry proteins not included in crystals, purified crystals or even composition with purified crystals or with a mixture of crystals and spores where a portion of the supernatant obtained when the liquid culture medium of growing the strain is centrifuged to separate the crystals and spores from the liquid where the bacteria have been growing. The use of the strain, and the compositions comprising it or comprising crystals, Cry proteins, and/or Vip protein, produced by the strain or resulting from its growing in a liquid fermentation medium, as insecticides or for preparing insecticides, particularly against *S. frugiperda,* are also encompassed by the scope of the present invention.

Disclosed, but not forming part of the invention, are the new genes of SEQ ID NO:1 and SEQ ID NO:7, the proteins encoded by SEQ ID NO:1 and SEQ ID NO:7 (namely the proteins of SEQ ID NO:2 and SEQ ID NO:8), and proteins comprising a polypeptide with at least a 99% of identity with SEQ ID NO:2 or SEQ ID NO:8 and with insecticidal properties. Mentioned, but not forming part of the invention, are mutant strains that may derive from SVBS-1801, particularly the recombinant bacterial strains obtained with the mentioned genes (that can be obtained, for instance, following the procedure described in Example 4) or, even, recombinant cells of yeasts, fungi or even plant cells, as well as vectors that allow the expression of the mentioned proteins or the insertion of the genes of SEQ ID NO:1 or SEQ ID NO:7 in its genome, such as plasmids or recombinant viruses In the case of plant cells, the insertion not only of the gene of SEQ ID NO:7, but also of the gene of SEQ ID NO:3 and SEQ ID NO:5, or the introduction of expression vectors of all the three genes, might be of interests, since the proteins expressed by said three genes have shown activity against *S. frugiperda* when tested separately, but they have also shown synergistic effects when tested in combination.

*B. thuringiensis* strain SVBS-1801 presents the following characteristics:

Growth: *B. thuringiensis* SVBS-1801 grows preferably at 28-30°C in salt-rich media such as the CCY medium described and used in Example 1. In such conditions, spores germinate and produce a bipyramidal crystal that can be observed under an optical microscope (Fig. 1). When grown in solid media (CCY or Luria-Bertani (LB) broth, supplemented with agar), the morphology of the colonies is very similar to that of other Bt strains, rounded with an irregular edge and a waxy appearance. SVBS-1801 can be grown at an industrial scale when using in the appropriate media: salt-rich media such as the CCY medium and temperature conditions, which may vary between 15-45 °C, although the optimal range goes from 28 to 30°C. A continuous agitation (220 rpm, for instance) is also required.

Absence of exotoxin. As explained above, some Bt strains may produce a secreted exotoxin, β-exotoxin, which can impair the RNA polymerase and cause cell mortality to a wide variety of species and preventing the use of the strain, or product based on it, as an insecticide. As shown in Example 2, SVBS-1801 does not produce β-exotoxin during the fermentation process. For this reason, it is considered to have a wide and unspecific range of action.

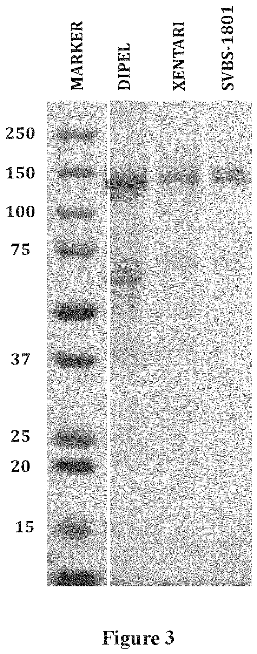

Bt toxins/crystal proteins: The Cry proteins synthetized by the strain SVBS-1801 of the present invention, aggregate and crystalize during the sporulation phase, giving rise to parasporal crystals, which are named simply "crystals" in the present application. Their pattern of migration in SDS-PAGE gels can be described as two bands in close proximity to each other with an approximate weight of 120-135 kDa (Cry1Ab, Cry1Da3, Cry1Ea7 and Cry1Ja1) (Fig. 3). Crystal proteins from the commercial products based on the strain ABTS-351 (Bt strain of serovar *kurstaki),* Dipel®, (Cry1Aa, Cry1Ab, Cry1Ac y Cry2Aa) and on the ABTS-1857strain (Bt serovar *aizawai),* XenTari® (Cry1Aa, Cry1Ab, Cry1Ca, and Cry1Da) migrate similarly, with bands of a comparable weight to those of strain SVBS-1801 (130-135); however, the total pool of proteins produce unique migration patterns for each strain. It is known that Dipel® additionally generates a band of about 70-75 kDa that corresponds to protein Cry2Aa.

Insecticidal capacity. As mentioned above, it is noteworthy that SVBS-1801 crystal proteins are 5.7 and 1235.4 fold more effective against *S. frugiperda* than their XenTari® and Dipel® counterparts, respectively, as indicated in the Examples of the present application. Thus, products based on SVBS-1801 strain (that is, compositions comprising vegetative cells, bacterial cells containing spores, spores, crystals, Cry proteins or Vip proteins, or combinations thereof), of SBVS-1801, can be used as an insecticide or for the preparation of insecticides. It is particularly preferred that the active ingredient used to prepare such insecticide composition is a combination of spores and crystal proteins obtained after subjecting to a precipitation process (preferably, by centrifugation) the content of a culture (understanding as such the culture medium and the grown bacterial cells, remains of lysed cells and bacterial products such as spore, crystals, bacterial proteins not forming part of crystals and other bacteria produced compounds) where SBVS-1801 bacteria have been grown until they have undergone sporulation, lysis and release of their crystals and spores, as in Example 1: the equivalent to the active ingredients used for the preparation of Bt based commercial insecticide products such as Dipel® or Xetari®.

Such active ingredient (AI) (strain SVBS-1801) and the mixture of crystals and spores usually included in its formulation, can be acquired as explained in Example 1 or by any analogous method. It is as aspect of the present invention a method which comprises the steps of:

a) growing SBVS-1801 bacteria until more than 50% of the bacterial cells have undergone sporulation, have lysed and released their crystals and spores to the culture medium;
b) purifying the crystals and spores by precipitating them from the culture medium in which they are in suspension, preferably by centrifugation, and collecting the precipitate;
c) optionally, washing the precipitate with a protease inhibiting solution and provoking again the sedimentation of the crystals and spores;
d) storing the purified crystals and spores either:

a. at room temperature, after having lyophilized the precipitate obtained in step b) or c), or
b. at a temperature of the interval of -18°C to -20°C or lower, after having resuspended the precipitate obtained in b) or c) in water or in an aqueous solution.

Such AI contains a mixture of crystals and spores that can be reformulated as a composition in the form of a concentrated liquid suspension or as a wettable powder, which composition can also contain one or more excipients or additives, particularly those commonly use in the agricultural field. Any of such reformulated forms could be applied to the fields and can be used as an insecticide, preferably to protect plants. As commented above, it can be used to control pests, for example *S. frugiperda.*

But it is remarkable that one of the advantages of the strain SVBS-1801 is that the present inventors have found that different compositions with different elements produced by the strain SVBS-1801 of the present invention also have activity against *S. frugiperda,* which observed activity, in some of the treatments, is even higher than the activity of the mixture of crystals and spores obtained by the method of the invention mentioned above. Adding a portion of the supernatant obtained by the same method to the mixture of crystals and spores gives rise to a significant increase in the activity, as it is shown by the $LC_{50}$ value (see Table 5). The assays carried out with purified crystals, free of spores, also show an increase in activity with regard to the mixture of crystals and spores, which is even higher (lower values of $LC_{50}$) when the crystals are mixed with the same supernatant. These results facilitate the use of different compositions for the preparation of AIs, each one with different components produced or derived from the strain SVBS-1801, namely: a) a mixture of spores and crystals; b) a mixture of spores, crystals and a portion of the supernatant obtained by performing steps a) and b) the method of preparing a mixture of spores and crystals of strain SBVS-1801 which is an aspect of the present invention; c) crystals; d) crystals and a portion of the supernatant obtained by performing steps a) and b) the method of preparing a mixture of spores and crystals of strain SBVS-1801 which is an aspect of the present invention.

The purified crystals can be prepared by the method used in Example 8, which is also an aspect of the invention, but also by any other method known by those skilled in the art, such as the ultracentrifugation on a sucrose gradient (Thomas and Ellar, 1983), which will usually be a discontinuous sucrose gradient (79-67%), where the crystal are trapped in the interphase and the spores can be collected from the pellet. A crystal preparation, or a composition comprising SBVS-1801 crystals, will be considered free, or essentially free, of spores if no spore is observed by confocal microscopy of samples of the preparation or composition, as described in Example 8 of the present application, or when the presence of spores is checked by an analogous method.

When spores are not desired in the composition, the purification of the crystals can be replaced by the addition of a germicide. The germicide can also be added to compositions with purified crystals, in order to guarantee the absence of spores by eliminating any possible spore that might remain after the crystal purification. The absence of spores can be checked by inoculating dilutions of the suspension of purified crystals in nutritive agar plates and verifying that no colony grows, and, consequently, the crystals are completely free of spores and its purity is maximal. If added, the germicide will be preferably selected to be compatible with the ecological properties of the strain and compositions of the present invention.

The results obtained in Example 8, where both the mixture of spores and crystals and the purified crystals show activity against *S. frugiperda,* indicates that it is possible to envision the preparation of mixtures of spores and crystals with different proportions of said elements (such as, for example, 50:50 or 80:20 crystals:spores, or any other), being possible to use all of them for the preparation of the active ingredient of an insecticide. Thus, for instance, one must consider comprised within the present invention those compositions with contains spores and crystals, where the crystals have been obtained by a method that give rise to crystals are not completely purified but they are only, for instance, 80% purified (or any other percentage such as 85%, 90% or 95% purified), so that the preparation will be, indeed, a mixture of crystals and spores also comprised within the scope of the present invention.

Also in Example 8, but in section 8.3., assays about the toxicity of the individual Cry proteins synthesized by the strain of the present invention are shown. In accordance with the results obtained (see Tables 6 and 7), the insecticidal activity of the parasporal crystals of the strain SVBS-1801 could be attributed to the Cry proteins Cry1Da3, Cry1Ea7 and Cry1Ja1-like, which are toxic when tested individually against *S. frugiperda* larvae. The activity of the protein Cry1Ja1-like is remarkable, because it is encoded by one the genes disclosed in the present application, *cry1Ja1-like* (SEQ ID NO:7), which shows certain differences with the already known gene *cry1Ja1,* as discussed above. Particularly noteworthy is the fact that the assays carried out with a mixture of the three proteins show an increase of activity with regard to the same concentration of any of the three proteins, indicating a combinatorial synergistic effect in the mixture of the three proteins, that has been confirmed in the same section of Example 8. The identified synergistic effect was not expected from the teachings of previously published documents and supports that it was not expectable that a strain with the combination of genes of SVBS-1801 could have high activity against *S. frugiperda,* particularly against larvae, which activity is higher than that of the most potent commercial insecticide currently on use against this strain, Xentari® (see Examples 9 to 11) and to that of the most potent strain described by Capalbo et al. (2001), to the extent that the lack of explanation about the methodology used to measure the $LC_{50}$ in the assays reported in that article allows a comparison.

The results found in the assays with Cry proteins make also possible to consider compositions comprising a mixture of proteins Cry1Da3, Cry1Ea7 and Cry1Ja1-like (SEQ ID NO:4, SEQ ID NO:6 and SEQ ID NO.8, respectively) as possible compositions, particularly if they are in equimolar proportions. Such compositions can also be of use as insecticides or for the preparation of insecticides against *S. frugiperda.*

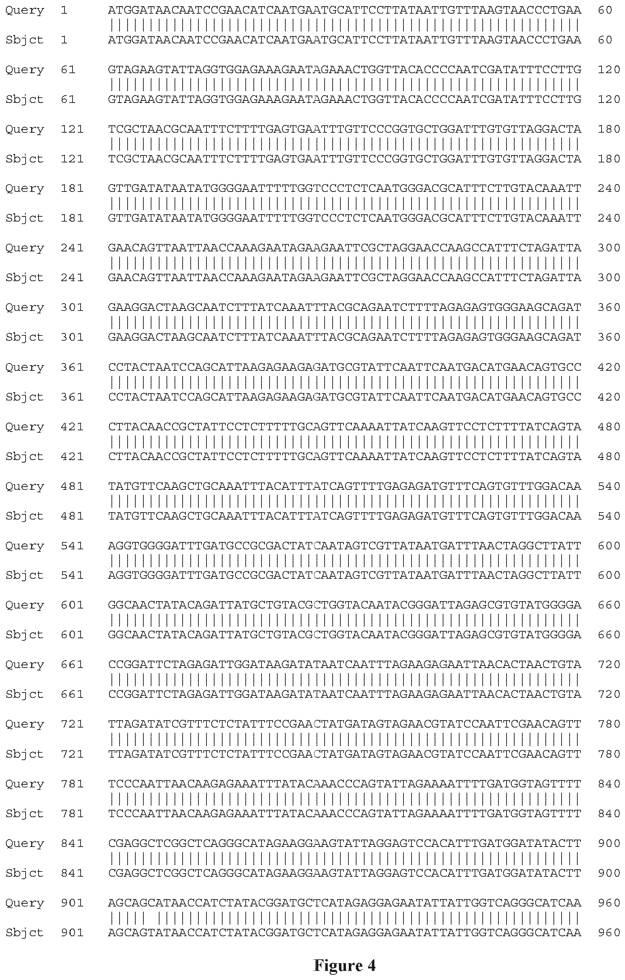

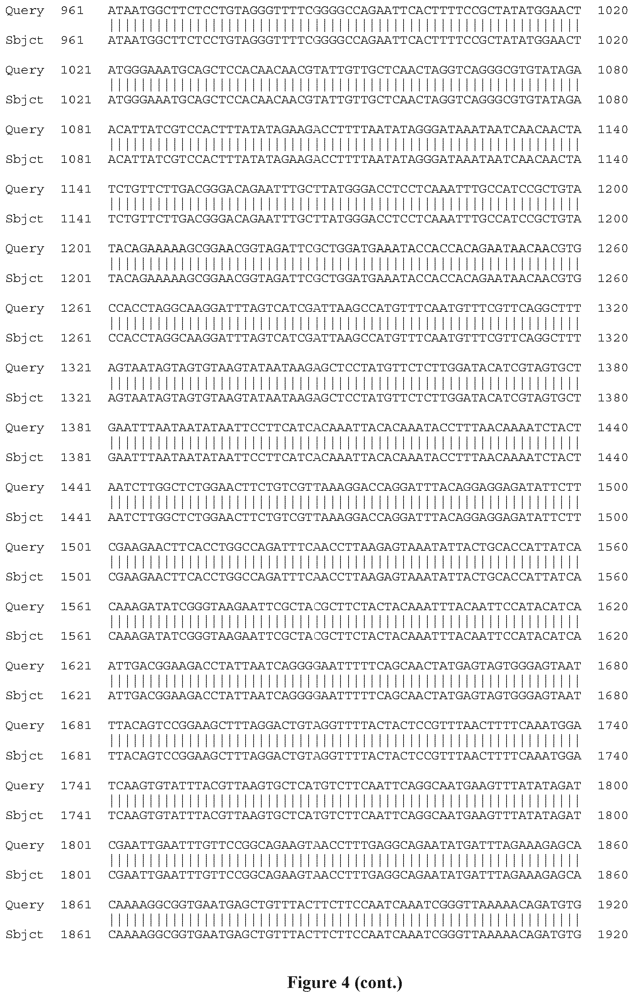

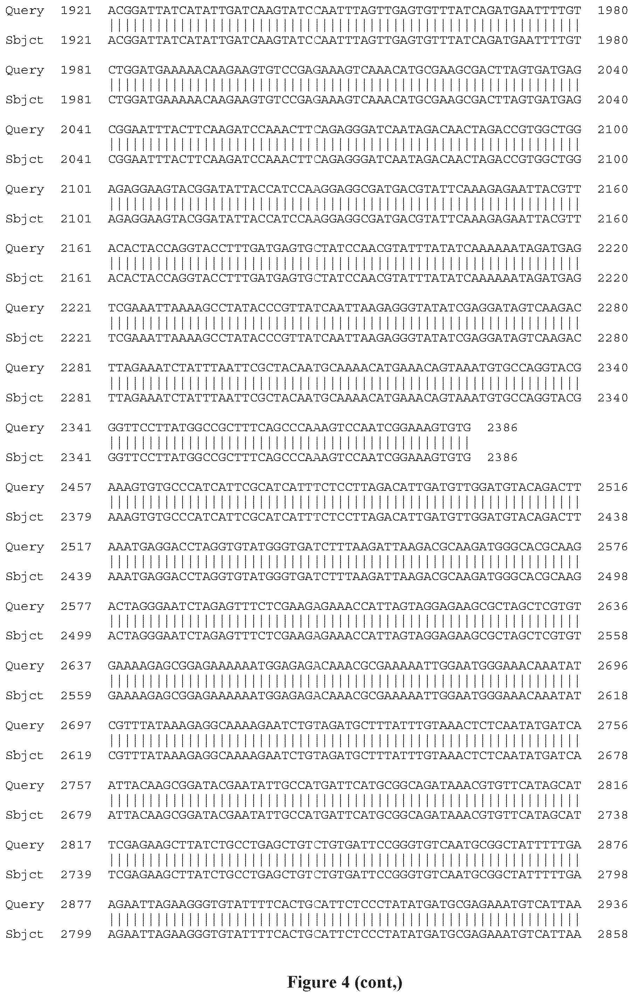

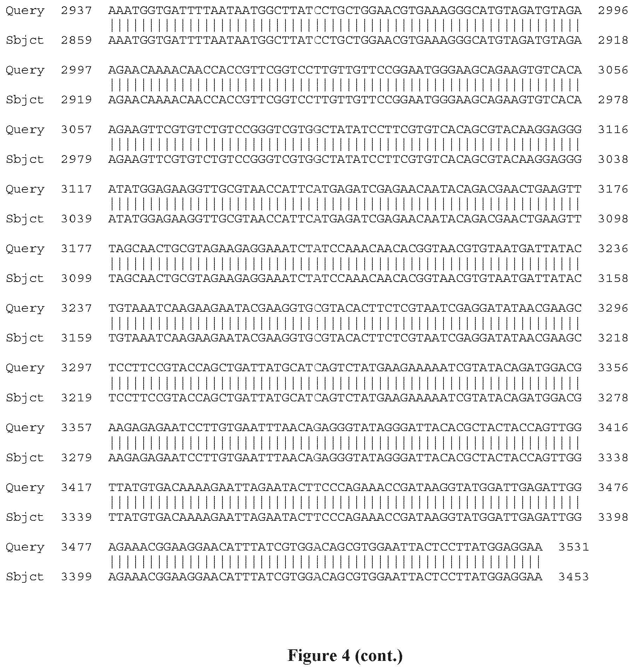

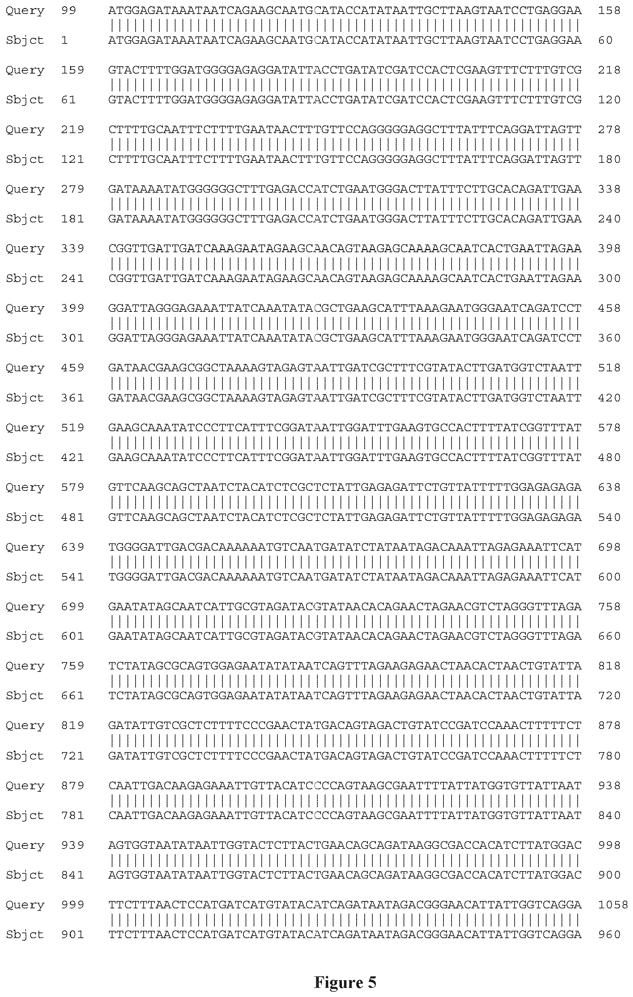

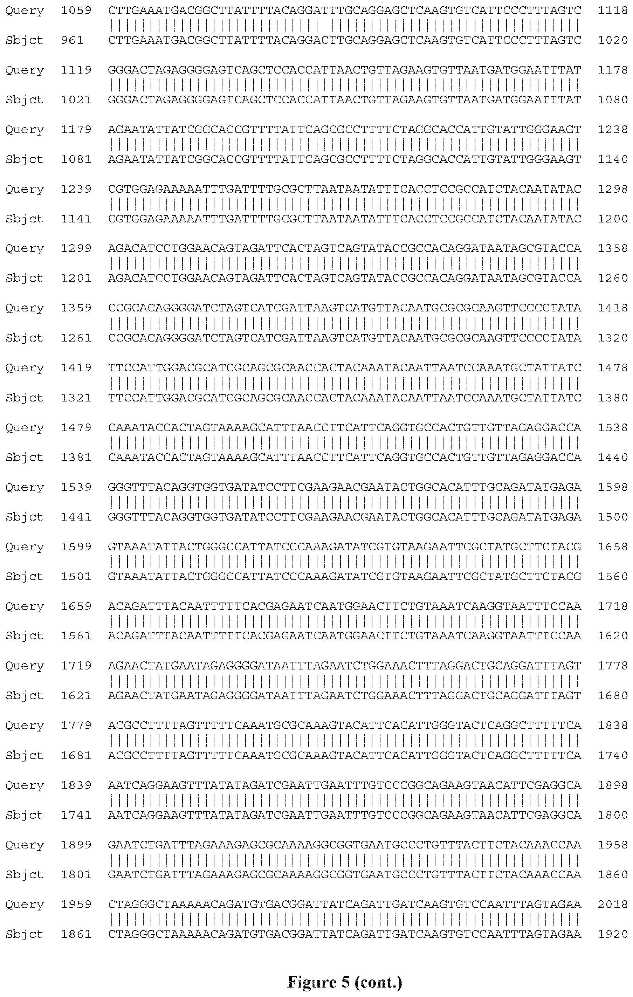

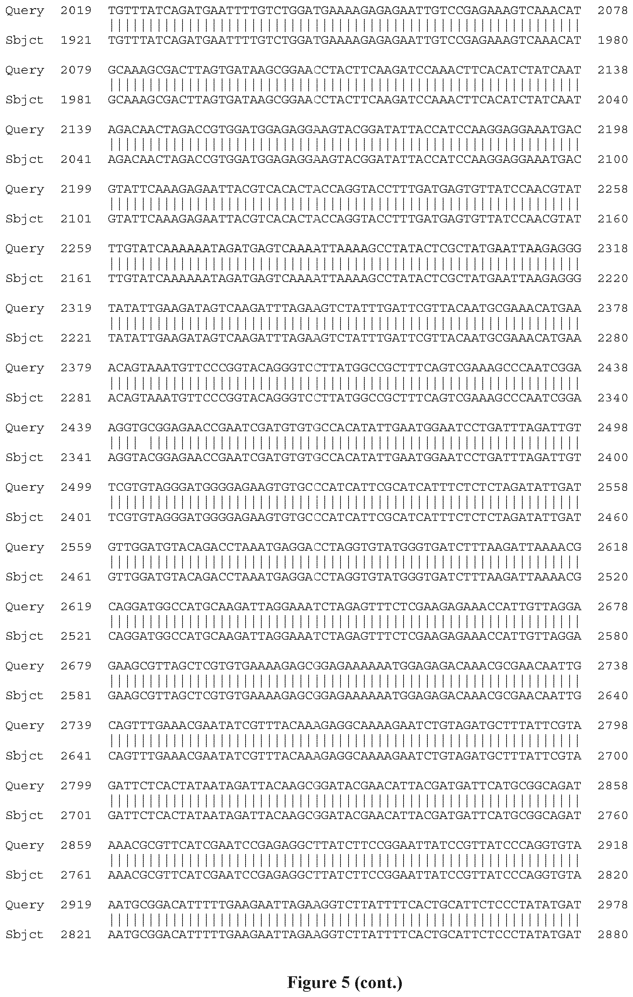

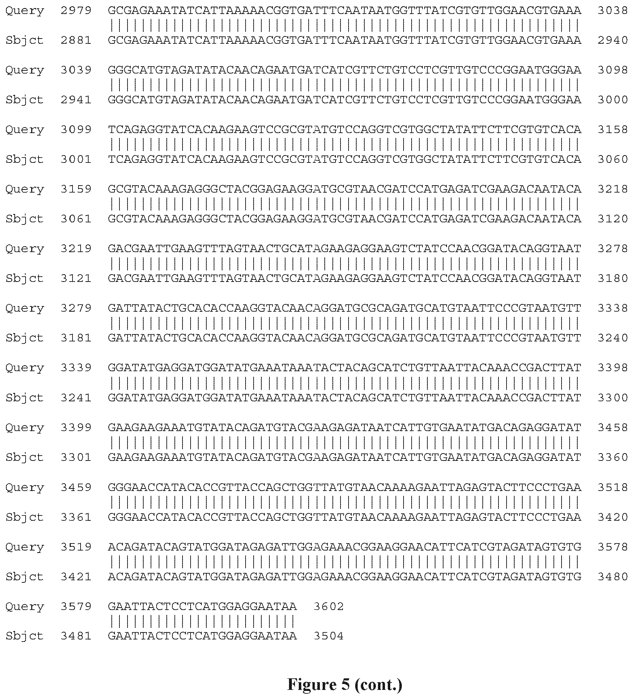

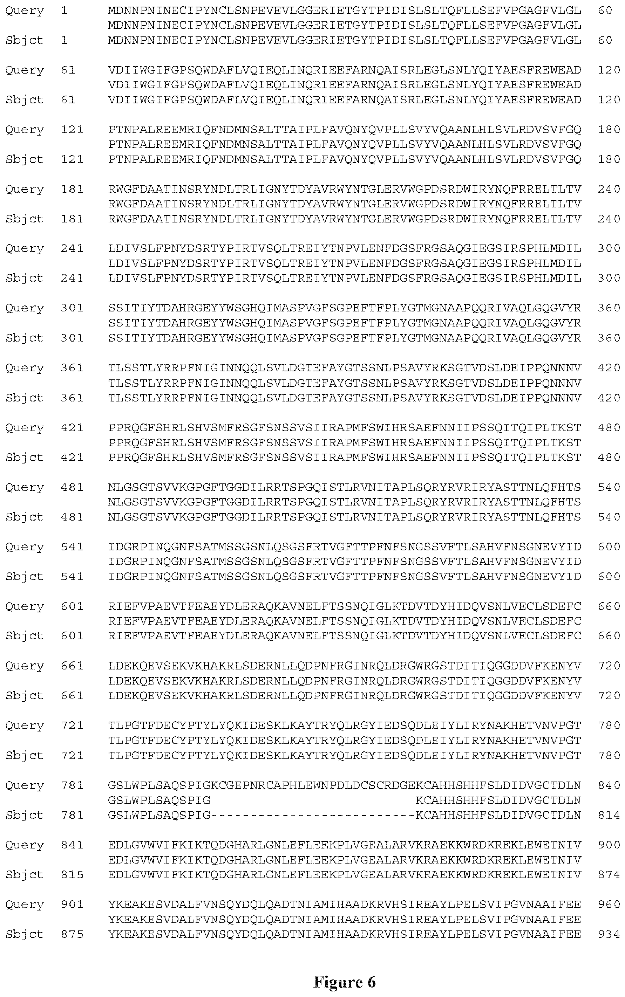

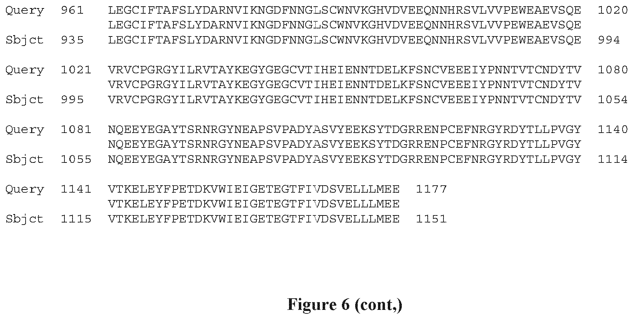

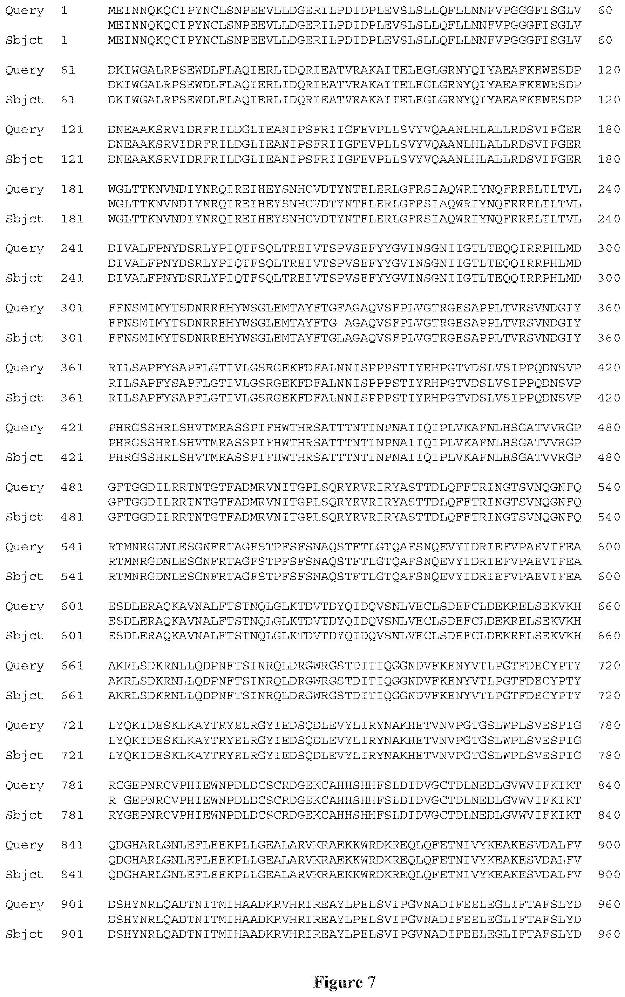

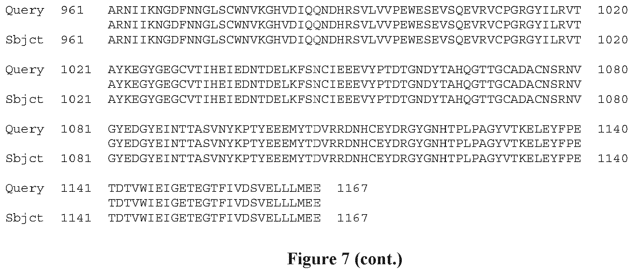

Insecticidal genes. As specified in Tables 2 and 3, the genome (chromosome + plasmids) of SVBS-1801 contains at least 6 different *cry* genes and 3 different *vip* genes, respectively. Two of the *cry* genes (*cry1Ab24-like* and *cry1Ja1-like*) are not completely identical to the most similar reference genes whose coding sequence is publicly available, *cry1Ab24* (GenBank accession number HQ439778) and *cry1Ja1* (GenBank accession number L32019). Therefore, the determination of the presence of at least one of said genes, which are different from other known Bt genes, might be enough to identify the strain SVBS-1801. To that aim, it is noteworthy that gene *cry1Ab24-like* presents a coding sequence (SEQ ID NO: 1) 78 nucleotides shorter than the coding sequence of *cry1Ab24* (see Fig. 4), which results in the protein encoded by *cry1Ab24* being 26 amino acids longer between positions 793 and 794 of the polypeptide encoded by SEQ ID NO:1 (see the alignment of Fig. 6). Such difference in size and sequence, in addition to the presence of T instead of C in the position 906 of SEQ ID NO:1, can be useful to differentiate *cry1Ab24-like* from *cry1Ab24* (GenBank accession number HQ439778). Regarding *cry1Ja1-like,* as indicated in Figures 5 and 7, its coding sequence is identical in length to the coding sequence of *cry1Ja1* (GenBank accession number HQ439784) but there are two different nucleotides between both sequences. The reference gene *cry1Ja1* presents a T instead of a C in position 987 of SEQ ID NO:7 and a G instead of an A in position 2345 of SEQ ID NO:7, respectively. These are variations that can be used to differentiate the sequences belonging to strain SVBS-1801.

The identification of the SVBS-1801 strain can be done by determining the presence of the set of six *cry* genes found in SVBS-1801 by the present inventors, as indicated in Table 2 (*cry1Ab24, cry1Da3, cry1Ea7, cry1Ja1, cry1Nb1, cry2Ad1,* or by the presence of the set of three *vip* genes found in SVBS-1801 by the present inventors, as indicated in Table 3 (*vip1Ca1, vip2Ac1, vip3Af3*) or, more preferably, by the simultaneous presence of at least the nine insecticidal genes, the set of six *cry* genes and the set of three *vip* genes. The identification of the presence of the selected genes can be done as in Example 3, with a complete DNA sequencing, contig assembling and gene prediction of the assembled contigs, or by any other method; analogously to the method used in Example 3, or by amplifying the gene fragments by PCR, as in Example 4, using for instance the same pairs of primers. The presence of a gene will require at least 95% of identity with the sequence of the gene in the database btnomenclature (http://www.btnomenclature.info, which database contains *Bacillus thuringiensis* genes that are also present in Genbank and which can be accessed with the same accession number), as it might correspond but it will be preferred that, when gene *cry1Ab24* or the gene *cry1Ja1* is one of the genes used to identify a strain as the SVBS-1801 strain, the presence of gene *cry1Ab24* or the gene *cry1Ja* is considered positive when the sequence of the corresponding DNA fragment is that one of SEQ ID NO:1 or that of SEQ ID NO:7, respectively, that is, the variants of said genes found in the strain SVBS-1801.

Genes and their uses. Strain SVBS-1801 may be used as a source of genes with a wide variety of applications in other fields like medicine, veterinary, biotechnology, etc. and they may be transferred, alongside their biological properties, to other organisms for different purposes, but do not form part of the invention. Thus, the genes whose coding sequences are those of SEQ ID NO:1, SEQ ID NO:3, SEQ ID NO:5, SEQ ID NO:7, SEQ ID NO:9, SEQ ID NO:11, SEQ ID NO:13, SEQ ID NO:15 and SEQ ID NO:17, are products of interest by themselves since they encode insecticidal proteins. Said genes (or the DNA fragments or regions where they are comprised) can be used, for instance, to obtain transgenic plants with insect resistance (analogously to the plants described in documents such as international patent application WO1995/024493 or United States application published as US20080016596A1) or nematode resistance (analogously to the plants described in documents such as international patent application WO2010/027793), for instance by using the techniques described in such documents, which are well known for those skilled in the art. The same DNA fragments or regions, can be used to obtain, for instance, non-human transgenic animals or recombinant cells (animal, plant, fungal or bacterial cells), provided that they are part of a construct where they are operatively linked to the appropriate control elements (promoters, enhancers...), depending on the conditions when they are desired to be expressed or, in the case of transgenic plants or animals, also depending on the tissues where their expression is desired. Such control elements are well known by those skilled in the art. The construct can be integrated in the genome or, particularly for the case of bacterial recombinant cells, can be part of an expression vector such as a plasmid, that can be introduced in the cell by methods well known by those skilled in the art. Such transformed cells or higher organisms can be useful to give rise to plants resistant to pest such as nematodes or insects (such as, for instance, insects of the *Spodoptera* genus, like *S. frugiperda)* or can be used for any other purposes (medical, veterinary, research in general...).

Thus, discussed, but not forming part of the invention, is any nucleic acid molecule comprising a fragment whose nucleotide sequence is: a) the sequence of any of the genes (SEQ ID NO:1 and SEQ ID NO:7), b) any nucleotide sequence which encodes a polypeptide comprising the amino acid sequence of any of the proteins encoded for the mentioned genes (the amino acid sequences of SEQ ID NO:2 and SEQ ID NO:8, which are proteins) , as well as the nucleotide sequences which also encodes proteins comprising a polypeptide fragment sequence of SEQ ID NO:2 or SEQ ID NO:8, but which are different from SEQ ID NO:1 and SEQ ID NO:7, due to the degenerative nature of the genetic code) or proteins comprising a polypeptide fragment which is similar as those mentioned in sequence and activity but that they cannot be classified as different proteins, that is: c) a nucleotide sequence which encodes a polypeptide having a sequence fragment of at least 98,5% (or at least 99%, 99,5%, 99,90%, 99,95% or 99,99%) of identity with the amino acid sequence of SEQ ID NO:2 or at least 99,85 % (or at least 99,90%, 99,95% or 99,99%) of identity with the amino acid sequence of SEQ ID NO:8 and with pesticidal activity (insecticidal, nematocide, or both of them). The pesticidal activity can be shown by the polypeptide as such or by a cleavage form thereof, as happens usually with Cry proteins, whose insect toxic activity appears after the protein is cleaved in two fragments in the insect gut.

Accordingly, the constructs containing any of such nucleic acid molecules and, particularly, the expression vectors (plasmids or recombinant viruses, for instance) where the nucleic acid molecules are operatively linked to a promoter and, optionally one or more control sequences, selected depending on the conditions where the protein is desired to be expressed and/or depending on the desired level of expression, are mentioned, but do not form part of the invention.

Also discussed, but not forming part of the invention, is any cell (bacterial, fungal, yeast, or plant cell) which comprises a nucleic acid molecule as a heterologous DNA fragment, or which comprises an expression vector thereof. The nucleic acid fragment can be integrated in the genome (or, in the case of bacteria, in the bacterial chromosome) or can be part of other nucleic acid elements such as plasmids or non-integrative viruses. The bacterial cell can even come from another *B. thuringiensis* strain, for instance one that does not naturally contain one or more of the genes of: SEQ ID NO:1, SEQ ID NO:3, SEQ ID NO:5, SEQ ID NO:7, SEQ ID NO:9, SEQ ID NO:11, SEQ ID NO:13, SEQ ID NO:15 or SEQ ID NO:17 and whose insecticidal or nematocidal activity can be increased or modified by the expression of one or more of the proteins matching sequences SEQ ID NO.2 and SEQ ID NO:8. This may be achieved by transforming the strain with a cassette comprising a DNA fragment of SEQ ID NO:1 or SEQ ID NO:7 (or any other nucleotide sequence encoding the polypeptide of sequence SEQ ID NO:2 or SEQ ID NO:8) operatively linked to a promoter that allows the expression of the polypeptide in the strain, either constitutively or under selected conditions. Additionally, those recombinant *B. thuringiensis* cells which, after the modification of its genome (chromosome and plasmids included), contain at least one of the set of genes whose coding regions match sequences SEQ ID NO:1, SEQ ID NO:3, SEQ ID NO:5, SEQ ID NO:7, SEQ ID NO:9 and SEQ ID NO:11 or by SEQ ID NO:13, SEQ ID NO:15 and SEQ ID NO:17, or both sets of genes, because it will have become a Bt strain with the characterizing genome of the strain SVBS- 1801.

Also mentioned are plants that comprise the DNA molecule discussed above, particularly when the DNA molecule or fragment is part of a construct where it is operatively linked to one or more control elements (promoters, enhancers, termination sequences...) which allow or facilitate the expression of the polypeptide encoded by the DNA molecule in one or more tissues of the plant or animal, constitutively or under certain conditions, including different times in the development process of the plant or animal. For transgenic plants, (as reviewed, for instance, by Shah et al., 2015) constitutive promoter such as the cauliflower mosaic (CaMV) 35S are commonly used, but it could be also interesting to be able to restrict the expression of the desired transgenic gene by the use of inducible promoters, such as the wound inducible promoter of tomato HMGR2 gene, the PR-1a promoter (induced by pathogen infection and chemical elicitors such as benzothiadiazole) or the maize In2-2 (inducible gene 2-2) promoter (which is induced by benzene sulfonamide safeners which act as herbicide tolerance increasing agrochemicals of plants). Other inducible elements of interest can be the cis-acting regulatory sequences that spatially regulate the expression of genes at different parts, or the two upstream activating sequences encoded by the promoter of the phaseolin gene, UAS1 (-295 to -109) and UAS2 (-468 to -391). Besides, targeted expression to specific tissues is becoming more important for the future development of value-added crops, so that promoters like the one of the ACC oxidase gene which, ranging from 21966 to 21159 bp from the start of transcription, is able to drive tipening-specific expression of a gene in fruits such as the tomato fruit, as it has been also reviewed by Shah *et al.,* 2015.Other different promoters and control elements can be found in the mentioned revision and in other documents available for those skilled in the art.

The invention will be now explained with further detail with the examples and drawings included below.

**EXAMPLES**

The following section provides examples that illustrate the procedures in which the invention may be applied to facilitate the industrial exploitation of SVBS-1801.

**EXAMPLE 1.- Isolation and growing of Bt strain SVBS-1801**

1.1.Isolation of the strain SVBS-1801

The SVBS-1801 strain was obtained from a sample of agricultural soil from the outskirts of the city of Badajoz, used in the construction of the gardens of the urbanization "Residencial San Gabriel" of this city. Said soil samples were collected in December 2016 and kept under refrigeration conditions (4°C) until the moment of processing (January 2017). To carry out the isolation and identification of the SVBS strain, samples were collected from the soil, after removing the first layer, mixed with distilled water and heated at 70°C for 15 min. A volume (15 μl) of a ten-fold dilution was plated onto CCY medium and incubated at 28°C for 72h. Positive Bt colonies were checked by phase contrast microscopy (Iriarte *et al.,* 1998).

1.2.Growth in a culture medium that promotes sporulation

A sample of SVBS-1801 is grown in 1L of CCY medium (Stewart *et al.,* 1981). CCY is a minimum medium that promotes sporulation, which ultimately leads to the formation of crystals and spores (Fig. 1). It is prepared by mixing buffer ingredients (Solution 1) and carbon source ingredients (Solution 2) in the appropriate proportions. Once the mix is complete, the medium is sterilized inside an autoclave and supplemented with 1ml of a previously filtered salt solution (Solution 3).

Table 1. CCY medium (Stewart *et al.,* 1981)

| Solution 1. Buffer solution, pH 7 (500 ml) | |
| --- | --- |
| $KH_2PO_4$ | 0.026 M |
| $K_2HPO_4$ | 0.052 M |

| Solution 2. Carbon source (10 ml) | |
| --- | --- |
| L-glutamine | 0.2 g |
| Casein hydrolysate | 10 g |
| Bacto casitone | 10g |
| Yeast extract | 4 g |
| Glycerin | 6 ml |
| Deionized $H_2O$ | Up to 100 ml |

| Solution 3. Salts solution (1 ml) | |
| --- | --- |
| $ZnCl_2$ | 0.05 M |
| $MgCl_2 \cdot 6H_2O$ | 0.5 M |
| $MnCl_2 \cdot 6H_2O$ | 0.01 M |
| $CaCl_2 \cdot 2H_2O$ | 0.2 M |
| $FeCl_3 \cdot 6H_2O$ | 0.05 M |
| HCl | 1% |

Bacterial growth is initiated by transferring 100 μl from a SVBS-1801 over night (ON) preinoculum to the CCY medium (1:1). Bacteria are grown at 30°C and 220 rpm for 48-72 h. The fermentation process concludes once 95% of the SVBS-1801 cells, which have undergone a sporulation process, lysate and release their crystal and spore. To verify that such stage has been achieved, 1 ml samples of the culture should be analyzed under an optical microscope.

To purify the crystals and spores that have been produced, the contents of the culture are centrifuged, and the precipitate is then washed with a 10% solution of NaCl 1M / EDTA 10 mM. Such solution is utilized to prevent proteases from degrading the protein crystals. Subsequently, the culture is again centrifuged, and the pellet is lyophilized and stored at room temperature until formulated or resuspended in KCl 10 mM (10 ml/l initial culture) and stored at -20°C.

**EXAMPLE 2.- Characterization of strain SVBS-1801: Procedure to determine if strain SVBS-1801 produces β-exotoxin**

To determine if a particular Bt strain is β-exotoxin positive, the supernatant of the growth medium should be analyzed. For this purpose, the procedure used by Hernández *et al.,* 2003, was used. Thus, a colony of Bt SVBS-1801 and HD-2 strains (used as a positive control) are grown in 10 ml of CCY at 30°C and 220 rpm for 48 h. Next, cultures are centrifuged at 9000xg for 10 min and their supernatants transferred to new tubes and sterilized in an autoclave at 120°C and 1 atm for 20 min.

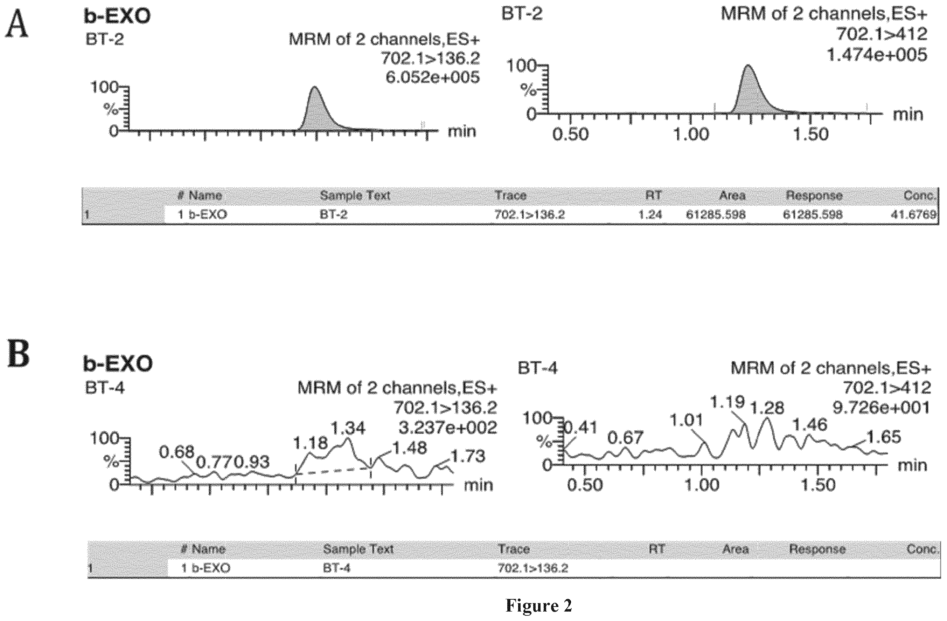

To detect the presence of β-exotoxin an HPLC is performed. $KH_2PO_4$ (50 mM, pH 3), at a flow rate of 2ml/min, constitutes the mobile phase and is filtered alongside the sterilized supernatant through a nylon membrane of 0,45 μm. 20 μl of supernatant are analyzed in a μ-Bondapak C18 column at 25-30°C and 260 nm. If the β-exotoxin is present, two clear peaks indicating its phosphorylated and dephosphorylated form should appear. In the present case, as shown in Figure 2, such peaks were not detected.

**EXAMPLE 3.- Characterization of strain SVBS-1801: DNA extraction, sequence, and genomic sequence processing**

3.1. DNA extraction, sequence, and genomic sequence processing

Total DNA of SVBS-1801 was isolated using the components and following the protocol for DNA isolation from Gram-positive bacteria supplied in the Wizard® Genomic DNA Purification Kit (Promega, Madison, WI, USA). The extracted DNA samples were used to prepare DNA libraries and subsequently were sequenced with Illumina NextSeq500 Sequencer in the Genomics Research Hub laboratory (Cardiff School of Biosciences, UK).

The analysis of the generated raw sequence data was carried out using CLC Genomics Workbench 10.1.1. Raw sequence data were quality filtered by removing low quality or ambiguous reads. Reads shorter than 50 bp were discarded. Reads were *de novo* assembled using a stringent criterion of an overlap of at least 95 bp of the read and 95% identity. Gene prediction on the assembled contigs was done using GeneMark.hmm prokaryotic 3.25. The predicted protein sequences obtained were compared using BLASTP to a database built from the Bt toxin list http://www.btnomenclature.info (Crickmore *et al.,* 2018).

This bioinformatic analysis allowed the present inventors to identify the complete gene sequence of insecticidal genes. Regardless, some *cry* and *vip* gene fragments were identified, as described below.

3.2. Insecticidal genes identified

Table 2 includes a full list of all *cry* gene fragments (coding sequences) identified in SVBS-1801, indicating their degree of identity (% id.) with closely related insecticidal genes previously described in: http://www.btnomenclature.info. The percentage of coverage of the identified sequences included in the comparison (% cov.), as well as the number of nucleotides (Ntd.) of the sequences identified in SVBS-1801 and the sequences present in the database are also indicated.

Table 2. SVBS-1801 *cry* gene content

| SVBS-1801 | | | | | Database* | | |
|---|---|---|---|---|---|---|---|
| *cry* gene | SEQ ID NO: | Ntd | % id. | % cov. | *cry* gene | Acc. Num. | Ntd |
| cry1Ab24-like | 1 | 3456 | 98 | 100 | cry1Ab24 | HQ439778 | 3531 |
| cry1Da3 | 3 | 3492 | 100 | 100 | cry1Da3 | HQ439784 | 3492 |
| cry1Ea7 | 5 | 3516 | 100 | 100 | cry1Ea7 | AY894137 | 3516 |
| cry1Ja1-like | 7 | 3504 | 94 | 100 | cry1Ja1 | L32019 | 350 |
| cry1Nb1 | 9 | 2016 | 100 | 100 | cry1Nb1 | KC156678 | 2031 |
| cry2Ad1 | 11 | 1902 | 100 | 100 | Cry2Ad1 | AF200816 | 1902 |

\* Database: http://www.btnomenclature.info

Similarly, Table 3 contains a complete list of all the *vip* or *sip* gene fragments identified in the SVBS-1801 strain, indicating their degree of identity with closely related insecticidal genes previously described in: http://www.btnomen clature.info.

Table 3. SVBS-1801 *vip* gene content

| SVBS-1801 | | | | | Database* | | |
|---|---|---|---|---|---|---|---|
| *cry* gene | SEQ ID NO: | Ntd | % id. | % cov. | *cry* gene | Acc. Num. | Ntd |
| *vip1Ca1* | 13 | 2327 | 100 | 100 | *vip1Ca1* | AY245547 | 2327 |
| *vip2Ac1* | 15 | 1388 | 100 | 100 | *vip2Ac1* | AY245547 | 1388 |
| *vip3Af3* | 17 | 2366 | 100 | 100 | *vip3Af3* | HM117634 | 2367 |

* Database: http://www.btnomenclature.info

3.3. Molecular characteristics of SVBS-1801 insecticidal genes

Nucleotide sequences of SVBS-1801 *cry* genes were analyzed using BLAST (Altschul *et al.,* 1990) and the results showed that they presented their highest degree of identity with the genes *cry1Ab24* (HQ439778), *cry1Da3* (HQ439784), *cry1Ea7* (AY894137), *cry1Ja1(L32019), cryNb1* (KC156678), and *cry2Ad1* (AF200816) from previously described Bt strains (http://www.btnomenclature.info). Analogously, SVBS-1801 *vip* gene sequences were found closer to previously characterized *vip1Ca1* (AY245547), *vip2Ac1* (AY245547), and *vip3Af3* (HM117634). As reflected in Tables 2 and 3, the percentage of identity was 100% except for the case of *cry1Ab24* and *cry1Ja1,* but the percentage of identity (near 95%) was high enough not to consider the SVBS-1801 genes *cry1Ab24-like* and *cry1Ja1-like* different genes according to the usual rules of nomenclature used for *B. thuringiensis* genes..

**EXAMPLE 4.- Cloning: PCR amplification of insecticidal *cry* genes**

Specific oligonucleotide primer pairs, including restriction sites for cloning, were designed to amplify the Open Reading Frames (ORF) of *cry1Ab24, cry1Da3, cry1Ea7, cry1Ja1, cry1Nb1* and *cry2Ad1* genes based on the sequencing results of the Bt strain. The sequences of said primer pairs are indicated in Table 4 below, where the information in the second column indicates, for each primer, whether it is a forward (Fwd) or a reverse (Rev) primer and, also, the restriction enzyme intended to be used for cloning. The underlined fragment of each primer corresponds to the enzyme cleavage site.

Table 4: Primer pairs used for the amplification of the identified SVBS-1801 genes

| *cry* gene | | Oligonucleotide sequence (5'-3') | |
|---|---|---|---|
| *cry1Ab24-like* | *Fwd-SalI* | SEQ ID NO:19 | GGTCGACGGTATCTTAATAAAAGAGATGGAG |
| | *Rev-PaeI* | SEQ ID NO:20 | GCATGCTTATTCCTCCATAAGGAGTAATTCCAC |
| *cry1Da3* | *Fwd-SalI* | SEQ ID NO:21 | GGTCGACGGACTTTAGTAATTTAATAAAAAAAGGG |
| | *Rev-PaeI* | SEQ ID NO:22 | GCATGCTTATTCCTCCATAAGGAGTAATTCCAC |
| *cry1Ea7* | Fwd-*SalI* | SEQ ID NO:23 | GTGTCGACCAGTACCAAATTATAAGAACTTTGG |
| | Rev-*PaeI* | SEQ ID NO:24 | GCATGCTTATTCCTCCATAAGGAGTAATTCCAC |
| *cry1Ja1-like* | Fwd-*SalI* | SEQ ID NO:25 | GGTCGACAACCAAAGAGAAAGGGGTAAC |
| | Rev-*PaeI* | SEQ ID NO:26 | GGCATGCGTTACAGAGATTAGACGACTAC |
| *cry1Nb1* | *Fwd-SalI* | SEQ ID NO:27 | GTCGACCTAAAAATAATGAATATTGGAGGAAAG |
| | *Rev-PstI* | SEQ ID NO:28 | CTGCAGCTACATGTTACGCTCAATATTGAG |
| *cry2Ad1* | Fwd-*SalI* | SEQ ID NO:29 | GTGTCGACCCTAATATTTAAGGAGGAATTTTATATG |
| | *Rev-PaeI* | SEQ ID NO:30 | GCGCATGCCTCAAACTTTAATAAAGTGGTG |

DNA extracted from strain SVBS-1801 was used as template in a 50 $\mu$l of reaction mixture containing 10 $\mu$l reaction buffer 5x HF, 1 $\mu$l of dNTPs mixture 100 mM, 1 $\mu$l of each forward and reverse primer 10 $\mu$M, 0,5 U Phusion High-fidelity DNA polymerase (NEB) and 100 ng of total DNA. PCR cycling profiles were: 1 initial denaturation cycle at 98 °C for 30 s, 30 amplification cycles of 98 °C for 10 s, 55 °C for 1 min and 72°C for 3 min and 30 s, followed by a final extension step at 72°C for 10 min in a C1000 Touch thermal cycler (Bio-Rad). The PCR products were analyzed by electrophoresis on a 1% agarose gel in TAE buffer (40 mM Tris, 20 mM acetic acid and 1 mM EDTA; pH 8) at 100 V for 30 min.

The PCR products corresponding to the *cry1Ab, cry1Da, cry1Ea, cry1Ja, cry1Nb* and cry2Ad CDS were purified from the agarose gel with NucleoSpin Gel and PCR clean up kit (Macherey-Nagel Inc., Bethlehem, PA) and cloned bluntly into the pJET vector (CloneJET PCR Cloning Kit, Fermentas, Canada) to obtain the pJET-protoxin constructions (pJET-1Ab, pJET-1Da, pJET-1Ea, pJET-1Ja, pJET-1Nb and p-JET-2Ad). Transformation of *E. coli* XL1-Blue was performed following a standard protocol (Sambrook *et al.,* 1989) for recombinant plasmid production containing each of the *cry* gene CDS. Putative positive clones were checked by PCR in a 20 $\mu$l of reaction mixture containing 2 $\mu$l reaction buffer 10xNH4. 1 $\mu$l MgCl$_2$ 50 mM, 1 $\mu$l of dNTPs mixture 100 mM, 1 $\mu$l of each forward and reverse primer 10 $\mu$M, and 0,2 $\mu$l of BioTaq Polymerse (Bioline). *E. coli* XL1-Blue cells harbouring the pJET constructions were cultured in 5 ml LB broth supplemented with Ampicillin (100 mg/mL) at 37 °C and 200 rpm overnight. Recombinant plasmids were extracted with NucleoSpin plasmid kit (Macherey-Nagel Inc., Bethlehem, PA) according to manufacturer's protocol and verified by sequencing using an intermediate forward primer for each gene and the reverse primer for the pJET vector (StabVida, Caparica, Portugal). The *cry* gene nucleotide and protein sequences were aligned with those of toxins available in the GenBank database using Geneious R8. Figures 4 and 6 show the alignment of the sequences of the *cry1Ab24-like* and *cry1Ab24* genes and that of their corresponding protein sequences, respectively; analogously the alignment of the *cry1Ja1-like* and *cry1Ja1* sequences is shown in Figure 5, while the alignment of their corresponding protein sequences is reflected in Figure 7.

Digestion of the recombinant pJET-protoxin constructions of Example 4 was performed with the corresponding combination of restriction enzymes (Thermo Scientific) and subsequent purification of digestion products, previously resolved in an agarose gel, was performed with NucleoSpin Gel and PCR clean up kit (Macherey-Nagel Inc., Bethlehem, PA). The expression pSTAB vector (Park *et al.,* 1998), previously digested with the corresponding restriction enzymes, was used as receptor for the inserts using the Rapid DNA ligation kit (Thermo Scientific) to obtain recombinant plasmids with the pSTAB-protoxin constructions. *E. coli* XL1-Blue cells were transformed with the ligation products, as described before, and putative positive clones were checked by colony PCR before plasmid extraction. An acrystalliferous Bt strain, BMB 171 (Li *et al.,* 2000) was used as receptor in a transformation process performed according to previous authors (Cucarella *et al.,* 2001). Putative positive clones were checked for protein production via colony PCR

### EXAMPLE 5.- Protein Expression

The BMB 171-protoxin constructions of Example 4 were cultured in 50 ml CCY medium supplemented with 20 $\mu$g/ml erythromycin in a rotary shaker set at 28°C and 200 rpm for two-three days, until sporulation and lysis of 95% of the cells was observed. Inclusion bodies were observed by phase contrast microscopy at x1000 magnification. Spore and crystal mixtures were then centrifuged at 9000g for 10 min at 4°C. Pellets were washed three times in 1 M NaCl, six times in cold water and finally resupended in 10 mM KCl.

For protein quantification a volume (100 $\mu$l) of each recombinant protein was solubilized in 1000 $\mu$l of 50 mM Na$_2$CO$_3$ (pH 11.3) and 10 mM dithiothreitol (DTT) solution by gentle agitation during 2 h at 37 °C. Non-solubilized crystals were removed by centrifugation at 9000g for 10 min at 4 °C. An aliquot (500 $\mu$l) was used for protein quantification by Bradford assay (Bradford, 1976) (Bio-Rad) using bovine serum albumin (BSA) as standard.

### EXAMPLE 6.- Molecular weight of SVBS-1801 crystal proteins.

The molecular weight of crystal proteins is usually determined through SDS-PAGE (Laemmli, 1970). In order to do so, a purification of the crystal proteins must be carried out first. Purification may be achieved through different methodologies, including the gradient method proposed by Thomas and Ellar, 1983. Once the crystal proteins are retrieved, a 10 $\mu$l aliquot is mixed with 5 $\mu$l of 30$\times$ Reducing Agent (1/10 of the volume) and 3$\times$SDS Sample Buffer (1 volume) (New England Biolabs) and denaturized at 100°C for 5 min. The mixture is then loaded into a 10% polyacrylamide gel and run for 1h at 36 mA. Next, the gel is stained using a solution of 50 % (v/v) ethanol, 10 % (v/v) acetic acid and 0,1 % Coomasie blue R 250.

As shown in Fig 3, SVBS-1801 presents two bands of about 130 and 133 kDa, which is the size corresponding to Cry1 proteins. Cry2 proteins, in turn, have a size of 633 kDa. The absence of a band in the area corresponding to such size may indicate that protein Cry2Ad1 might not be expressed in the growing conditions used in Example 1.

### EXAMPLE 7.- Procedure for obtaining SVBS-1801 active ingredient (AI)

The AI may be obtained by growing strain SVBS-1801 in a medium that promotes sporulation, such as CCY (Stewart *et al.,* 1981), as in section 1.2 of Example 1. When 95% of the spore-forming bacterial cells are naturally lysed, the contents of the medium are centrifugated. The resulting pellets should contain a mixture of spores and crystals that were released to the medium during the lysis of the sporulating cells. Concentration by physical dewatering may be done by using a continuous centrifuge operating at > 8.000 g to produce a slurry with a solid content of 12-15%, which slurry is the fermentation product with a 50-fold reduction in volume, where the mixtures of spores and crystals are more concentrated but which also includes a part of the liquid and bacterial products comprised in the supernatant. The supernatant contains Vip3A proteins but not Cry proteins, since the only Cry proteins that are secreted to the medium are the Cry1I; as genes *cry1I* are absent of the genome of the strain SVBS-1801, the mentioned strain cannot synthesized Cry1I proteins and they are absent of the supernatant.

The centrifugation to obtain the slurry can be followed by either formulation and spray drying to a powder or by direct formulation into a flowable.

**EXAMPLE 8.- Insecticidal properties of different preparations obtained from SVBS-1801 culture or proteins for neonate *Spodoptera frugiperda* larvae**

*8.1. Spodoptera frugiperda diet for SVBS-1801 active ingredient toxicity assays in vivo*

The standard diet, widely used for rearing *S. frugiperda,* is a modified version from the tobacco hornworm diet of Hoffman and Lawson (1964).The diet is prepared as follows: ingredients are added in a beaker and thoroughly mixed before being heat sterilized (121°C, 20 min). Antibiotics (streptomycin, chlortetracycline), a vitamin mixture, ascorbic acid and choline must be supplemented when the mixture has cooled to 50°C. The completed diets can be blended with a kitchen mixer. Finally, the diet is poured into sterile containers of 400 ml ($17 \times 10 \times 2.5$ cm) and allowed to solidify at room temperature.

8.2. Insecticidal properties of different bacterial preparations obtained from the SVBS 1801 whole culture for neonate *Spodoptera frugiperda* larvae

To determine the concentration-mortality response, insects were treated with different bacterial preparations obtained from the culture of the Bt strain SVBS-1801 (Table 5). Seven concentrations were prepared and tested for each treatment. The treatments were:

- As indicated in Example 7, an aqueous solution containing a spores and crystals (S + C) mixture which usually compose the active ingredient of Bt-based products, so that seven concentrations of such aqueous solution were tested.
- Furthermore, preparations in presence of the supernatant were tested for possible interactions between the spores and crystals mixture and the secreted toxins that may be present in the culture medium.
- Moreover, as the insecticidal toxicity of a Bt strain is mainly attributed to the delta endotoxins that make up the parasporal crystal, crystals were separated from spores and, after recovering the crystals, treatments containing purified crystals were tested to determine their efficacy in absence of the spores.

- Besides, possible interactions between the purified crystals and the supernatant were evaluated
- Preparations containing spores, supernatant and mixtures of both, spores and supernatant, were also tested.

*Preparation of treatments*

For all treatments, a total of seven concentrations, ranging from 0.3 to 300 ng/$\mu$l of insecticidal protein, were prepared from an aliquot previously quantified in order to determine the toxicity of the Bt strain and different products of the strain itself or resulting from the strain culture.

Aqueous solutions of mixtures of spores and crystals (S+C). A total of seven concentrations, ranging from 0.3 to 300 ng/$\mu$l of insecticidal protein, were prepared from an aliquot previously quantified (Example 5) in order to determine the toxicity of the Bt strain.

Mixtures of spores and crystals (S+C) with supernatant. The corresponding amount of spore and crystal mixture was centrifuged, and the resulting pellet was resuspended in supernatant. The same serial dilutions described for S+C treatment were tested.

Separation of parasporal crystals from spores and preparation of treatments with purified crystals. A modified version of the protocol described by Mounsef *et al* 2014 was applied in order to obtain purified crystals from the samples. A volume (200 $\mu$l) of a spore and crystal mixture was washed three times with NaCl 1M and centrifuged at 9000g for 10 min. Pellets were then resuspended in 900 $\mu$l PBS 1x and 100 $\mu$l of hexane were added. Tubes were vortexed for 1 min and centrifuged at 6000g, 4°C for 10 min. The supernatant was discarded and the resulting pellet was subjected to the same procedure at least three times. Crystals were washed three times in cold distilled water and absence of spores was checked by phase contrast microscopy at x1000 magnification. Quantification of purified crystals was performed as described at the end of Example 5. Treatments containing the amount of purified crystals that corresponded to the concentrations of insecticidal proteins (0.3 to 300 ng/$\mu$l) of the S+C serial dilutions were prepared.

_Purified crystals in supernatant._ The possible interaction between the purified crystals and the supernatant were evaluated by resuspending the corresponding amount of parasporal crystals, purified as explained in the previous paragraph, in supernatant.

_Purified suspension of spores._ In order to obtain a purified suspension of spores a mixture of spore and crystal mixture was subjected to solubilization by adding a solution containing 50 mM $Na_2CO_3$ (pH 11.3) and 10 mM dithiothreitol (DTT) and by gentle agitation during 2 h at 37 °C. Solubilized crystals in the supernatant were removed by centrifugation at 9000g for 10 min at 4 °C. This process was repeated five times. Absence of crystals was checked by phase contrast microscopy at x1000 magnification. Purified spores were counted on a Petroff-Hausser chamber.

_Spores in supernatant._ The different dilutions of the treatment were prepared from the purified spores obtained according to the previous paragraph by adding supernatant.

_Bioassay performance_

All bioassays, including those set up in section 8.3 below, were performed spraying the surface of the artificial diet with the corresponding bacterial preparation using groups of 28 individualized _S. frugiperda_ newly hatched (<12 h) larvae. Toxicity experiments were replicated at least three times on different days using independent preparations and maintained in a controlled environment chamber at 25 + 1 °C with a 16-h light/8-h dark cycle for 5 days and were checked for mortality. Concentration-mortality data were pooled and subjected to Probit regression analysis (Finney, 1971) in the POLO-PC program (Le Ora Software, 1987). Mean lethal concentration ($LC_{50}$) values were considered to differ significantly if their 95% fiducial limits did not overlap.

The results of the concentration-mortality responses obtained for different preparations containing the spores and crystals mixture and the purified crystals, both in presence and absence of the effect provided by the supernatant are shown in Table 5.

Table 5. Relative potency of the insecticidal activity of different bacterial preparations obtained from the culture of the Bt strain SVBS-1801, for newly hatched larvae of _S. frugiperda._

| Bt Treatment | Slope $\pm$SE | Intercept | $LC_{50}$ (ng/$\mu$l) | $\chi^2$ (df) | Relative Potency | 95% F. L. | |
|---|---|---|---|---|---|---|---|
| | | | | | | Lower | Upper |
| **S+C Mixture** | 1.56$\pm$0.13 | 3.67 | **7.16** | 7.40 (3) | 1 | - | - |
| **S+C Mixture + Supernatant** | 1.61$\pm$0.15 | 4.23 | **2.99** | 6.21 (3) | 2.39 | 1.72 | 3.33 |
| **Purified Crystals** | 1.48$\pm$0.15 | 4.28 | **3.05** | 4.31 (3) | 2.34 | 1.64 | 3.34 |
| **Purified Crystals + supernatant** | 1.02$\pm$0.22 | 4.48 | **0.25** | 1.56 (3) | 28.19 | 7.27 | 109.31 |

The parameters corresponding to the regression lines obtained for the treatments in Table 5 demonstrate significant differences in the $LC_{50}$ values for the spores and crystals mixture, being 2.39-fold more toxic in presence of the supernatant. Purified crystals are significantly more active in absence of spores, suggesting that an adverse effect takes place when inoculated together. However, a synergistic effect is evidenced when the purified crystals are combined with the supernatant, reporting a 28.19-fold more potent treatment when compared to aqueous preparations of spores and crystals mixtures.

No activity was reported for bacterial preparations containing spores, supernatant or combinations of both.

8.3. Insecticidal properties of SVBS-1801 Cry proteins for neonate _Spodoptera frugiperda_ larvae

As commented above, the insecticidal toxicity of a Bt strain is mainly attributed to the delta endotoxins that make up the parasporal crystal. But not all of them show activity or the same degree of activity against a particular insect. Thus, single concentration (100 ng/$\mu$l) bioassays were performed in order to determine the activity of single proteins contained in the parasporal crystal, following the same methodology described in Section 8.2. Protein Cry2Ad1 was not included in the assay due to the results obtained in the SDS-PAGE assay of Example 6, which appear to indicate that the protein could not be expressed in the bacterial growing conditions used in Example 1 of the present application.

Table 6 shows the individual activity of each individual Cry protein when administered at a single concentration of insecticidal protein (100 ng/$\mu$l). It demonstrates that out of five proteins the insecticidal activity of the parasporal crystal should be attributed to Cry1Da3, Cry1Ea7 and Cry1Ja1-like, due to the lack of activity of Cry1Ab1 and Cry1Nb1 for newly hatched _S. frugiperda_ larvae.

Table 6. Toxicity of individual proteins for newly hatched larvae of *S. frugiperda*.

| Recombinant Protein | Toxic/Not toxic (100 ng/μl) |
|---|---|
| Cry1Ab24-like | Not toxic |
| Cry1Da3 | Toxic |
| Cry1Ea7 | Toxic |
| Cry1Ja1-like | Toxic |
| Cry1Nb1 | Not toxic |

Subsequently, toxic proteins were further evaluated and mean lethal concentrations (LC$_{50}$) were estimated. Finally, protein interactions were evaluated by testing a mixture containing equimolar concentrations of the previous recombinant proteins.

Table 7 shows the parameters corresponding to the regression lines obtained for the three reported toxic proteins (Cry1Da3, Cry1Ea7 and Cry1Ja1-like) when evaluated individually and in an equimolar mixture containing all three in the same ratio.

Table 7. Insecticidal potency of three individual proteins (Cry1Da, Cry1Ea and Cry1Ja-like) and an artificial equimolar mixture containing all three in the ratio 1: 1: 1, for *S. frugiperda* neonate larvae.

| Bt Treatment | Slope ±SE | Intercept | LC$_{50}$ (ng/μl) | $\chi^2$ (df) | Relative Potency | 95 % F. L. | |
|---|---|---|---|---|---|---|---|
| | | | | | | Lower | Upper |
| **Cry1Ea7** | 1.03±0.09 | 3.53 | 25.75 | 20.57 (3) | 1 | - | - |
| **Cry1Da3** | 1.69±0.16 | 4.33 | 2.48 | 3.53 (3) | 10.34 | 7.24 | 14.86 |
| **Cry1Ja1-like** | 0.88±0.12 | 4.56 | 3.12 | 4.41 (3) | 8.25 | 4.62 | 14.74 |
| **Protein Mixture (1:1:1)** | 1.27±0.17 | 4.95 | 1.09 | 2.93 | 23.59 | 15.92 | 41.96 |

As can be seen in Table 7, *S. frugiperda* larvae are very susceptible to Cry1Da3 and Cry1Ja1-like, reporting 10.34- and 8.25-fold more toxic than Cry1Ea7, respectively. The absence of overlap between the fiducial limits (FL) of the relative potencies of Cry1Ea7 clearly indicates that the differences are statistically significant when compared to Cry1Da3 or Cry1Ja-like. When the three proteins were combined in an equimolar mixture the LC$_{50}$ value is reduced to 1.09 ng/μl, being significantly more potent when compared to the three individual toxins, suggesting a synergistic effect among them.

In order to calculate the synergistic factor, the expected mean lethal concentration (LC$_{50}$) of the mixture relative to that of the three single proteins on basis of the relative proportion of Cry1Da3 ($r_a$), Cry1Ea7 ($r_a$) and Cry1Ja1-like ($r_c$) in the mixture must be calculated (Tabashnik, 1992) and compared to the obtained LC$_{50}$ of the mixture (Table 7).

$$LC_{50(m)} = \left[ \frac{r_a}{LC_{50(a)}} + \frac{r_b}{LC_{50(b)}} + \frac{r_c}{LC_{50(c)}} \right]^{-1}$$

$$LC_{50(m)} = \left[ \frac{0.33}{2.48} + \frac{0.33}{25.75} + \frac{0.33}{3.12} \right]^{-1} = 3.96 \text{ ng/μl}$$

A synergistic factor of 3.63 was obtained for the mixture of Cry1Da3, Cry1Ea7 and Cry1Ja1-like for newly hatched *S. frugiperda* larvae.

**EXAMPLE 9.- Toxicity assay to determine the insecticidal potency of the SVBS-1801 strain relative to the commercial strains of Dipel (ABTS-351) and XenTari (ABTS-1875) in *S. frugiperda* second instar larvae**

9.1. Procedure for obtaining SVBS-1801 active ingredient (AI)

The procedure for obtaining SVBS-1801 active ingredients was as set forth in Example 7.

9.2. Preparation of active ingredients from Bt strains of commercial products

In order to compare the activity of the active ingredients prepared from SVBS-1801 strain and Bt strains that give rise to commercial products (ABTS-351 in the case of Dipel®, ABTS-1857 in the case of (XenTari®), the *B. thuringiensis* strains ABTS-351 and ABTS-1857 were directly isolated from the commercial products DiPel® DF (Kenogard, Valent BioScience Corporation; manufacturing batch 261-355-PG; date of manufacture 01/2016) and XenTari® GD (Kenogard, Valent BioScience Corporation; manufacturing batch 264-637-PG; date of manufacture 04/2016), respectively, sold in the European Union. Each bacterial strain was grown at 28°C for 72 h in sterile CCY medium (Stewart *et al.,* 1981) and the process explained in Example 5 was also follow for these strains, until a slurry with a solid content of 12-15% was obtained for any of the strains, what was considered to be the AI to be compared with the AI obtained from strain SVBS-1801.

9.3. Toxicity assay by the *droplet feeding method* using crystal and spore mixtures from the ABTS-351 (Dipel®), ABTS-1857 (XenTari®) and SVBS-1801 strains in *S. frugiperda* second instar larvae.

In order to perform the toxicity assay, the following materials are required: 28 well blisters, artificial diet (see section 8.1) and *S. frugiperda* newly molted second instar larvae. The mixture of crystals and spores should be diluted five times using a dilution factor between 3 and 5, which will result in 6 different concentrations for the AI to be tested in the next step. Likewise, a negative control lacking the AI but preserving the rest of the components shall be prepared. *S. frugiperda* newly molted second instar larvae are fed drops of one of the 6 different concentrations of the AI, following the droplet feeding method (Hughes and Wood, 1981). Fed larvae were placed in individual wells from a 28-well blister containing 1.5 cm$^3$ of artificial diet.

For this study, a total of 28 newly molted second instar larvae were treated with each protein concentration and a range of five concentrations were used for each Bt strain. The bioassay was performed three times. Control insects were fed artificial diet without toxin. The multiwell plates were incubated at 25°C, 60% R.H., and a 14h/10h (light/dark) photoperiod. Mortality was recorded after 6 days. Concentration-mortality data were subjected to probit regression analysis (Finney, 1971) in the POLO-PC program (LeOra Software, 1987).

The results of the concentration-mortality responses obtained with the three Bt strains ABTS-351, isolated from Dipel®, ABTS-1857, isolated from XenTari®, and SVBS-1801 in second-instar *S. frugiperda* larvae are shown in Tables 8, 9, and 10. The slopes of the fitted regression lines for the three Bt isolates ranged from 0.51 for ABTS-351 to 1.47 for SVBS-1801. The values of the slopes were sufficiently different to not allow adjusting the three regression lines in parallel, with a common slope. Therefore, to estimate the relative potencies, among the three Bt strains, it was necessary to compare the regression lines two by two.

Table 8 shows the parameters corresponding to the regression lines obtained for Bt strains ABTS-351, isolated from Dipel® and ABTS-1857, isolated from XenTari®. In Table 4, it can be seen that strain ABTS-1857 is 217.2 times more potent than strain ABTS-351 on *S. frugiperda* second instar. The absence of overlap between the fiducial limits (FL) of the relative potencies of both strains clearly indicate that the differences observed are statistically significant.

Table 8. LC$_{50}$ values and relative potencies of ABTS-1857 respect to ABTS-351 on *Spodoptera frugiperda* second instars.

| Bt strain | Slope ±SE | Intercept ±SE | LC$_{50}$ ($\mu$g/ml) | $\chi^2$ (*df*) | Relative Potency | 95% F. L. | |
|---|---|---|---|---|---|---|---|
| | | | | | | Lower | Upper |
| ABTS-351 | 0.51±0.2 | 2.74±0.3 | 26,260.0 | 2.96 (*3*) | 1 | - | - |
| ABTS-1857 | 0.96±0.1 | 3.01±0.2 | 120.9 | 1.09 (*3*) | 217.2 | 5.5 | 8,635.9 |

Table 9 shows the parameters corresponding to the regression lines obtained for Bt strains ABTS-351, isolated from Dipel®, and SVBS-1801. Table 9 reflects that strain SVBS-1801 is 1,235.4 times more potent than strain ABTS-351 on *S. frugiperda* second instar larvae. The absence of overlap between the fiducial limits (FL) of the relative potencies of both strains clearly indicates that the observed differences are statistically significant.

Table 9. LC$_{50}$ values and relative potencies of SVBS-1801 respect to ABTS-351 on *Spodoptera frugiperda* second instars.

| Bt strain | Slope ±SE | Intercept ±SE | LC$_{50}$ ($\mu$g/ml) | $\chi^2$ (*df*) | Relative Potency | 95 % F L | |
|---|---|---|---|---|---|---|---|
| | | | | | | Lower | Upper |
| ABTS-351 | 0.51±0.2 | 2.74±0.3 | 26,260.0 | 2.96 (*3*) | 1 | - | - |
| SVBS-1801 | 1.47±0.1 | 3.05±0.13 | 21.3 | 3.14 (*3*) | 1,235.4 | 31.3 | 48,708.5 |

Table 10 shows the parameters corresponding to the regression lines obtained for Bt strains ABTS-1857, isolated from XenTari® and SVBS-1801. SVBS-1801 is 5.7 times more potent than ABTS-351 on *S. frugiperda* second instar larvae. The absence of overlap between the fiducial limits (FL) of the relative potencies of both strains clearly indicates that the differences are statistically significant.

Table 10. LC$_{50}$ values and relative potencies of SVBS-1801 compared to ABTS-1857 on *Spodoptera frugiperda* second instars.

| Bt strain | Slope $\pm$SE | Intercept $\pm$SE | LC$_{50}$ ($\mu$g/ml) | $\chi^2$ (*df*) | Relative Potency | 95 % F L | |
|---|---|---|---|---|---|---|---|
| | | | | | | Lower | Upper |
| **ABTS-1857** | 0.96$\pm$0.15 | 3.01$\pm$0.15 | 120.9 | 1.09 (3) | 1 | - | - |
| **SVBS-1801** | 1.47$\pm$0.1 | 3.05$\pm$0.13 | 21.3 | 3.14 (3) | 5.7 | 4.1 | 7.9 |

**EXAMPLE 10.- Insecticidal potency of the SVBS-1801 active ingredient relative to that of ABTS-351 (Dipel®) and ABTS-1857 (XenTari®) for *S. frugiperda* second instar larvae**

As indicated in Example 9.3, the toxicity of ABTS-351 (Dipel®), ABTS-1857 (XenTari®) and SVBS-1801 AI was assessed by the droplet feeding method (Hughes and Wood, 1981). ABTS-351 *(B. thuringiensis* ser. *kurstaki)* is widely accepted as an international reference for several lepidopteran species and constitutes the AI of commercial products such as Dipel® 2x (Abbot). Likewise, ABTS-1857 (isolated from XenTari®) is often found as a reference AI in bioassays aimed at testing insecticidal potency of products against lepidopterans from the genus *Spodoptera.*

The potency of SVBS-1801 insecticidal proteins relative to those of ABTS-351 (Dipel®) and ABTS-1857 (XenTari®) was calculated with regard to the potency (reference potency) indicated in the label of the commercial product used as reference as described by Dulmage (1981).

**Formula:**

$$\frac{\text{reference LC}_{50}}{\text{sample LC}_{50}} \times \text{reference potency (IU/mg)} = \text{sample potency (IU/mg)}$$

IU: international units

SVBS-1801 **potency relative to ABTS-351 (Dipel®):** Dipel® potency resulted in 32,000 IU/mg

$$\frac{26{,}260.0 \text{ ng/}\mu l}{21.3 \text{ ng/}\mu l} \times 32{,}000 \text{ (IU/mg)} = \mathbf{39{,}452{,}\ 643} \text{ (IU/mg)}$$

SVBS-1801 **potency relative to ABTS-1857 (Xentari®):** Xentari® potency resulted in 15,000 IU/mg:

$$\frac{120.9 \text{ ng/}\mu l}{21.3 \text{ ng/}\mu l} \times 15{,}000 \text{ (IU/mg)} = \mathbf{85{,}141} \text{ (IU/mg)}$$

**EXAMPLE 11.- Insecticidal efficacy of SVBS-1801 relative to HD1 and ABTS-1857 (Xentari®) on *S. frugiperda* second instar larvae in semi-field maize plants**

Maize plants in the phenological stage V3 (with 5-6 sheets and 30-35 cm height) were infested with *S. frugiperda* eggs (150 eggs/plant) that were ready to hatch within 24 h. Once the larvae emerged, they were monitored until they reached second instar. Next, plants were treated with a mixture of crystals and spores (at a concentration of crystals and spores corresponding to 100 mg/L for ABTS-1857 and SVBS-1801 strains). Applications were made using a compressed-

EP 3 982 729 B1

air hand sprayer (Matabi 7®, Antzuola, Guipuzcoa, Spain) and they included 0.05% Agral wetter-sticker. 10 ml of spray treatment to run off, which is equivalent to the standard volume of spray application used in maize crops in the region (around 1000 liters/Ha)

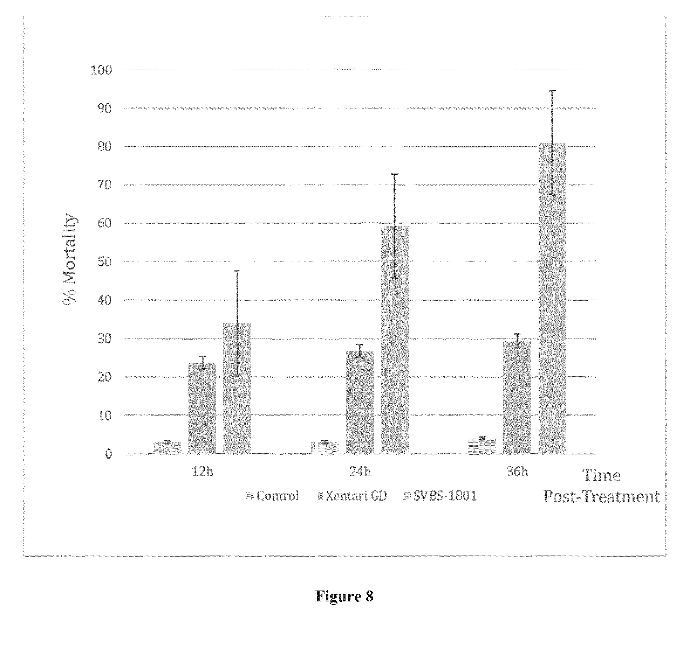

Twenty-eight *S. frugiperda* larvae were randomly collected by hand from plants in each plot at 20, 30 and 40 hours after application, and reared in the laboratory in 25 ml plastic cups with artificial diet at 25 °C until death or pupation. Mortality was registered daily. Percentage mortality was calculated for each treatment, normalized by arcsine transformation and subjected to repeat measures analysis of variance (ANOVA) in SPSS ver.12.0 (SPSS, Chicago, IL). The characteristics of the variance-covariance matrix were examined for this and all subsequent multivariate analyses by applying Mauchly's sphericity test. The results are summarized in Fig. 8.

## DEPOSIT OF BIOLOGICAL MATERIAL

Strain SVBS-1801, has been deposited in the Colección Española de Cultivos Tipo (CECT) (Parque Científico de la Universidad de Valencia; calle Catedrático Agustín Escardino, 9; 46980 Paterna, Valencia. Spain) which accepts bacterial strain deposits according to Royal Decree 664/1997 of 12 May 1997. SVBS-1801 has been deposited for patent purposes following the Budapest Treaty guidelines.

The original deposit receipt (BP/4) and the Viability Certificate (BP/9) of such strain reflect the following relevant data:

- final deposit date: 8 November 2018,
- strain reference number (accession number): CECT 9753.
- Depositor: Serena Valley Biological Systems S.L. (Paseo Universidad 49, 31192 Mutilva, Navarra, Spain). The depositor is different from the applicant.

## References

Altschul, S.F., Gish, W., Miller, W., Myers, E.W. & Lipman, D.J. (1990) "Basic local alignment search tool." J. Mol. Biol. 215:403-410.

Baig, D.N., Bukhari, D.A., and Shakoori, A.R. (2010) cry Genes profiling and the toxicity of isolates of Bacillus thuringiensis from soil samples against American bollworm, Helicoverpa armigera. J. Appl. Microbiol., 109, 1967-1978.

Barretto, M. R. et al. (1999). Insecticidal activity of culture supernatants from Bacillus thuringiensis Berliner strains against Spodoptera frugiperda Smith (Lepidoptera. Noctuidae) Larvae", An. Soc.Entomol. Brasil , 28: 675-685

Bradford, M.M., 1976. A rapid and sensitive method for the quantitation of microgram quantities of protein utilizing the principle of protein-dye binding. Anal. Biochem. 72, 248-254

Capalbo D. et al. (2001) Solid-state fermentation of Bacillus thuringiensis tolworthi to control fall armyworm in maize, EJB Electronic Journal of Biotechnology 4(2): 717-3458

Carozzi, N.B., Kramer, V.C., Warren, G.W., Evola, S., and Koziel, M.G. (1991) Prediction of insecticidal activity of Bacillus thuringiensis strains by polymerase chain reaction product profiles. Appl. Environ. Microbiol., 57, 3057-3061.

Cerón, J., Ortiz, A., Quintero, R., Güereca, I., and Bravo, A. (1995) Specific PCR primers directed to identify *cryI* and *cryIII* genes within a *Bacillus thuringiensis* strain collection. Appl. Envir. Microbiol., 61, 3826-3831.

Crickmore, N., Baum, J., Bravo, A., Lereclus, D., Narva, K., Sampson, K., et al. (2018) Bacillus thuringiensis toxin nomenclature.

Crickmore, N., Zeigler, D.R., Feitelson, J., Schnepf, E., Van Rie, J., Lereclus, D., et al. (1998) Revision of the nomenclature for the Bacillus thuringiensis pesticidal crystal proteins. Microbiol. Mol. Biol. Rev., 62, 807-13.

Cucarella, C., Solano, C., Valle, J., Amorena, B., Lasa, I., and Penadés, J.. (2001) Bap, a Staphylococcus aureus surface protein involved in biofilm formation. J. Bacteriol., 183, 2888-2896.

Donovan, W.P., Engleman, J.T., Donovan, J.C., Baum, J.A., Bunkers, G.J., Chi, D.J., et al. (2006) Discovery and characterization of Sip1A: a novel secreted protein from Bacillus thuringiensis with activity against coleopteran larvae. Appl. Microbiol. Biotechnol., 72, 713-719.

Dulmage, H.T. (1981) Insecticidal activity of isolated of Bacillus thuringiensis and their potential for pest control. Microb. Control pests plant Dis., 1970-1980, Ed.H.D Burges, Academic Press, London.

Estruch, J.J., Warren, G.W., Mullins, M.A., Nye, G.J., Craig, J.A., and Koziel, M.G. (1996) Vip3A, a novel Bacillus thuringiensis vegetative insecticidal protein with a wide spectrum of activities against lepidopteran insects. Proc. Natl. Acad. Sci., 93, 5389-5394.

Ferrandis, M.D., Júrez-Pérez, V.M., Frutos, R., Bel, Y., and Ferré, J. (1999) Distribution of cryI, cryII and cryV genes within Bacillus thuringiensis isolates from Spain. Syst. Appl. Microbiol., 22, 179-185.

Finney, D.. (1971) Probit analysis. 3rd Ed. Cambridge Univ. Press, 333.

Frankenhuyzen, K. van (2009) Insecticidal activity of Bacillus thuringiensis crystal proteins. J. Invertebr. Pathol., 101, 1-16.

Hernández, C.S., Martinez, C., Porcar, M., Caballero, P., and Ferré, J. (2003) Correlation between serovars of Bacillus thuringiensis and type I β-exotoxin production. J. Invertebr. Pathol., 82, 57-62.

Hilbeck A. and Otto M. 2015. "Specifity and combinatorial effects of Bacillus thuringiensis Cry toxins in ...". Frontiers in Environmental Science, Review published on 09 November 2015

Hoffman, D.J. and Lawson, F.R. (1964) Preliminary Studies on Mass Rearing of the Tobacco Hornworm. J. Econ. Entomol., 57, 354-355.

Hu, Y., Platzer, E.G., Bellier, A., and Aroian, R. V. (2010) Discovery of a highly synergistic anthelmintic combination that shows mutual hypersusceptibility. Proc. Natl. Acad. Sci., 107, 5955-5960.

Hughes, P.R. and Wood, H.A. (1981) A synchronous peroral technique for the bioassay of insect viruses. J. Invertebr. Pathol., 37, 154-159.

Iriarte, J., Bel, Y., Ferrandis, M.D., Andrew, R., Murillo, J., Ferré, J., and Caballero, P. (1998) Environmental Distribution and Diversity of Bacillus thuringiensis in Spain. Syst. Appl. Microbiol., 21, 97-106.

Laemmli, U.K. (1970) Cleavage of Structural Proteins during the Assembly of the Head of Bacteriophage T4. Nature, 227, 680-685.

Lecadet, M.-M., Frachon, E., Dumanoir, V.C., Ripouteau, H., Hamon, S., Laurent, P., and Thiery, I. (1999) Updating the H-antigen classification of Bacillus thuringiensis. J. Appl. Microbiol., 86, 660-672.

LeOra Software (1987) LeOra Software (1987) POLO PC, a User Guide to Probit or Logit Analysis. Le Ora Software, Berkelay, CA, USA.

Li, L., Yang, C., Liu, Z., Li, F., and Yu, Z. (2000) [Screening of acrystalliferous mutants from Bacillus thuringiensis and their transformation properties]. Wei Sheng Wu Xue Bao, 40, 85-90.

López-Pazos, S.A., Rojas Arias, A.C., Ospina, S.A., and Cerón, J. (2010) Activity of Bacillus thuringiensis hybrid protein against a lepidopteran and a coleopteran pest. FEMS Microbiol. Lett., 302, 93-98.

Masson, L., Erlandson, M., Puzstai-Carey, M., Brousseau, R., Juárez-Pérez, V., and Frutos, R. (1998) A holistic approach for determining the entomopathogenic potential of Bacillus thuringiensis strains. Appl. Environ. Microbiol., 64, 4782-8.

Milner, R.J. (1994) History of Bacillus thuringiensis. Agric. Ecosyst. Environ., 49, 9-13.

## EP 3 982 729 B1

Mounsef JR, Salameh D, Awad M kallassy, Chamy L, Brandam C, Lteif R. 2014. A simple method for the separation of Bacillus thuringiensis spores and crystals. J Microbiol Methods 107:147-149

Ohba, M., Mizuki, E., and Uemori, A. (2009) Parasporin, a new anticancer protein group from Bacillus thuringiensis. Anticancer Res., 29, 427-33.

Park, H.W., Ge, B., Bauer, L.S., and Federici, B.A. (1998) Optimization of Cry3A yields in Bacillus thuringiensis by use of sporulation-dependent promoters in combination with the STAB-SD mRNA sequence. Appl. Environ. Microbiol., 64, 3932-8.

Polanczyk, R.A. et al. (2000) Effectiveness of Bacillus thuringiensis strains against Spodoptera frugiperda (lepidopera: noctuidae) ". Brazilian Journal of Microbiology, 31: 165-167..

Roh, J.Y., Kim, Y.S., Wang, Y., Liu, Q., Tao, X., Xu, H.G., et al. (2010) Expression of Bacillus thuringiensis mosquitocidal toxin in an antimicrobial Bacillus brevis strain. J. Asia. Pac. Entomol., 13, 61-64.

Sambrook, J., Fritsch, E.F., and Maniatis, T. (1989) Molecular cloning: a laboratory manual. Cold Spring Harbor Laboratory Press.

Schnepf, H.E. and Whiteley, H.R. (1981) Cloning and expression of the Bacillus thuringiensis crystal protein gene in Escherichia coli. Proc. Natl. Acad. Sci. U. S. A., 78, 2893-7.

Šebesta, K. and Horská, K. (1970) *Mechanism of inhibition of DNA-dependent RNA polymerase by exotoxin of Bacillus thuringiensis. Biochim. Biophys. Acta - Nucleic Acids Protein Synth.,* **209,** 357-367.

Shah, S.H., Jan, S.A., Ahmad, N., Khan, S.U., Kumar, T., Iqbal, A., et al. (2015) Use of Different Promoters in Transgenic Plant Development : Current Challenges and Future Perspectives. Am. J. Agric. Environ. Sci., 15, 664-675.

Sharma, H.C., Dhillon, M.K., and Arora, R. (2008) Effects of Bacillus thuringiensis $\delta$-endotoxin-fed Helicoverpa armigera on the survival and development of the parasitoid Campoletis chlorideae. Entomol. Exp. Appl., 126, 1-8.

Stewart, G.S., Johnstone, K., Hagelberg, E., and Ellar, D.J. (1981) Commitment of bacterial spores to germinate A measure of the trigger reaction. Biochem. J., 198, 101-106.

Tabashnik, B.E. (1992) Evaluation of synergism among Bacillus thuringiensis toxins. Appl. Environ. Microbiol., 58, 3343-3346.

Thomas, W.E. and Ellar, D.J. (1983) Bacillus thuringiensis var israelensis crystal delta-endotoxin: effects on insect and mammalian cells in vitro and in vivo. J. Cell Sci., 60, 181-97.

## Claims

1. A strain of *Bacillus thuringiensis* which is **characterized by**:
   being the strain SVBS-1801 deposited in the Colección Española de Cultivos Tipo (CECT) with the deposit reference number CECT 9753.

2. A method for preparing a composition comprising a mixture of crystals and spores of the SVBS-1801 strain of claim 1, which comprises the steps of:

   a) growing SBVS-1801 bacteria until more than 50% of the bacterial cells have undergone sporulation, have lysed and released their crystals and spores to the culture medium;
   b) purifying the crystals and spores by provoking their sedimentation from the culture medium where they are in suspension, preferably by centrifugation, and collecting the precipitate;
   c) optionally, washing the precipitate with a protease inhibiting solution and provoking again the sedimentation of the crystals and spores;
   d) storing the purified crystals and spores either:

a. at room temperature, after having lyophilized the precipitate obtained in step b) or c), or

b. at a temperature of the interval of -18°C to -20°C or lower, after having resuspended the precipitate obtained in b) or c) in water or in an aqueous solution.

3. A method for obtaining purified crystals of the *B. thuringiensis* strain of claim 1, which comprises the steps of:

i) washing a mixture of crystals and spores of the strain of claim 1 with NaCl 1M and centrifuging it at 9000g for 10 min.,

ii) collecting the pellet and resuspending it in PBS,

iii) adding hexane to the suspension obtained in step ii) and vortexing it;

iv) centrifuging the suspension of iii) at 6000 g, 4°C, 10 min,

v) repeating steps ii) to iv) at least three times,

vi) collecting the pellet of crystals obtained in v) and washing it three times in cold distilled water.

4. A composition which comprises:

a) vegetative cells,

b) bacterial cells containing spores,

c) spores,

d) crystals,

e) a mixture of spores, crystals and crystal proteins,

f) Cry proteins not included in a crystal,

g) at least a Vip protein,

or combinations thereof,

of the *Bacillus thuringiensis* strain of claim 1,

wherein, when no member of the group defined in a) to e) is present

a. the composition comprises at least a Cry protein not included in a crystal and at least the protein of SEQ ID NO:8 is present in the composition,

and/or

b. all the three Vip proteins of the group of proteins of SEQ ID NO: 14, SEQ ID NO:16 and SEQ ID NO: 18 are present in the composition.

5. The composition according to claim 4, wherein the composition additionally comprises a portion of the supernatant obtained in step b) of the method of claim 2.

6. The composition according to claim 4 or 5, which comprises at least either

a) a mixture of spores and crystals,

b) a mixture of spores, crystals and a portion of the supernatant obtained in step b) of the method of claim 2,

c) crystals,

d) crystals and a portion of the supernatant obtained in step b) of the method of claim 2,

of the *Bacillus thuringiensis* strain of claim 1.

7. The composition according to claim 6, which comprises either crystals or a mixture of crystals and a portion of the supernatant obtained in step b) of the method of claim 2, wherein the crystals have been previously purified by the method of claim 3 and the composition is essentially free of spores of the *Bacillus thuringiensis* strain of claim 1.

8. The composition according to claim 4, which comprises at least a mixture of spores, crystals and crystal proteins of the *Bacillus thuringiensis* strain of claim 1.

9. The composition according to claim 4, which comprises Cry proteins not included in crystals and wherein the composition comprises

at least all the Cry proteins of the group of SEQ ID NO:4, SEQ ID NO:6 and SEQ ID NO:8, not being included in crystals.

10. The composition according to any one of claims 4 to 8, which additionally comprises an inhibitor of the germination of spores.

11. The composition according to any one of claim 4 to 10, which additionally comprises at least an agriculturally acceptable excipient.

12. Non-therapeutic use of the *B. thuringiensis* strain of claim 1 or the composition of any one of claims 4 to 11 as an insecticide or to prepare an insecticide.

13. The use according to claim 12, to control insect pests of the species *Spodoptera frugiperda.*

14. The use according to any one of claims 12 to 13 to protect plants.

**Patentansprüche**

1. Ein Stamm von *Bacillus thuringiensis,* der folgendermaßen gekennzeichnet ist:
es ist der Stamm SVBS-1801, der in der Coleccion Espanola de Cultivos Tipo (CECT - Spanische Sammlung von Standardkulturen) unter Hinterlegungsreferenznummer CECT 9753 hinterlegt ist.

2. Ein Verfahren zur Herstellung einer Zusammensetzung, die eine Mischung aus Kristallen und Sporen des SVBS-1801-Stamms nach Anspruch 1 umfasst, umfassend die folgenden Schritte:

   a) Züchten von SBVS-1801-Bakterien, bis mehr als 50% der Bakterienzellen sporuliert, lysiert und ihre Kristalle und Sporen an das Kulturmedium abgegeben haben;
   b) Reinigen der Kristalle und Sporen durch Herbeiführen ihrer Sedimentation aus dem Kulturmedium, in dem sie sich in Suspension befinden, vorzugsweise durch Zentrifugation, und Sammeln der Ausfällung;
   c) optional Waschen der Ausfällung mit einer proteasehemmenden Lösung und erneutes Herbeiführen der Sedimentation der Kristalle und Sporen;
   d) Lagern der gereinigten Kristalle und Sporen entweder:

      a. bei Raumtemperatur, nachdem die in Schritt b) oder c) erhaltene Ausfällung lyophilisiert wurde, oder
      b. bei einer Temperatur im Bereich von -18 °C bis -20 °C oder weniger, nachdem die in b) oder c) erhaltene Ausfällung in Wasser oder in einer wässrigen Lösung resuspendiert wurde.

3. Ein Verfahren zum Erhalten gereinigter Kristalle des *B. thuringiensis-Stamms* nach Anspruch 1, das die folgenden Schritte umfasst:

   i) Waschen einer Mischung aus Kristallen und Sporen des Stammes nach Anspruch 1 mit NaCl 1M und Zentrifugieren bei 9000 g für 10 min.,
   ii) Sammeln des Pellets und Resuspendieren in PBS,
   iii) Zugeben von Hexan zu der in Schritt ii) erhaltenen Suspension und Verwirbeln;
   iv) Zentrifugieren der Suspension von iii) bei 6000 g, 4 °C, 10 min,
   v) Wiederholen der Schritte ii) bis iv) mindestens dreimal,
   vi) Sammeln des in v) erhaltenen Kristallpellets und dreimaliges Waschen in kaltem destilliertem Wasser.

4. Eine Zusammensetzung, umfassend:

   a) vegetative Zellen,
   b) sporenhaltige Bakterienzellen,
   c) Sporen,
   d) Kristalle,
   e) eine Mischung aus Sporen, Kristallen und Kristallproteinen,
   f) Cry-Proteine, die nicht in einem Kristall enthalten sind,
   g) mindestens ein Vip-Protein,
   oder Kombinationen davon,
   des Stammes *Bacillus thuringiensis* nach Anspruch 1,
   wobei, wenn kein Element der in a) bis e) definierten Gruppe vorhanden ist

a. die Zusammensetzung mindestens ein Cry-Protein, das nicht in einem Kristall enthalten ist, umfasst und mindestens das Protein von SEQ ID NO: 8 in der Zusammensetzung vorhanden ist, und/oder

b. alle drei VIP-Proteine der Gruppe der Proteine von SEQ ID NO: 14, SEQ ID NO: 16 und SEQ ID NO: 18 in der Zusammensetzung vorhanden sind.

5. Die Zusammensetzung nach Anspruch 4, wobei die Zusammensetzung zusätzlich einen Teil des in Schritt b) des Verfahrens nach Anspruch 2 erhaltenen Überstands umfasst.

6. Die Zusammensetzung nach Anspruch 4 oder 5, die mindestens entweder

a) eine Mischung aus Sporen und Kristallen,
b) eine Mischung aus Sporen, Kristallen und einem Teil des in Schritt b) des Verfahrens nach Anspruch 2 erhaltenen Überstands,
c) Kristalle,
d) Kristalle und einen Teil des in Schritt b) des Verfahrens nach Anspruch 2 erhaltenen Überstands,
des Stammes *Bacillus thuringiensis* nach Anspruch 1 umfasst.

7. Die Zusammensetzung nach Anspruch 6, die entweder Kristalle oder eine Mischung von Kristallen und einen Teil des in Schritt b) des Verfahrens nach Anspruch 2 erhaltenen Überstands umfasst, wobei die Kristalle zuvor durch das Verfahren nach Anspruch 3 gereinigt wurden und die Zusammensetzung im Wesentlichen frei von Sporen des *Bacillus thuringiensis-Stamms* nach Anspruch 1 ist.

8. Die Zusammensetzung nach Anspruch 4, die mindestens eine Mischung aus Sporen, Kristallen und Kristallproteinen des *Bacillus thuringiensis-Stamms* nach Anspruch 1 umfasst.

9. Die Zusammensetzung nach Anspruch 4, die Cry-Proteine umfasst, die nicht in Kristallen enthalten sind, und wobei die Zusammensetzung mindestens alle Cry-Proteine der Gruppe aus SEQ ID NO: 4, SEQ ID NO: 6 und SEQ ID NO: 8, die nicht in Kristallen enthalten sind, umfasst.

10. Die Zusammensetzung nach einem der Ansprüche 4 bis 8, die zusätzlich einen Inhibitor der Keimung von Sporen umfasst.

11. Die Zusammensetzung nach einem der Ansprüche 4 bis 10, die zusätzlich mindestens einen landwirtschaftlich verträglichen Hilfsstoff umfasst.

12. Nicht-therapeutische Verwendung des *B. thuringiensis* -Stamms nach Anspruch 1 oder der Zusammensetzung nach einem der Ansprüche 4 bis 11 als Insektizid oder zur Herstellung eines Insektizids.

13. Die Verwendung nach Anspruch 12 zur Bekämpfung von Insektenschädlingen der Art *Spodoptera frugiperda.*

14. Die Verwendung nach einem der Ansprüche 12 bis 13 zum Schutz von Pflanzen.

**Revendications**

1. Souche de *Bacillus thuringiensis* qui est **caractérisée en ce qu'**elle est :
la souche SVBS-1801 déposée au Colección Española de Cultivos Tipo (CECT) sous le numéro de référence de dépôt CECT 9753.

2. Procédé de préparation d'une composition comprenant un mélange de cristaux et de spores de la souche SVBS-1801 selon la revendication 1, qui comprend les étapes consistant à :

a) faire croître les bactéries SBVS-1801 jusqu'à ce que plus de 50 % des cellules bactériennes aient subi une sporulation, aient lysé et aient libéré leurs cristaux et spores dans le milieu de culture ;
b) purifier les cristaux et les spores en provoquant leur sédimentation à partir du milieu de culture où ils sont en suspension, de préférence par centrifugation, et recueillir le précipité ;

c) éventuellement, laver le précipité avec une solution inhibitrice de protéase et provoquer à nouveau la sédimentation des cristaux et des spores ;
d) stocker les cristaux et spores purifiés soit :

    a. à température ambiante, après avoir lyophilisé le précipité obtenu à l'étape b) ou c), soit
    b. à une température comprise dans l'intervalle de -18 °C à -20 °C ou inférieure, après avoir remis en suspension le précipité obtenu en b) ou c) dans de l'eau ou dans une solution aqueuse.

3. Procédé pour obtenir des cristaux purifiés de la souche de *B. thuringiensis* selon la revendication 1, qui comprend les étapes consistant à :

    i) laver un mélange de cristaux et de spores de la souche selon la revendication 1 avec NaCl 1M et le centrifuger à 9000 g pendant 10 min,
    ii) recueillir le culot et le remettre en suspension dans du PBS,
    iii) ajouter de l'hexane à la suspension obtenue à l'étape ii) et la vortexer ;
    iv) centrifuger la suspension de iii) à 6000 g, 4°C, 10 min,
    v) répéter les étapes ii) à iv) au moins trois fois,
    vi) recueillir le culot de cristaux obtenu en v) et le laver trois fois à l'eau distillée froide.

4. Composition comprenant :

    a) des cellules végétatives,
    b) des cellules bactériennes contenant des spores,
    c) des spores,
    d) des cristaux,
    e) un mélange de spores, de cristaux et de protéines cristallines,
    f) des protéines Cry non incluses dans un cristal,
    g) au moins une protéine Vip,
    ou des combinaisons de ceux-ci,
    de la souche de *Bacillus thuringiensis* selon la revendication 1,
    dans laquelle, lorsqu'aucun membre du groupe défini en a) à e) n'est présent

    a. la composition comprend au moins une protéine Cry non incluse dans un cristal et au moins la protéine de SEQ ID NO : 8 est présente dans la composition,
    et/ou
    b. les trois protéines Vip du groupe de protéines de SEQ ID NO : 14, SEQ ID NO: 16 et SEQ ID NO: 18 sont présentes dans la composition.

5. Composition selon la revendication 4, dans laquelle la composition comprend en outre une partie du surnageant obtenu à l'étape b) du procédé selon la revendication 2.

6. Composition selon la revendication 4 ou 5, qui comprend au moins soit

    a) un mélange de spores et de cristaux,
    b) un mélange de spores, de cristaux et d'une partie du surnageant obtenu à l'étape b) du procédé selon la revendication 2,
    c) des cristaux,
    d) des cristaux et une partie du surnageant obtenu à l'étape b) du procédé selon la revendication 2,

de la souche de *Bacillus thuringiensis* selon la revendication 1.

7. Composition selon la revendication 6, qui comprend soit des cristaux, soit un mélange de cristaux et d'une partie du surnageant obtenu à l'étape b) du procédé selon la revendication 2, dans laquelle les cristaux ont été préalablement purifiés par le procédé selon la revendication 3 et la composition est essentiellement exempte de spores de la souche de *Bacillus thuringiensis* selon la revendication 1.

8. Composition selon la revendication 4, qui comprend au moins un mélange de spores, de cristaux et de protéines cristallines de la souche de *Bacillus thuringiensis* selon la revendication 1.

9. Composition selon la revendication 4, qui comprend des protéines Cry non incluses dans les cristaux et au moins toutes les protéines Cry du groupe de SEQ ID NO : 4, SEQ ID NO: 6 et SEQ ID NO: 8, non incluses dans les cristaux.

10. Composition selon l'une quelconque des revendications 4 à 8, qui comprend en outre un inhibiteur de la germination de spores.

11. Composition selon l'une quelconque des revendications 4 à 10, qui comprend en outre au moins un excipient acceptable sur le plan agricole.

12. Utilisation non thérapeutique de la souche de *B. thuringiensis* selon la revendication 1 ou de la composition selon l'une quelconque des revendications 4 à 11 en tant qu'insecticide ou pour préparer un insecticide.

13. Utilisation selon la revendication 12, pour lutter contre les insectes nuisibles de l'espèce *Spodoptera frugiperda.*

14. Utilisation selon l'une quelconque des revendications 12 à 13 destinée à protéger les plantes.

Figure 1

Figure 2

Figure 3

```
Query   1    ATGGATAACAATCCGAACATCAATGAATGCATTCCTTATAATTGTTTAAGTAACCCTGAA   60
             ||||||||||||||||||||||||||||||||||||||||||||||||||||||||||||
Sbjct   1    ATGGATAACAATCCGAACATCAATGAATGCATTCCTTATAATTGTTTAAGTAACCCTGAA   60

Query   61   GTAGAAGTATTAGGTGGAGAAAGAATAGAAACTGGTTACACCCCAATCGATATTTCCTTG   120
             ||||||||||||||||||||||||||||||||||||||||||||||||||||||||||||
Sbjct   61   GTAGAAGTATTAGGTGGAGAAAGAATAGAAACTGGTTACACCCCAATCGATATTTCCTTG   120

Query   121  TCGCTAACGCAATTTCTTTTGAGTGAATTTGTTCCCGGTGCTGGATTTGTGTTAGGACTA   180
             ||||||||||||||||||||||||||||||||||||||||||||||||||||||||||||
Sbjct   121  TCGCTAACGCAATTTCTTTTGAGTGAATTTGTTCCCGGTGCTGGATTTGTGTTAGGACTA   180

Query   181  GTTGATATAATATGGGGAATTTTTGGTCCCTCTCAATGGGACGCATTTCTTGTACAAATT   240
             ||||||||||||||||||||||||||||||||||||||||||||||||||||||||||||
Sbjct   181  GTTGATATAATATGGGGAATTTTTGGTCCCTCTCAATGGGACGCATTTCTTGTACAAATT   240

Query   241  GAACAGTTAATTAACCAAAGAATAGAAGAATTCGCTAGGAACCAAGCCATTTCTAGATTA   300
             ||||||||||||||||||||||||||||||||||||||||||||||||||||||||||||
Sbjct   241  GAACAGTTAATTAACCAAAGAATAGAAGAATTCGCTAGGAACCAAGCCATTTCTAGATTA   300

Query   301  GAAGGACTAAGCAATCTTTATCAAATTTACGCAGAATCTTTTAGAGAGTGGGAAGCAGAT   360
             ||||||||||||||||||||||||||||||||||||||||||||||||||||||||||||
Sbjct   301  GAAGGACTAAGCAATCTTTATCAAATTTACGCAGAATCTTTTAGAGAGTGGGAAGCAGAT   360

Query   361  CCTACTAATCCAGCATTAAGAGAAGAGATGCGTATTCAATTCAATGACATGAACAGTGCC   420
             ||||||||||||||||||||||||||||||||||||||||||||||||||||||||||||
Sbjct   361  CCTACTAATCCAGCATTAAGAGAAGAGATGCGTATTCAATTCAATGACATGAACAGTGCC   420

Query   421  CTTACAACCGCTATTCCTCTTTTTGCAGTTCAAAATTATCAAGTTCCTCTTTTATCAGTA   480
             ||||||||||||||||||||||||||||||||||||||||||||||||||||||||||||
Sbjct   421  CTTACAACCGCTATTCCTCTTTTTGCAGTTCAAAATTATCAAGTTCCTCTTTTATCAGTA   480

Query   481  TATGTTCAAGCTGCAAATTTACATTTATCAGTTTTGAGAGATGTTTCAGTGTTTGGACAA   540
             ||||||||||||||||||||||||||||||||||||||||||||||||||||||||||||
Sbjct   481  TATGTTCAAGCTGCAAATTTACATTTATCAGTTTTGAGAGATGTTTCAGTGTTTGGACAA   540

Query   541  AGGTGGGGATTTGATGCCGCGACTATCAATAGTCGTTATAATGATTTAACTAGGCTTATT   600
             ||||||||||||||||||||||||||||||||||||||||||||||||||||||||||||
Sbjct   541  AGGTGGGGATTTGATGCCGCGACTATCAATAGTCGTTATAATGATTTAACTAGGCTTATT   600

Query   601  GGCAACTATACAGATTATGCTGTACGCTGGTACAATACGGGATTAGAGCGTGTATGGGGA   660
             ||||||||||||||||||||||||||||||||||||||||||||||||||||||||||||
Sbjct   601  GGCAACTATACAGATTATGCTGTACGCTGGTACAATACGGGATTAGAGCGTGTATGGGGA   660

Query   661  CCGGATTCTAGAGATTGGATAAGATATAATCAATTTAGAAGAGAATTAACACTAACTGTA   720
             ||||||||||||||||||||||||||||||||||||||||||||||||||||||||||||
Sbjct   661  CCGGATTCTAGAGATTGGATAAGATATAATCAATTTAGAAGAGAATTAACACTAACTGTA   720

Query   721  TTAGATATCGTTTCTCTATTTCCGAACTATGATAGTAGAACGTATCCAATTCGAACAGTT   780
             ||||||||||||||||||||||||||||||||||||||||||||||||||||||||||||
Sbjct   721  TTAGATATCGTTTCTCTATTTCCGAACTATGATAGTAGAACGTATCCAATTCGAACAGTT   780

Query   781  TCCCAATTAACAAGAGAAATTTATACAAACCCAGTATTAGAAAATTTTGATGGTAGTTTT   840
             ||||||||||||||||||||||||||||||||||||||||||||||||||||||||||||
Sbjct   781  TCCCAATTAACAAGAGAAATTTATACAAACCCAGTATTAGAAAATTTTGATGGTAGTTTT   840

Query   841  CGAGGCTCGGCTCAGGGCATAGAAGGAAGTATTAGGAGTCCACATTTGATGGATATACTT   900
             ||||||||||||||||||||||||||||||||||||||||||||||||||||||||||||
Sbjct   841  CGAGGCTCGGCTCAGGGCATAGAAGGAAGTATTAGGAGTCCACATTTGATGGATATACTT   900

Query   901  AGCAGCATAACCATCTATACGGATGCTCATAGAGGAGAATATTATTGGTCAGGGCATCAA   960
             |||||  |||||||||||||||||||||||||||||||||||||||||||||||||||||
Sbjct   901  AGCAGTATAACCATCTATACGGATGCTCATAGAGGAGAATATTATTGGTCAGGGCATCAA   960
```

**Figure 4**

```
Query   961   ATAATGGCTTCTCCTGTAGGGTTTTCGGGGCCAGAATTCACTTTTCCGCTATATGGAACT   1020
              ||||||||||||||||||||||||||||||||||||||||||||||||||||||||||||
Sbjct   961   ATAATGGCTTCTCCTGTAGGGTTTTCGGGGCCAGAATTCACTTTTCCGCTATATGGAACT   1020

Query  1021   ATGGGAAATGCAGCTCCACAACAACGTATTGTTGCTCAACTAGGTCAGGGCGTGTATAGA   1080
              ||||||||||||||||||||||||||||||||||||||||||||||||||||||||||||
Sbjct  1021   ATGGGAAATGCAGCTCCACAACAACGTATTGTTGCTCAACTAGGTCAGGGCGTGTATAGA   1080

Query  1081   ACATTATCGTCCACTTTATATAGAAGACCTTTTAATATAGGGATAAATAATCAACAACTA   1140
              ||||||||||||||||||||||||||||||||||||||||||||||||||||||||||||
Sbjct  1081   ACATTATCGTCCACTTTATATAGAAGACCTTTTAATATAGGGATAAATAATCAACAACTA   1140

Query  1141   TCTGTTCTTGACGGGACAGAATTTGCTTATGGGACCTCCTCAAATTTGCCATCCGCTGTA   1200
              ||||||||||||||||||||||||||||||||||||||||||||||||||||||||||||
Sbjct  1141   TCTGTTCTTGACGGGACAGAATTTGCTTATGGGACCTCCTCAAATTTGCCATCCGCTGTA   1200

Query  1201   TACAGAAAAAGCGGAACGGTAGATTCGCTGGATGAAATACCACCACAGAATAACAACGTG   1260
              ||||||||||||||||||||||||||||||||||||||||||||||||||||||||||||
Sbjct  1201   TACAGAAAAAGCGGAACGGTAGATTCGCTGGATGAAATACCACCACAGAATAACAACGTG   1260

Query  1261   CCACCTAGGCAAGGATTTAGTCATCGATTAAGCCATGTTTCAATGTTTCGTTCAGGCTTT   1320
              ||||||||||||||||||||||||||||||||||||||||||||||||||||||||||||
Sbjct  1261   CCACCTAGGCAAGGATTTAGTCATCGATTAAGCCATGTTTCAATGTTTCGTTCAGGCTTT   1320

Query  1321   AGTAATAGTAGTGTAAGTATAATAAGAGCTCCTATGTTCTCTTGGATACATCGTAGTGCT   1380
              ||||||||||||||||||||||||||||||||||||||||||||||||||||||||||||
Sbjct  1321   AGTAATAGTAGTGTAAGTATAATAAGAGCTCCTATGTTCTCTTGGATACATCGTAGTGCT   1380

Query  1381   GAATTTAATAATATAATTCCTTCATCACAAATTACACAAATACCTTTAACAAAATCTACT   1440
              ||||||||||||||||||||||||||||||||||||||||||||||||||||||||||||
Sbjct  1381   GAATTTAATAATATAATTCCTTCATCACAAATTACACAAATACCTTTAACAAAATCTACT   1440

Query  1441   AATCTTGGCTCTGGAACTTCTGTCGTTAAAGGACCAGGATTTACAGGAGGAGATATTCTT   1500
              ||||||||||||||||||||||||||||||||||||||||||||||||||||||||||||
Sbjct  1441   AATCTTGGCTCTGGAACTTCTGTCGTTAAAGGACCAGGATTTACAGGAGGAGATATTCTT   1500

Query  1501   CGAAGAACTTCACCTGGCCAGATTTCAACCTTAAGAGTAAATATTACTGCACCATTATCA   1560
              ||||||||||||||||||||||||||||||||||||||||||||||||||||||||||||
Sbjct  1501   CGAAGAACTTCACCTGGCCAGATTTCAACCTTAAGAGTAAATATTACTGCACCATTATCA   1560

Query  1561   CAAAGATATCGGGTAAGAATTCGCTACGCTTCTACTACAAATTTACAATTCCATACATCA   1620
              ||||||||||||||||||||||||||||||||||||||||||||||||||||||||||||
Sbjct  1561   CAAAGATATCGGGTAAGAATTCGCTACGCTTCTACTACAAATTTACAATTCCATACATCA   1620

Query  1621   ATTGACGGAAGACCTATTAATCAGGGGAATTTTTCAGCAACTATGAGTAGTGGGAGTAAT   1680
              ||||||||||||||||||||||||||||||||||||||||||||||||||||||||||||
Sbjct  1621   ATTGACGGAAGACCTATTAATCAGGGGAATTTTTCAGCAACTATGAGTAGTGGGAGTAAT   1680

Query  1681   TTACAGTCCGGAAGCTTTAGGACTGTAGGTTTTACTACTCCGTTTAACTTTTCAAATGGA   1740
              ||||||||||||||||||||||||||||||||||||||||||||||||||||||||||||
Sbjct  1681   TTACAGTCCGGAAGCTTTAGGACTGTAGGTTTTACTACTCCGTTTAACTTTTCAAATGGA   1740

Query  1741   TCAAGTGTATTTACGTTAAGTGCTCATGTCTTCAATTCAGGCAATGAAGTTTATATAGAT   1800
              ||||||||||||||||||||||||||||||||||||||||||||||||||||||||||||
Sbjct  1741   TCAAGTGTATTTACGTTAAGTGCTCATGTCTTCAATTCAGGCAATGAAGTTTATATAGAT   1800

Query  1801   CGAATTGAATTTGTTCCGGCAGAAGTAACCTTTGAGGCAGAATATGATTTAGAAAGAGCA   1860
              ||||||||||||||||||||||||||||||||||||||||||||||||||||||||||||
Sbjct  1801   CGAATTGAATTTGTTCCGGCAGAAGTAACCTTTGAGGCAGAATATGATTTAGAAAGAGCA   1860

Query  1861   CAAAAGGCGGTGAATGAGCTGTTTACTTCTTCCAATCAAATCGGGTTAAAAACAGATGTG   1920
              ||||||||||||||||||||||||||||||||||||||||||||||||||||||||||||
Sbjct  1861   CAAAAGGCGGTGAATGAGCTGTTTACTTCTTCCAATCAAATCGGGTTAAAAACAGATGTG   1920
```

**Figure 4 (cont.)**

```
Query  1921  ACGGATTATCATATTGATCAAGTATCCAATTTAGTTGAGTGTTTATCAGATGAATTTTGT  1980
             ||||||||||||||||||||||||||||||||||||||||||||||||||||||||||||
Sbjct  1921  ACGGATTATCATATTGATCAAGTATCCAATTTAGTTGAGTGTTTATCAGATGAATTTTGT  1980

Query  1981  CTGGATGAAAAACAAGAAGTGTCCGAGAAAGTCAAACATGCGAAGCGACTTAGTGATGAG  2040
             ||||||||||||||||||||||||||||||||||||||||||||||||||||||||||||
Sbjct  1981  CTGGATGAAAAACAAGAAGTGTCCGAGAAAGTCAAACATGCGAAGCGACTTAGTGATGAG  2040

Query  2041  CGGAATTTACTTCAAGATCCAAACTTCAGAGGGATCAATAGACAACTAGACCGTGGCTGG  2100
             ||||||||||||||||||||||||||||||||||||||||||||||||||||||||||||
Sbjct  2041  CGGAATTTACTTCAAGATCCAAACTTCAGAGGGATCAATAGACAACTAGACCGTGGCTGG  2100

Query  2101  AGAGGAAGTACGGATATTACCATCCAAGGAGGCGATGACGTATTCAAAGAGAATTACGTT  2160
             ||||||||||||||||||||||||||||||||||||||||||||||||||||||||||||
Sbjct  2101  AGAGGAAGTACGGATATTACCATCCAAGGAGGCGATGACGTATTCAAAGAGAATTACGTT  2160

Query  2161  ACACTACCAGGTACCTTTGATGAGTGCTATCCAACGTATTTATATCAAAAAATAGATGAG  2220
             ||||||||||||||||||||||||||||||||||||||||||||||||||||||||||||
Sbjct  2161  ACACTACCAGGTACCTTTGATGAGTGCTATCCAACGTATTTATATCAAAAAATAGATGAG  2220

Query  2221  TCGAAATTAAAAGCCTATACCCGTTATCAATTAAGAGGGTATATCGAGGATAGTCAAGAC  2280
             ||||||||||||||||||||||||||||||||||||||||||||||||||||||||||||
Sbjct  2221  TCGAAATTAAAAGCCTATACCCGTTATCAATTAAGAGGGTATATCGAGGATAGTCAAGAC  2280

Query  2281  TTAGAAATCTATTTAATTCGCTACAATGCAAAACATGAAACAGTAAATGTGCCAGGTACG  2340
             ||||||||||||||||||||||||||||||||||||||||||||||||||||||||||||
Sbjct  2281  TTAGAAATCTATTTAATTCGCTACAATGCAAAACATGAAACAGTAAATGTGCCAGGTACG  2340

Query  2341  GGTTCCTTATGGCCGCTTTCAGCCCAAAGTCCAATCGGAAAGTGTG    2386
             ||||||||||||||||||||||||||||||||||||||||||||||
Sbjct  2341  GGTTCCTTATGGCCGCTTTCAGCCCAAAGTCCAATCGGAAAGTGTG    2386

Query  2457  AAAGTGTGCCCATCATTCGCATCATTTCTCCTTAGACATTGATGTTGGATGTACAGACTT  2516
             ||||||||||||||||||||||||||||||||||||||||||||||||||||||||||||
Sbjct  2379  AAAGTGTGCCCATCATTCGCATCATTTCTCCTTAGACATTGATGTTGGATGTACAGACTT  2438

Query  2517  AAATGAGGACCTAGGTGTATGGGTGATCTTTAAGATTAAGACGCAAGATGGGCACGCAAG  2576
             ||||||||||||||||||||||||||||||||||||||||||||||||||||||||||||
Sbjct  2439  AAATGAGGACCTAGGTGTATGGGTGATCTTTAAGATTAAGACGCAAGATGGGCACGCAAG  2498

Query  2577  ACTAGGGAATCTAGAGTTTCTCGAAGAGAAACCATTAGTAGGAGAAGCGCTAGCTCGTGT  2636
             ||||||||||||||||||||||||||||||||||||||||||||||||||||||||||||
Sbjct  2499  ACTAGGGAATCTAGAGTTTCTCGAAGAGAAACCATTAGTAGGAGAAGCGCTAGCTCGTGT  2558

Query  2637  GAAAAGAGCGGAGAAAAAATGGAGAGACAAACGCGAAAAATTGGAATGGGAAACAAATAT  2696
             ||||||||||||||||||||||||||||||||||||||||||||||||||||||||||||
Sbjct  2559  GAAAAGAGCGGAGAAAAAATGGAGAGACAAACGCGAAAAATTGGAATGGGAAACAAATAT  2618

Query  2697  CGTTTATAAAGAGGCAAAAGAATCTGTAGATGCTTTATTTGTAAACTCTCAATATGATCA  2756
             ||||||||||||||||||||||||||||||||||||||||||||||||||||||||||||
Sbjct  2619  CGTTTATAAAGAGGCAAAAGAATCTGTAGATGCTTTATTTGTAAACTCTCAATATGATCA  2678

Query  2757  ATTACAAGCGGATACGAATATTGCCATGATTCATGCGGCAGATAAACGTGTTCATAGCAT  2816
             ||||||||||||||||||||||||||||||||||||||||||||||||||||||||||||
Sbjct  2679  ATTACAAGCGGATACGAATATTGCCATGATTCATGCGGCAGATAAACGTGTTCATAGCAT  2738

Query  2817  TCGAGAAGCTTATCTGCCTGAGCTGTCTGTGATTCCGGGTGTCAATGCGGCTATTTTTGA  2876
             ||||||||||||||||||||||||||||||||||||||||||||||||||||||||||||
Sbjct  2739  TCGAGAAGCTTATCTGCCTGAGCTGTCTGTGATTCCGGGTGTCAATGCGGCTATTTTTGA  2798

Query  2877  AGAATTAGAAGGGTGTATTTTTCACTGCATTCTCCCTATATGATGCGAGAAATGTCATTAA  2936
             |||||||||||||||||||||||||||||||||||||||||||||||||||||||||||
Sbjct  2799  AGAATTAGAAGGGTGTATTTTTCACTGCATTCTCCCTATATGATGCGAGAAATGTCATTAA  2858
```

**Figure 4 (cont,)**

36

```
Query  2937  AAATGGTGATTTTAATAATGGCTTATCCTGCTGGAACGTGAAAGGGCATGTAGATGTAGA  2996
             ||||||||||||||||||||||||||||||||||||||||||||||||||||||||||||
Sbjct  2859  AAATGGTGATTTTAATAATGGCTTATCCTGCTGGAACGTGAAAGGGCATGTAGATGTAGA  2918

Query  2997  AGAACAAAACAACCACCGTTCGGTCCTTGTTGTTCCGGAATGGGAAGCAGAAGTGTCACA  3056
             ||||||||||||||||||||||||||||||||||||||||||||||||||||||||||||
Sbjct  2919  AGAACAAAACAACCACCGTTCGGTCCTTGTTGTTCCGGAATGGGAAGCAGAAGTGTCACA  2978

Query  3057  AGAAGTTCGTGTCTGTCCGGGTCGTGGCTATATCCTTCGTGTCACAGCGTACAAGGAGGG  3116
             ||||||||||||||||||||||||||||||||||||||||||||||||||||||||||||
Sbjct  2979  AGAAGTTCGTGTCTGTCCGGGTCGTGGCTATATCCTTCGTGTCACAGCGTACAAGGAGGG  3038

Query  3117  ATATGGAGAAGGTTGCGTAACCATTCATGAGATCGAGAACAATACAGACGAACTGAAGTT  3176
             ||||||||||||||||||||||||||||||||||||||||||||||||||||||||||||
Sbjct  3039  ATATGGAGAAGGTTGCGTAACCATTCATGAGATCGAGAACAATACAGACGAACTGAAGTT  3098

Query  3177  TAGCAACTGCGTAGAAGAGGAAATCTATCCAAACAACACGGTAACGTGTAATGATTATAC  3236
             ||||||||||||||||||||||||||||||||||||||||||||||||||||||||||||
Sbjct  3099  TAGCAACTGCGTAGAAGAGGAAATCTATCCAAACAACACGGTAACGTGTAATGATTATAC  3158

Query  3237  TGTAAATCAAGAAGAATACGAAGGTGCGTACACTTCTCGTAATCGAGGATATAACGAAGC  3296
             ||||||||||||||||||||||||||||||||||||||||||||||||||||||||||||
Sbjct  3159  TGTAAATCAAGAAGAATACGAAGGTGCGTACACTTCTCGTAATCGAGGATATAACGAAGC  3218

Query  3297  TCCTTCCGTACCAGCTGATTATGCATCAGTCTATGAAGAAAAATCGTATACAGATGGACG  3356
             ||||||||||||||||||||||||||||||||||||||||||||||||||||||||||||
Sbjct  3219  TCCTTCCGTACCAGCTGATTATGCATCAGTCTATGAAGAAAAATCGTATACAGATGGACG  3278

Query  3357  AAGAGAGAATCCTTGTGAATTTAACAGAGGGTATAGGGATTACACGCTACTACCAGTTGG  3416
             ||||||||||||||||||||||||||||||||||||||||||||||||||||||||||||
Sbjct  3279  AAGAGAGAATCCTTGTGAATTTAACAGAGGGTATAGGGATTACACGCTACTACCAGTTGG  3338

Query  3417  TTATGTGACAAAAGAATTAGAATACTTCCCAGAAACCGATAAGGTATGGATTGAGATTGG  3476
             ||||||||||||||||||||||||||||||||||||||||||||||||||||||||||||
Sbjct  3339  TTATGTGACAAAAGAATTAGAATACTTCCCAGAAACCGATAAGGTATGGATTGAGATTGG  3398

Query  3477  AGAAACGGAAGGAACATTTATCGTGGACAGCGTGGAATTACTCCTTATGGAGGAA  3531
             ||||||||||||||||||||||||||||||||||||||||||||||||||||||
Sbjct  3399  AGAAACGGAAGGAACATTTATCGTGGACAGCGTGGAATTACTCCTTATGGAGGAA  3453
```

**Figure 4 (cont.)**

```
Query  99    ATGGAGATAAATAATCAGAAGCAATGCATACCATATAATTGCTTAAGTAATCCTGAGGAA  158
             ||||||||||||||||||||||||||||||||||||||||||||||||||||||||||||
Sbjct  1     ATGGAGATAAATAATCAGAAGCAATGCATACCATATAATTGCTTAAGTAATCCTGAGGAA  60

Query  159   GTACTTTTGGATGGGGAGAGGATATTACCTGATATCGATCCACTCGAAGTTTCTTTGTCG  218
             ||||||||||||||||||||||||||||||||||||||||||||||||||||||||||||
Sbjct  61    GTACTTTTGGATGGGGAGAGGATATTACCTGATATCGATCCACTCGAAGTTTCTTTGTCG  120

Query  219   CTTTTGCAATTTCTTTTGAATAACTTTGTTCCAGGGGGAGGCTTTATTTCAGGATTAGTT  278
             ||||||||||||||||||||||||||||||||||||||||||||||||||||||||||||
Sbjct  121   CTTTTGCAATTTCTTTTGAATAACTTTGTTCCAGGGGGAGGCTTTATTTCAGGATTAGTT  180

Query  279   GATAAAATATGGGGGGCTTTGAGACCATCTGAATGGGACTTATTTCTTGCACAGATTGAA  338
             ||||||||||||||||||||||||||||||||||||||||||||||||||||||||||||
Sbjct  181   GATAAAATATGGGGGGCTTTGAGACCATCTGAATGGGACTTATTTCTTGCACAGATTGAA  240

Query  339   CGGTTGATTGATCAAAGAATAGAAGCAACAGTAAGAGCAAAAGCAATCACTGAATTAGAA  398
             ||||||||||||||||||||||||||||||||||||||||||||||||||||||||||||
Sbjct  241   CGGTTGATTGATCAAAGAATAGAAGCAACAGTAAGAGCAAAAGCAATCACTGAATTAGAA  300

Query  399   GGATTAGGGAGAAATTATCAAATATACGCTGAAGCATTTAAAGAATGGGAATCAGATCCT  458
             ||||||||||||||||||||||||||||||||||||||||||||||||||||||||||||
Sbjct  301   GGATTAGGGAGAAATTATCAAATATACGCTGAAGCATTTAAAGAATGGGAATCAGATCCT  360

Query  459   GATAACGAAGCGGCTAAAAGTAGAGTAATTGATCGCTTTCGTATACTTGATGGTCTAATT  518
             ||||||||||||||||||||||||||||||||||||||||||||||||||||||||||||
Sbjct  361   GATAACGAAGCGGCTAAAAGTAGAGTAATTGATCGCTTTCGTATACTTGATGGTCTAATT  420

Query  519   GAAGCAAATATCCCTTCATTTCGGATAATTGGATTTGAAGTGCCACTTTTATCGGTTTAT  578
             ||||||||||||||||||||||||||||||||||||||||||||||||||||||||||||
Sbjct  421   GAAGCAAATATCCCTTCATTTCGGATAATTGGATTTGAAGTGCCACTTTTATCGGTTTAT  480

Query  579   GTTCAAGCAGCTAATCTACATCTCGCTCTATTGAGAGATTCTGTTATTTTTGGAGAGAGA  638
             ||||||||||||||||||||||||||||||||||||||||||||||||||||||||||||
Sbjct  481   GTTCAAGCAGCTAATCTACATCTCGCTCTATTGAGAGATTCTGTTATTTTTGGAGAGAGA  540

Query  639   TGGGGATTGACGACAAAAAATGTCAATGATATCTATAATAGACAAATTAGAGAAATTCAT  698
             ||||||||||||||||||||||||||||||||||||||||||||||||||||||||||||
Sbjct  541   TGGGGATTGACGACAAAAAATGTCAATGATATCTATAATAGACAAATTAGAGAAATTCAT  600

Query  699   GAATATAGCAATCATTGCGTAGATACGTATAACACAGAACTAGAACGTCTAGGGTTTAGA  758
             ||||||||||||||||||||||||||||||||||||||||||||||||||||||||||||
Sbjct  601   GAATATAGCAATCATTGCGTAGATACGTATAACACAGAACTAGAACGTCTAGGGTTTAGA  660

Query  759   TCTATAGCGCAGTGGAGAATATATAATCAGTTTAGAAGAGAACTAACACTAACTGTATTA  818
             ||||||||||||||||||||||||||||||||||||||||||||||||||||||||||||
Sbjct  661   TCTATAGCGCAGTGGAGAATATATAATCAGTTTAGAAGAGAACTAACACTAACTGTATTA  720

Query  819   GATATTGTCGCTCTTTTCCCGAACTATGACAGTAGACTGTATCCGATCCAAACTTTTTCT  878
             ||||||||||||||||||||||||||||||||||||||||||||||||||||||||||||
Sbjct  721   GATATTGTCGCTCTTTTCCCGAACTATGACAGTAGACTGTATCCGATCCAAACTTTTTCT  780

Query  879   CAATTGACAAGAGAAATTGTTACATCCCCAGTAAGCGAATTTTATTATGGTGTTATTAAT  938
             ||||||||||||||||||||||||||||||||||||||||||||||||||||||||||||
Sbjct  781   CAATTGACAAGAGAAATTGTTACATCCCCAGTAAGCGAATTTTATTATGGTGTTATTAAT  840

Query  939   AGTGGTAATATAATTGGTACTCTTACTGAACAGCAGATAAGGCGACCACATCTTATGGAC  998
             ||||||||||||||||||||||||||||||||||||||||||||||||||||||||||||
Sbjct  841   AGTGGTAATATAATTGGTACTCTTACTGAACAGCAGATAAGGCGACCACATCTTATGGAC  900

Query  999   TTCTTTAACTCCATGATCATGTATACATCAGATAATAGACGGGAACATTATTGGTCAGGA  1058
             ||||||||||||||||||||||||||||||||||||||||||||||||||||||||||||
Sbjct  901   TTCTTTAACTCCATGATCATGTATACATCAGATAATAGACGGGAACATTATTGGTCAGGA  960
```

**Figure 5**

```
Query  1059  CTTGAAATGACGGCTTATTTTACAGGATTTGCAGGAGCTCAAGTGTCATTCCCTTTAGTC  1118
             |||||||||||||||||||||||||||||| |||||||||||||||||||||||||||||||
Sbjct  961   CTTGAAATGACGGCTTATTTTACAGGACTTGCAGGAGCTCAAGTGTCATTCCCTTTAGTC  1020

Query  1119  GGGACTAGAGGGGAGTCAGCTCCACCATTAACTGTTAGAAGTGTTAATGATGGAATTTAT  1178
             ||||||||||||||||||||||||||||||||||||||||||||||||||||||||||||
Sbjct  1021  GGGACTAGAGGGGAGTCAGCTCCACCATTAACTGTTAGAAGTGTTAATGATGGAATTTAT  1080

Query  1179  AGAATATTATCGGCACCGTTTTATTCAGCGCCTTTTCTAGGCACCATTGTATTGGGAAGT  1238
             ||||||||||||||||||||||||||||||||||||||||||||||||||||||||||||
Sbjct  1081  AGAATATTATCGGCACCGTTTTATTCAGCGCCTTTTCTAGGCACCATTGTATTGGGAAGT  1140

Query  1239  CGTGGAGAAAAATTTGATTTTGCGCTTAATAATATTTCACCTCCGCCATCTACAATATAC  1298
             ||||||||||||||||||||||||||||||||||||||||||||||||||||||||||||
Sbjct  1141  CGTGGAGAAAAATTTGATTTTGCGCTTAATAATATTTCACCTCCGCCATCTACAATATAC  1200

Query  1299  AGACATCCTGGAACAGTAGATTCACTAGTCAGTATACCGCCACAGGATAATAGCGTACCA  1358
             ||||||||||||||||||||||||||||||||||||||||||||||||||||||||||||
Sbjct  1201  AGACATCCTGGAACAGTAGATTCACTAGTCAGTATACCGCCACAGGATAATAGCGTACCA  1260

Query  1359  CCGCACAGGGGATCTAGTCATCGATTAAGTCATGTTACAATGCGCGCAAGTTCCCCTATA  1418
             ||||||||||||||||||||||||||||||||||||||||||||||||||||||||||||
Sbjct  1261  CCGCACAGGGGATCTAGTCATCGATTAAGTCATGTTACAATGCGCGCAAGTTCCCCTATA  1320

Query  1419  TTCCATTGGACGCATCGCAGCGCAACCACTACAAATACAATTAATCCAAATGCTATTATC  1478
             ||||||||||||||||||||||||||||||||||||||||||||||||||||||||||||
Sbjct  1321  TTCCATTGGACGCATCGCAGCGCAACCACTACAAATACAATTAATCCAAATGCTATTATC  1380

Query  1479  CAAATACCACTAGTAAAAGCATTTAACCTTCATTCAGGTGCCACTGTTGTTAGAGGACCA  1538
             ||||||||||||||||||||||||||||||||||||||||||||||||||||||||||||
Sbjct  1381  CAAATACCACTAGTAAAAGCATTTAACCTTCATTCAGGTGCCACTGTTGTTAGAGGACCA  1440

Query  1539  GGGTTTACAGGTGGTGATATCCTTCGAAGAACGAATACTGGCACATTTGCAGATATGAGA  1598
             ||||||||||||||||||||||||||||||||||||||||||||||||||||||||||||
Sbjct  1441  GGGTTTACAGGTGGTGATATCCTTCGAAGAACGAATACTGGCACATTTGCAGATATGAGA  1500

Query  1599  GTAAATATTACTGGGCCATTATCCCAAAGATATCGTGTAAGAATTCGCTATGCTTCTACG  1658
             ||||||||||||||||||||||||||||||||||||||||||||||||||||||||||||
Sbjct  1501  GTAAATATTACTGGGCCATTATCCCAAAGATATCGTGTAAGAATTCGCTATGCTTCTACG  1560

Query  1659  ACAGATTTACAATTTTTCACGAGAATCAATGGAACTTCTGTAAATCAAGGTAATTTCCAA  1718
             ||||||||||||||||||||||||||||||||||||||||||||||||||||||||||||
Sbjct  1561  ACAGATTTACAATTTTTCACGAGAATCAATGGAACTTCTGTAAATCAAGGTAATTTCCAA  1620

Query  1719  AGAACTATGAATAGAGGGGATAATTTAGAATCTGGAAACTTTAGGACTGCAGGATTTAGT  1778
             ||||||||||||||||||||||||||||||||||||||||||||||||||||||||||||
Sbjct  1621  AGAACTATGAATAGAGGGGATAATTTAGAATCTGGAAACTTTAGGACTGCAGGATTTAGT  1680

Query  1779  ACGCCTTTTAGTTTTTCAAATGCGCAAAGTACATTCACATTGGGTACTCAGGCTTTTTCA  1838
             ||||||||||||||||||||||||||||||||||||||||||||||||||||||||||||
Sbjct  1681  ACGCCTTTTAGTTTTTCAAATGCGCAAAGTACATTCACATTGGGTACTCAGGCTTTTTCA  1740

Query  1839  AATCAGGAAGTTTATATAGATCGAATTGAATTTGTCCCGGCAGAAGTAACATTCGAGGCA  1898
             ||||||||||||||||||||||||||||||||||||||||||||||||||||||||||||
Sbjct  1741  AATCAGGAAGTTTATATAGATCGAATTGAATTTGTCCCGGCAGAAGTAACATTCGAGGCA  1800

Query  1899  GAATCTGATTTAGAAAGAGCGCAAAAGGCGGTGAATGCCCTGTTTACTTCTACAAACCAA  1958
             ||||||||||||||||||||||||||||||||||||||||||||||||||||||||||||
Sbjct  1801  GAATCTGATTTAGAAAGAGCGCAAAAGGCGGTGAATGCCCTGTTTACTTCTACAAACCAA  1860

Query  1959  CTAGGGCTAAAAACAGATGTGACGGATTATCAGATTGATCAAGTGTCCAATTTAGTAGAA  2018
             ||||||||||||||||||||||||||||||||||||||||||||||||||||||||||||
Sbjct  1861  CTAGGGCTAAAAACAGATGTGACGGATTATCAGATTGATCAAGTGTCCAATTTAGTAGAA  1920
```

**Figure 5 (cont.)**

```
Query  2019  TGTTTATCAGATGAATTTTGTCTGGATGAAAAGAGAGAATTGTCCGAGAAAGTCAAACAT  2078
             ||||||||||||||||||||||||||||||||||||||||||||||||||||||||||||
Sbjct  1921  TGTTTATCAGATGAATTTTGTCTGGATGAAAAGAGAGAATTGTCCGAGAAAGTCAAACAT  1980

Query  2079  GCAAAGCGACTTAGTGATAAGCGGAACCTACTTCAAGATCCAAACTTCACATCTATCAAT  2138
             ||||||||||||||||||||||||||||||||||||||||||||||||||||||||||||
Sbjct  1981  GCAAAGCGACTTAGTGATAAGCGGAACCTACTTCAAGATCCAAACTTCACATCTATCAAT  2040

Query  2139  AGACAACTAGACCGTGGATGGAGAGGAAGTACGGATATTACCATCCAAGGAGGAAATGAC  2198
             ||||||||||||||||||||||||||||||||||||||||||||||||||||||||||||
Sbjct  2041  AGACAACTAGACCGTGGATGGAGAGGAAGTACGGATATTACCATCCAAGGAGGAAATGAC  2100

Query  2199  GTATTCAAAGAGAATTACGTCACACTACCAGGTACCTTTGATGAGTGTTATCCAACGTAT  2258
             ||||||||||||||||||||||||||||||||||||||||||||||||||||||||||||
Sbjct  2101  GTATTCAAAGAGAATTACGTCACACTACCAGGTACCTTTGATGAGTGTTATCCAACGTAT  2160

Query  2259  TTGTATCAAAAAATAGATGAGTCAAAATTAAAAGCCTATACTCGCTATGAATTAAGAGGG  2318
             ||||||||||||||||||||||||||||||||||||||||||||||||||||||||||||
Sbjct  2161  TTGTATCAAAAAATAGATGAGTCAAAATTAAAAGCCTATACTCGCTATGAATTAAGAGGG  2220

Query  2319  TATATTGAAGATAGTCAAGATTTAGAAGTCTATTTGATTCGTTACAATGCGAAACATGAA  2378
             ||||||||||||||||||||||||||||||||||||||||||||||||||||||||||||
Sbjct  2221  TATATTGAAGATAGTCAAGATTTAGAAGTCTATTTGATTCGTTACAATGCGAAACATGAA  2280

Query  2379  ACAGTAAATGTTCCCGGTACAGGGTCCTTATGGCCGCTTTCAGTCGAAAGCCCAATCGGA  2438
             ||||||||||||||||||||||||||||||||||||||||||||||||||||||||||||
Sbjct  2281  ACAGTAAATGTTCCCGGTACAGGGTCCTTATGGCCGCTTTCAGTCGAAAGCCCAATCGGA  2340

Query  2439  AGGTGCGGAGAACCGAATCGATGTGTGCCACATATTGAATGGAATCCTGATTTAGATTGT  2498
             |||| |||||||||||||||||||||||||||||||||||||||||||||||||||||||
Sbjct  2341  AGGTACGGAGAACCGAATCGATGTGTGCCACATATTGAATGGAATCCTGATTTAGATTGT  2400

Query  2499  TCGTGTAGGGATGGGGAGAAGTGTGCCCATCATTCGCATCATTTCTCTCTAGATATTGAT  2558
             ||||||||||||||||||||||||||||||||||||||||||||||||||||||||||||
Sbjct  2401  TCGTGTAGGGATGGGGAGAAGTGTGCCCATCATTCGCATCATTTCTCTCTAGATATTGAT  2460

Query  2559  GTTGGATGTACAGACCTAAATGAGGACCTAGGTGTATGGGTGATCTTTAAGATTAAAACG  2618
             ||||||||||||||||||||||||||||||||||||||||||||||||||||||||||||
Sbjct  2461  GTTGGATGTACAGACCTAAATGAGGACCTAGGTGTATGGGTGATCTTTAAGATTAAAACG  2520

Query  2619  CAGGATGGCCATGCAAGATTAGGAAATCTAGAGTTTCTCGAAGAGAAACCATTGTTAGGA  2678
             ||||||||||||||||||||||||||||||||||||||||||||||||||||||||||||
Sbjct  2521  CAGGATGGCCATGCAAGATTAGGAAATCTAGAGTTTCTCGAAGAGAAACCATTGTTAGGA  2580

Query  2679  GAAGCGTTAGCTCGTGTGAAAAGAGCGGAGAAAAAATGGAGAGACAAACGCGAACAATTG  2738
             ||||||||||||||||||||||||||||||||||||||||||||||||||||||||||||
Sbjct  2581  GAAGCGTTAGCTCGTGTGAAAAGAGCGGAGAAAAAATGGAGAGACAAACGCGAACAATTG  2640

Query  2739  CAGTTTGAAACGAATATCGTTTACAAAGAGGCAAAAGAATCTGTAGATGCTTTATTCGTA  2798
             ||||||||||||||||||||||||||||||||||||||||||||||||||||||||||||
Sbjct  2641  CAGTTTGAAACGAATATCGTTTACAAAGAGGCAAAAGAATCTGTAGATGCTTTATTCGTA  2700

Query  2799  GATTCTCACTATAATAGATTACAAGCGGATACGAACATTACGATGATTCATGCGGCAGAT  2858
             ||||||||||||||||||||||||||||||||||||||||||||||||||||||||||||
Sbjct  2701  GATTCTCACTATAATAGATTACAAGCGGATACGAACATTACGATGATTCATGCGGCAGAT  2760

Query  2859  AAACGCGTTCATCGAATCCGAGAGGCTTATCTTCCGGAATTATCCGTTATCCCAGGTGTA  2918
             ||||||||||||||||||||||||||||||||||||||||||||||||||||||||||||
Sbjct  2761  AAACGCGTTCATCGAATCCGAGAGGCTTATCTTCCGGAATTATCCGTTATCCCAGGTGTA  2820

Query  2919  AATGCGGACATTTTTGAAGAATTAGAAGGTCTTATTTTCACTGCATTCTCCCTATATGAT  2978
             ||||||||||||||||||||||||||||||||||||||||||||||||||||||||||||
Sbjct  2821  AATGCGGACATTTTTGAAGAATTAGAAGGTCTTATTTTCACTGCATTCTCCCTATATGAT  2880
```

**Figure 5 (cont.)**

```
Query  2979  GCGAGAAATATCATTAAAAACGGTGATTTCAATAATGGTTTATCGTGTTGGAACGTGAAA  3038
             ||||||||||||||||||||||||||||||||||||||||||||||||||||||||||||
Sbjct  2881  GCGAGAAATATCATTAAAAACGGTGATTTCAATAATGGTTTATCGTGTTGGAACGTGAAA  2940

Query  3039  GGGCATGTAGATATACAACAGAATGATCATCGTTCTGTCCTCGTTGTCCCGGAATGGGAA  3098
             ||||||||||||||||||||||||||||||||||||||||||||||||||||||||||||
Sbjct  2941  GGGCATGTAGATATACAACAGAATGATCATCGTTCTGTCCTCGTTGTCCCGGAATGGGAA  3000

Query  3099  TCAGAGGTATCACAAGAAGTCCGCGTATGTCCAGGTCGTGGCTATATTCTTCGTGTCACA  3158
             ||||||||||||||||||||||||||||||||||||||||||||||||||||||||||||
Sbjct  3001  TCAGAGGTATCACAAGAAGTCCGCGTATGTCCAGGTCGTGGCTATATTCTTCGTGTCACA  3060

Query  3159  GCGTACAAAGAGGGCTACGGAGAAGGATGCGTAACGATCCATGAGATCGAAGACAATACA  3218
             ||||||||||||||||||||||||||||||||||||||||||||||||||||||||||||
Sbjct  3061  GCGTACAAAGAGGGCTACGGAGAAGGATGCGTAACGATCCATGAGATCGAAGACAATACA  3120

Query  3219  GACGAATTGAAGTTTAGTAACTGCATAGAAGAGGAAGTCTATCCAACGGATACAGGTAAT  3278
             ||||||||||||||||||||||||||||||||||||||||||||||||||||||||||||
Sbjct  3121  GACGAATTGAAGTTTAGTAACTGCATAGAAGAGGAAGTCTATCCAACGGATACAGGTAAT  3180

Query  3279  GATTATACTGCACACCAAGGTACAACAGGATGCGCAGATGCATGTAATTCCCGTAATGTT  3338
             ||||||||||||||||||||||||||||||||||||||||||||||||||||||||||||
Sbjct  3181  GATTATACTGCACACCAAGGTACAACAGGATGCGCAGATGCATGTAATTCCCGTAATGTT  3240

Query  3339  GGATATGAGGATGGATATGAAATAAATACTACAGCATCTGTTAATTACAAACCGACTTAT  3398
             ||||||||||||||||||||||||||||||||||||||||||||||||||||||||||||
Sbjct  3241  GGATATGAGGATGGATATGAAATAAATACTACAGCATCTGTTAATTACAAACCGACTTAT  3300

Query  3399  GAAGAAGAAATGTATACAGATGTACGAAGAGATAATCATTGTGAATATGACAGAGGATAT  3458
             ||||||||||||||||||||||||||||||||||||||||||||||||||||||||||||
Sbjct  3301  GAAGAAGAAATGTATACAGATGTACGAAGAGATAATCATTGTGAATATGACAGAGGATAT  3360

Query  3459  GGGAACCATACACCGTTACCAGCTGGTTATGTAACAAAAGAATTAGAGTACTTCCCTGAA  3518
             ||||||||||||||||||||||||||||||||||||||||||||||||||||||||||||
Sbjct  3361  GGGAACCATACACCGTTACCAGCTGGTTATGTAACAAAAGAATTAGAGTACTTCCCTGAA  3420

Query  3519  ACAGATACAGTATGGATAGAGATTGGAGAAACGGAAGGAACATTCATCGTAGATAGTGTG  3578
             ||||||||||||||||||||||||||||||||||||||||||||||||||||||||||||
Sbjct  3421  ACAGATACAGTATGGATAGAGATTGGAGAAACGGAAGGAACATTCATCGTAGATAGTGTG  3480

Query  3579  GAATTACTCCTCATGGAGGAATAA  3602
             ||||||||||||||||||||||||
Sbjct  3481  GAATTACTCCTCATGGAGGAATAA  3504
```

**Figure 5 (cont.)**

41

```
Query    1    MDNNPNINECIPYNCLSNPEVEVLGGERIETGYTPIDISLSLTQFLLSEFVPGAGFVLGL    60
              MDNNPNINECIPYNCLSNPEVEVLGGERIETGYTPIDISLSLTQFLLSEFVPGAGFVLGL
Sbjct    1    MDNNPNINECIPYNCLSNPEVEVLGGERIETGYTPIDISLSLTQFLLSEFVPGAGFVLGL    60

Query   61    VDIIWGIFGPSQWDAFLVQIEQLINQRIEEFARNQAISRLEGLSNLYQIYAESFREWEAD   120
              VDIIWGIFGPSQWDAFLVQIEQLINQRIEEFARNQAISRLEGLSNLYQIYAESFREWEAD
Sbjct   61    VDIIWGIFGPSQWDAFLVQIEQLINQRIEEFARNQAISRLEGLSNLYQIYAESFREWEAD   120

Query  121    PTNPALREEMRIQFNDMNSALTTAIPLFAVQNYQVPLLSVYVQAANLHLSVLRDVSVFGQ   180
              PTNPALREEMRIQFNDMNSALTTAIPLFAVQNYQVPLLSVYVQAANLHLSVLRDVSVFGQ
Sbjct  121    PTNPALREEMRIQFNDMNSALTTAIPLFAVQNYQVPLLSVYVQAANLHLSVLRDVSVFGQ   180

Query  181    RWGFDAATINSRYNDLTRLIGNYTDYAVRWYNTGLERVWGPDSRDWIRYNQFRRELTLTV   240
              RWGFDAATINSRYNDLTRLIGNYTDYAVRWYNTGLERVWGPDSRDWIRYNQFRRELTLTV
Sbjct  181    RWGFDAATINSRYNDLTRLIGNYTDYAVRWYNTGLERVWGPDSRDWIRYNQFRRELTLTV   240

Query  241    LDIVSLFPNYDSRTYPIRTVSQLTREIYTNPVLENFDGSFRGSAQGIEGSIRSPHLMDIL   300
              LDIVSLFPNYDSRTYPIRTVSQLTREIYTNPVLENFDGSFRGSAQGIEGSIRSPHLMDIL
Sbjct  241    LDIVSLFPNYDSRTYPIRTVSQLTREIYTNPVLENFDGSFRGSAQGIEGSIRSPHLMDIL   300

Query  301    SSITIYTDAHRGEYYWSGHQIMASPVGFSGPEFTFPLYGTMGNAAPQQRIVAQLGQGVYR   360
              SSITIYTDAHRGEYYWSGHQIMASPVGFSGPEFTFPLYGTMGNAAPQQRIVAQLGQGVYR
Sbjct  301    SSITIYTDAHRGEYYWSGHQIMASPVGFSGPEFTFPLYGTMGNAAPQQRIVAQLGQGVYR   360

Query  361    TLSSTLYRRPFNIGINNQQLSVLDGTEFAYGTSSNLPSAVYRKSGTVDSLDEIPPQNNNV   420
              TLSSTLYRRPFNIGINNQQLSVLDGTEFAYGTSSNLPSAVYRKSGTVDSLDEIPPQNNNV
Sbjct  361    TLSSTLYRRPFNIGINNQQLSVLDGTEFAYGTSSNLPSAVYRKSGTVDSLDEIPPQNNNV   420

Query  421    PPRQGFSHRLSHVSMFRSGFSNSSVSIIRAPMFSWIHRSAEFNNIIPSSQITQIPLTKST   480
              PPRQGFSHRLSHVSMFRSGFSNSSVSIIRAPMFSWIHRSAEFNNIIPSSQITQIPLTKST
Sbjct  421    PPRQGFSHRLSHVSMFRSGFSNSSVSIIRAPMFSWIHRSAEFNNIIPSSQITQIPLTKST   480

Query  481    NLGSGTSVVKGPGFTGGDILRRTSPGQISTLRVNITAPLSQRYRVRIRYASTTNLQFHTS   540
              NLGSGTSVVKGPGFTGGDILRRTSPGQISTLRVNITAPLSQRYRVRIRYASTTNLQFHTS
Sbjct  481    NLGSGTSVVKGPGFTGGDILRRTSPGQISTLRVNITAPLSQRYRVRIRYASTTNLQFHTS   540

Query  541    IDGRPINQGNFSATMSSGSNLQSGSFRTVGFTTPFNFSNGSSVFTLSAHVFNSGNEVYID   600
              IDGRPINQGNFSATMSSGSNLQSGSFRTVGFTTPFNFSNGSSVFTLSAHVFNSGNEVYID
Sbjct  541    IDGRPINQGNFSATMSSGSNLQSGSFRTVGFTTPFNFSNGSSVFTLSAHVFNSGNEVYID   600

Query  601    RIEFVPAEVTFEAEYDLERAQKAVNELFTSSNQIGLKTDVTDYHIDQVSNLVECLSDEFC   660
              RIEFVPAEVTFEAEYDLERAQKAVNELFTSSNQIGLKTDVTDYHIDQVSNLVECLSDEFC
Sbjct  601    RIEFVPAEVTFEAEYDLERAQKAVNELFTSSNQIGLKTDVTDYHIDQVSNLVECLSDEFC   660

Query  661    LDEKQEVSEKVKHAKRLSDERNLLQDPNFRGINRQLDRGWRGSTDITIQGGDDVFKENYV   720
              LDEKQEVSEKVKHAKRLSDERNLLQDPNFRGINRQLDRGWRGSTDITIQGGDDVFKENYV
Sbjct  661    LDEKQEVSEKVKHAKRLSDERNLLQDPNFRGINRQLDRGWRGSTDITIQGGDDVFKENYV   720

Query  721    TLPGTFDECYPTYLYQKIDESKLKAYTRYQLRGYIEDSQDLEIYLIRYNAKHETVNVPGT   780
              TLPGTFDECYPTYLYQKIDESKLKAYTRYQLRGYIEDSQDLEIYLIRYNAKHETVNVPGT
Sbjct  721    TLPGTFDECYPTYLYQKIDESKLKAYTRYQLRGYIEDSQDLEIYLIRYNAKHETVNVPGT   780

Query  781    GSLWPLSAQSPIGKCGEPNRCAPHLEWNPDLDCSCRDGEKCAHHSHHFSLDIDVGCTDLN   840
              GSLWPLSAQSPIG                           KCAHHSHHFSLDIDVGCTDLN
Sbjct  781    GSLWPLSAQSPIG-------------------------KCAHHSHHFSLDIDVGCTDLN   814

Query  841    EDLGVWVIFKIKTQDGHARLGNLEFLEEKPLVGEALARVKRAEKKWRDKREKLEWETNIV   900
              EDLGVWVIFKIKTQDGHARLGNLEFLEEKPLVGEALARVKRAEKKWRDKREKLEWETNIV
Sbjct  815    EDLGVWVIFKIKTQDGHARLGNLEFLEEKPLVGEALARVKRAEKKWRDKREKLEWETNIV   874

Query  901    YKEAKESVDALFVNSQYDQLQADTNIAMIHAADKRVHSIREAYLPELSVIPGVNAAIFEE   960
              YKEAKESVDALFVNSQYDQLQADTNIAMIHAADKRVHSIREAYLPELSVIPGVNAAIFEE
Sbjct  875    YKEAKESVDALFVNSQYDQLQADTNIAMIHAADKRVHSIREAYLPELSVIPGVNAAIFEE   934
```

**Figure 6**

```
Query   961    LEGCIFTAFSLYDARNVIKNGDFNNGLSCWNVKGHVDVEEQNNHRSVLVVPEWEAEVSQE   1020
               LEGCIFTAFSLYDARNVIKNGDFNNGLSCWNVKGHVDVEEQNNHRSVLVVPEWEAEVSQE
Sbjct   935    LEGCIFTAFSLYDARNVIKNGDFNNGLSCWNVKGHVDVEEQNNHRSVLVVPEWEAEVSQE   994

Query   1021   VRVCPGRGYILRVTAYKEGYGEGCVTIHEIENNTDELKFSNCVEEEIYPNNTVTCNDYTV   1080
               VRVCPGRGYILRVTAYKEGYGEGCVTIHEIENNTDELKFSNCVEEEIYPNNTVTCNDYTV
Sbjct   995    VRVCPGRGYILRVTAYKEGYGEGCVTIHEIENNTDELKFSNCVEEEIYPNNTVTCNDYTV   1054

Query   1081   NQEEYEGAYTSRNRGYNEAPSVPADYASVYEEKSYTDGRRENPCEFNRGYRDYTLLPVGY   1140
               NQEEYEGAYTSRNRGYNEAPSVPADYASVYEEKSYTDGRRENPCEFNRGYRDYTLLPVGY
Sbjct   1055   NQEEYEGAYTSRNRGYNEAPSVPADYASVYEEKSYTDGRRENPCEFNRGYRDYTLLPVGY   1114

Query   1141   VTKELEYFPETDKVWIEIGETEGTFIVDSVELLLMEE   1177
               VTKELEYFPETDKVWIEIGETEGTFIVDSVELLLMEE
Sbjct   1115   VTKELEYFPETDKVWIEIGETEGTFIVDSVELLLMEE   1151
```

**Figure 6 (cont,)**

```
Query    1    MEINNQKQCIPYNCLSNPEEVLLDGERILPDIDPLEVSLSLLQFLLNNFVPGGGFISGLV    60
              MEINNQKQCIPYNCLSNPEEVLLDGERILPDIDPLEVSLSLLQFLLNNFVPGGGFISGLV
Sbjct    1    MEINNQKQCIPYNCLSNPEEVLLDGERILPDIDPLEVSLSLLQFLLNNFVPGGGFISGLV    60

Query   61    DKIWGALRPSEWDLFLAQIERLIDQRIEATVRAKAITELEGLGRNYQIYAEAFKEWESDP   120
              DKIWGALRPSEWDLFLAQIERLIDQRIEATVRAKAITELEGLGRNYQIYAEAFKEWESDP
Sbjct   61    DKIWGALRPSEWDLFLAQIERLIDQRIEATVRAKAITELEGLGRNYQIYAEAFKEWESDP   120

Query  121    DNEAAKSRVIDRFRILDGLIEANIPSFRIIGFEVPLLSVYVQAANLHLALLRDSVIFGER   180
              DNEAAKSRVIDRFRILDGLIEANIPSFRIIGFEVPLLSVYVQAANLHLALLRDSVIFGER
Sbjct  121    DNEAAKSRVIDRFRILDGLIEANIPSFRIIGFEVPLLSVYVQAANLHLALLRDSVIFGER   180

Query  181    WGLTTKNVNDIYNRQIREIHEYSNHCVDTYNTELERLGFRSIAQWRIYNQFRRELTLTVL   240
              WGLTTKNVNDIYNRQIREIHEYSNHCVDTYNTELERLGFRSIAQWRIYNQFRRELTLTVL
Sbjct  181    WGLTTKNVNDIYNRQIREIHEYSNHCVDTYNTELERLGFRSIAQWRIYNQFRRELTLTVL   240

Query  241    DIVALFPNYDSRLYPIQTFSQLTREIVTSPVSEFYYGVINSGNIIGTLTEQQIRRPHLMD   300
              DIVALFPNYDSRLYPIQTFSQLTREIVTSPVSEFYYGVINSGNIIGTLTEQQIRRPHLMD
Sbjct  241    DIVALFPNYDSRLYPIQTFSQLTREIVTSPVSEFYYGVINSGNIIGTLTEQQIRRPHLMD   300

Query  301    FFNSMIMYTSDNRREHYWSGLEMTAYFTGFAGAQVSFPLVGTRGESAPPLTVRSVNDGIY   360
              FFNSMIMYTSDNRREHYWSGLEMTAYFTG AGAQVSFPLVGTRGESAPPLTVRSVNDGIY
Sbjct  301    FFNSMIMYTSDNRREHYWSGLEMTAYFTGLAGAQVSFPLVGTRGESAPPLTVRSVNDGIY   360

Query  361    RILSAPFYSAPFLGTIVLGSRGEKFDFALNNISPPPSTIYRHPGTVDSLVSIPPQDNSVP   420
              RILSAPFYSAPFLGTIVLGSRGEKFDFALNNISPPPSTIYRHPGTVDSLVSIPPQDNSVP
Sbjct  361    RILSAPFYSAPFLGTIVLGSRGEKFDFALNNISPPPSTIYRHPGTVDSLVSIPPQDNSVP   420

Query  421    PHRGSSHRLSHVTMRASSPIFHWTHRSATTTNTINPNAIIQIPLVKAFNLHSGATVVRGP   480
              PHRGSSHRLSHVTMRASSPIFHWTHRSATTTNTINPNAIIQIPLVKAFNLHSGATVVRGP
Sbjct  421    PHRGSSHRLSHVTMRASSPIFHWTHRSATTTNTINPNAIIQIPLVKAFNLHSGATVVRGP   480

Query  481    GFTGGDILRRTNTGTFADMRVNITGPLSQRYRVRIRYASTTDLQFFTRINGTSVNQGNFQ   540
              GFTGGDILRRTNTGTFADMRVNITGPLSQRYRVRIRYASTTDLQFFTRINGTSVNQGNFQ
Sbjct  481    GFTGGDILRRTNTGTFADMRVNITGPLSQRYRVRIRYASTTDLQFFTRINGTSVNQGNFQ   540

Query  541    RTMNRGDNLESGNFRTAGFSTPFSFSNAQSTFTLGTQAFSNQEVYIDRIEFVPAEVTFEA   600
              RTMNRGDNLESGNFRTAGFSTPFSFSNAQSTFTLGTQAFSNQEVYIDRIEFVPAEVTFEA
Sbjct  541    RTMNRGDNLESGNFRTAGFSTPFSFSNAQSTFTLGTQAFSNQEVYIDRIEFVPAEVTFEA   600

Query  601    ESDLERAQKAVNALFTSTNQLGLKTDVTDYQIDQVSNLVECLSDEFCLDEKRELSEKVKH   660
              ESDLERAQKAVNALFTSTNQLGLKTDVTDYQIDQVSNLVECLSDEFCLDEKRELSEKVKH
Sbjct  601    ESDLERAQKAVNALFTSTNQLGLKTDVTDYQIDQVSNLVECLSDEFCLDEKRELSEKVKH   660

Query  661    AKRLSDKRNLLQDPNFTSINRQLDRGWRGSTDITIQGGNDVFKENYVTLPGTFDECYPTY   720
              AKRLSDKRNLLQDPNFTSINRQLDRGWRGSTDITIQGGNDVFKENYVTLPGTFDECYPTY
Sbjct  661    AKRLSDKRNLLQDPNFTSINRQLDRGWRGSTDITIQGGNDVFKENYVTLPGTFDECYPTY   720

Query  721    LYQKIDESKLKAYTRYELRGYIEDSQDLEVYLIRYNAKHETVNVPGTGSLWPLSVESPIG   780
              LYQKIDESKLKAYTRYELRGYIEDSQDLEVYLIRYNAKHETVNVPGTGSLWPLSVESPIG
Sbjct  721    LYQKIDESKLKAYTRYELRGYIEDSQDLEVYLIRYNAKHETVNVPGTGSLWPLSVESPIG   780

Query  781    RCGEPNRCVPHIEWNPDLDCSCRDGEKCAHHSHHFSLDIDVGCTDLNEDLGVWVIFKIKT   840
              R GEPNRCVPHIEWNPDLDCSCRDGEKCAHHSHHFSLDIDVGCTDLNEDLGVWVIFKIKT
Sbjct  781    RYGEPNRCVPHIEWNPDLDCSCRDGEKCAHHSHHFSLDIDVGCTDLNEDLGVWVIFKIKT   840

Query  841    QDGHARLGNLEFLEEKPLLGEALARVKRAEKKWRDKREQLQFETNIVYKEAKESVDALFV   900
              QDGHARLGNLEFLEEKPLLGEALARVKRAEKKWRDKREQLQFETNIVYKEAKESVDALFV
Sbjct  841    QDGHARLGNLEFLEEKPLLGEALARVKRAEKKWRDKREQLQFETNIVYKEAKESVDALFV   900

Query  901    DSHYNRLQADTNITMIHAADKRVHRIREAYLPELSVIPGVNADIFEELEGLIFTAFSLYD   960
              DSHYNRLQADTNITMIHAADKRVHRIREAYLPELSVIPGVNADIFEELEGLIFTAFSLYD
Sbjct  901    DSHYNRLQADTNITMIHAADKRVHRIREAYLPELSVIPGVNADIFEELEGLIFTAFSLYD   960
```

**Figure 7**

```
Query  961   ARNIIKNGDFNNGLSCWNVKGHVDIQQNDHRSVLVVPEWESEVSQEVRVCPGRGYILRVT   1020
             ARNIIKNGDFNNGLSCWNVKGHVDIQQNDHRSVLVVPEWESEVSQEVRVCPGRGYILRVT
Sbjct  961   ARNIIKNGDFNNGLSCWNVKGHVDIQQNDHRSVLVVPEWESEVSQEVRVCPGRGYILRVT   1020


Query  1021  AYKEGYGEGCVTIHEIEDNTDELKFSNCIEEEVYPTDTGNDYTAHQGTTGCADACNSRNV   1080
             AYKEGYGEGCVTIHEIEDNTDELKFSNCIEEEVYPTDTGNDYTAHQGTTGCADACNSRNV
Sbjct  1021  AYKEGYGEGCVTIHEIEDNTDELKFSNCIEEEVYPTDTGNDYTAHQGTTGCADACNSRNV   1080


Query  1081  GYEDGYEINTTASVNYKPTYEEEMYTDVRRDNHCEYDRGYGNHTPLPAGYVTKELEYFPE   1140
             GYEDGYEINTTASVNYKPTYEEEMYTDVRRDNHCEYDRGYGNHTPLPAGYVTKELEYFPE
Sbjct  1081  GYEDGYEINTTASVNYKPTYEEEMYTDVRRDNHCEYDRGYGNHTPLPAGYVTKELEYFPE   1140


Query  1141  TDTVWIEIGETEGTFIVDSVELLLMEE    1167
             TDTVWIEIGETEGTFIVDSVELLLMEE
Sbjct  1141  TDTVWIEIGETEGTFIVDSVELLLMEE    1167
```

**Figure 7 (cont.)**

**Figure 8**

## REFERENCES CITED IN THE DESCRIPTION

### Patent documents cited in the description

- EP 0366397 A1
- US 2010160231 A1
- WO 2012131495 A2
- WO 2012131495 A
- CN 103333230 B
- WO 9504146 A2
- US 2009005306 A
- US 2018127771 A1
- WO 2013134734 A2
- WO 1995024493 A
- US 20080016596 A1
- WO 2010027793 A

### Non-patent literature cited in the description

- **ALTSCHUL, S.F. ; GISH, W. ; MILLER, W. ; MYERS, E.W. ; LIPMAN, D.J.** Basic local alignment search tool. *J. Mol. Biol.*, 1990, vol. 215, 403-410
- **BAIG, D.N. ; BUKHARI, D.A ; SHAKOORI, A.R**. cry Genes profiling and the toxicity of isolates of Bacillus thuringiensis from soil samples against American bollworm, Helicoverpa armigera. *J. Appl. Microbiol.*, 2010, vol. 109, 1967-1978
- **BARRETTO, M. R et al.** Insecticidal activity of culture supernatants from Bacillus thuringiensis Berliner strains against Spodoptera frugiperda Smith (Lepidoptera. Noctuidae) Larvae. *An. Soc.Entomol. Brasil*, 1999, vol. 28, 675-685
- **BRADFORD, M.M.** A rapid and sensitive method for the quantitation of microgram quantities of protein utilizing the principle of protein-dye binding. *Anal. Biochem.*, 1976, vol. 72, 248-254
- **CAPALBO D. et al.** Solid-state fermentation of Bacillus thuringiensis tolworthi to control fall armyworm in maize. *EJB Electronic Journal of Biotechnology*, 2001, vol. 4 (2), 717-3458
- **CAROZZI, N.B ; KRAMER, V.C. ; WARREN, G.W ; EVOLA, S. ; KOZIEL, M.G.** Prediction of insecticidal activity of Bacillus thuringiensis strains by polymerase chain reaction product profiles. *Appl. Environ. Microbiol.*, 1991, vol. 57, 3057-3061
- **CERÓN, J ; ORTIZ, A ; QUINTERO, R ; GÜERECA, I ; BRAVO, A.** Specific PCR primers directed to identify <i>cryl<i> and <i>cryIII<i> genes within a <i> Bacillus thuringiensis<i> strain collection. *Appl. Envir. Microbiol*, 1995, vol. 61, 3826-3831
- **CRICKMORE, N ; BAUM, J ; BRAVO, A. ; LERECLUS, D. ; NARVA, K ; SAMPSON, K et al.** *Bacillus thuringiensis toxin nomenclature*, 2018
- **CRICKMORE, N. ; ZEIGLER, D.R ; FEITELSON, J ; SCHNEPF, E ; VAN RIE, J ; LERECLUS, D et al.** Revision of the nomenclature for the Bacillus thuringiensis pesticidal crystal proteins. *Microbiol. Mol. Biol. Rev*, 1998, vol. 62, 807-13
- **CUCARELLA, C ; SOLANO, C. ; VALLE, J ; AMORENA, B ; LASA, I ; PENADÉS, J.** Bap, a Staphylococcus aureus surface protein involved in biofilm formation. *J. Bacteriol.*, 2001, vol. 183, 2888-2896
- **DONOVAN, W.P ; ENGLEMAN, J.T ; DONOVAN, J.C ; BAUM, J.A ; BUNKERS, G.J. ; CHI, D.J. et al.** Discovery and characterization of Sip1A: a novel secreted protein from Bacillus thuringiensis with activity against coleopteran larvae. *Appl. Microbiol. Biotechnol.*, 2006, vol. 72, 713-719
- Insecticidal activity of isolated of Bacillus thuringiensis and their potential for pest control. **DULMAGE, H.T.** Microb. Control pests plant Dis. Academic Press, 1970
- **ESTRUCH, J.J ; WARREN, G.W ; MULLINS, M.A ; NYE, G.J ; CRAIG, J.A ; KOZIEL, M.G**. Vip3A, a novel Bacillus thuringiensis vegetative insecticidal protein with a wide spectrum of activities against lepidopteran insects. *Proc. Natl. Acad. Sci.*, 1996, vol. 93, 5389-5394
- **FERRANDIS, M.D. ; JÚREZ-PÉREZ, V.M ; FRUTOS, R ; BEL, Y ; FERRÉ, J.** Distribution of cryI, cryII and cryV genes within Bacillus thuringiensis isolates from Spain. *Syst. Appl. Microbiol.*, 1999, vol. 22, 179-185
- **FINNEY, D..** Probit analysis. Cambridge Univ. Press, 1971, 333
- **FRANKENHUYZEN, K. VAN**. Insecticidal activity of Bacillus thuringiensis crystal proteins. *J. Invertebr. Pathol.*, 2009, vol. 101, 1-16
- **HERNÁNDEZ, C.S ; MARTINEZ, C ; PORCAR, M ; CABALLERO, P ; FERRÉ, J.** Correlation between serovars of Bacillus thuringiensis and type I β-exotoxin production.. *J. Invertebr. Pathol.*, 2003, vol. 82, 57-62

- **HILBECK A.** ; **OTTO M.** Specifity and combinatorial effects of Bacillus thuringiensis Cry toxins in .... *Frontiers in Environmental Science, Review*, 09 November 2015
- **HOFFMAN, D.J.** ; **LAWSON, F.R.** Preliminary Studies on Mass Rearing of the Tobacco Hornworm. *J. Econ. Entomol.*, 1964, vol. 57, 354-355
- **HU, Y** ; **PLATZER, E.G** ; **BELLIER, A.** ; **AROIAN, R. V**. Discovery of a highly synergistic anthelmintic combination that shows mutual hypersusceptibility. *Proc. Natl. Acad. Sci.*, 2010, vol. 107, 5955-5960
- **HUGHES, P.R.** ; **WOOD, H.A.** A synchronous peroral technique for the bioassay of insect viruses. *J. Invertebr. Pathol.*, 1981, vol. 37, 154-159
- **IRIARTE, J.** ; **BEL, Y** ; **FERRANDIS, M.D** ; **ANDREW, R** ; **MURILLO, J** ; **FERRÉ, J** ; **CABALLERO, P**. Environmental Distribution and Diversity of Bacillus thuringiensis in Spain. *Syst. Appl. Microbiol.*, 1998, vol. 21, 97-106
- **LAEMMLI, U.K**. Cleavage of Structural Proteins during the Assembly of the Head of Bacteriophage T4. *Nature*, 1970, vol. 227, 680-685
- **LECADET, M.-M.** ; **FRACHON, E** ; **DUMANOIR, V.C** ; **RIPOUTEAU, H** ; **HAMON, S** ; **LAURENT, P** ; **THIERY, I.** Updating the H-antigen classification of Bacillus thuringiensis. *J. Appl. Microbiol.*, 1999, vol. 86, 660-672
- LeOra Software (1987) LeOra Software (1987) POLO PC, a User Guide to Probit or Logit Analysis. *Le Ora Software*
- **LI, L** ; **YANG, C** ; **LIU, Z** ; **LI, F.** ; **YU, Z.** [Screening of acrystalliferous mutants from Bacillus thuringiensis and their transformation properties]. *Wei Sheng Wu Xue Bao*, 2000, vol. 40, 85-90
- **LÓPEZ-PAZOS, S.A** ; **ROJAS ARIAS, A.C** ; **OSPINA, S.A** ; **CERÓN, J.** Activity of Bacillus thuringiensis hybrid protein against a lepidopteran and a coleopteran pest. *FEMS Microbiol. Lett*, 2010, vol. 302, 93-98
- **MASSON, L** ; **ERLANDSON, M** ; **PUZSTAI-CAREY, M** ; **BROUSSEAU, R** ; **JUÁREZ-PÉREZ, V.** ; **FRUTOS, R.** A holistic approach for determining the entomopathogenic potential of Bacillus thuringiensis strains. *Appl. Environ. Microbiol.*, 1998, vol. 64, 4782-8
- **MILNER, R.J.** History of Bacillus thuringiensis. *Agric. Ecosyst. Environ.*, 1994, vol. 49, 9-13
- **MOUNSEF JR** ; **SALAMEH D** ; **AWAD M KALLASSY** ; **CHAMY L** ; **BRANDAM C** ; **LTEIF R**. A simple method for the separation of Bacillus thuringiensis spores and crystals. *J Microbiol Methods*, 2014, vol. 107, 147-149
- **OHBA, M** ; **MIZUKI, E** ; **UEMORI, A.** Parasporin, a new anticancer protein group from Bacillus thuringiensis.. *Anticancer Res.*, 2009, vol. 29, 427-33
- **PARK, H.W** ; **GE, B.** ; **BAUER, L.S.** ; **FEDERICI, B.A.** Optimization of Cry3A yields in Bacillus thuringiensis by use of sporulation-dependent promoters in combination with the STAB-SD mRNA sequence. *Appl. Environ. Microbiol.*, 1998, vol. 64, 3932-8
- **POLANCZYK, R.A. et al.** Effectiveness of Bacillus thuringiensis strains against Spodoptera frugiperda (lepidopera: noctuidae). *Brazilian Journal of Microbiology*, 2000, vol. 31, 165-167
- **ROH, J.Y.** ; **KIM, Y.S** ; **WANG, Y.** ; **LIU, Q** ; **TAO, X** ; **XU, H.G et al.** Expression of Bacillus thuringiensis mosquitocidal toxin in an antimicrobial Bacillus brevis strain. *J. Asia. Pac. Entomol.*, 2010, vol. 13, 61-64
- **SAMBROOK, J.** ; **FRITSCH, E.F** ; **MANIATIS, T.** Molecular cloning: a laboratory manual.. Cold Spring Harbor Laboratory Press, 1989
- **SCHNEPF, H.E.** ; **WHITELEY, H.R.** Cloning and expression of the Bacillus thuringiensis crystal protein gene in Escherichia coli. *Proc. Natl. Acad. Sci. U. S. A*, 1981, vol. 78, 2893-7
- **SHAH, S.H.** ; **JAN, S.A** ; **AHMAD, N.** ; **KHAN, S.U** ; **KUMAR, T** ; **IQBAL, A et al.** Use of Different Promoters in Transgenic Plant Development : Current Challenges and Future Perspectives. *Am. J. Agric. Environ. Sci.*, 2015, vol. 15, 664-675
- **SHARMA, H.C** ; **DHILLON, M.K.** ; **ARORA, R.** Effects of Bacillus thuringiensis δ-endotoxin-fed Helicoverpa armigera on the survival and development of the parasitoid Campoletis chlorideae. *Entomol. Exp. Appl.*, 2008, vol. 126, 1-8
- **STEWART, G.S** ; **JOHNSTONE, K.** ; **HAGELBERG, E** ; **ELLAR, D.J**. Commitment of bacterial spores to germinate A measure of the trigger reaction.. *Biochem. J.*, 1981, vol. 198, 101-106
- **TABASHNIK, B.E.** Evaluation of synergism among Bacillus thuringiensis toxins. *Appl. Environ. Microbiol.*, 1992, vol. 58, 3343-3346
- **THOMAS, W.E.** ; **ELLAR, D.J.** Bacillus thuringiensis var israelensis crystal delta-endotoxin: effects on insect and mammalian cells in vitro and in vivo. *J. Cell Sci.*, 1983, vol. 60, 181-97